# EUROPÄISCHE PATENTSCHRIFT

(11) **EP 0 626 387 B1**
(45) Veröffentlichungstag und Bekanntmachung des Hinweises auf die Patenterteilung: **10.03.1999**
(21) Anmeldenummer: 94810255.3
(22) Anmeldetag: 03.05.1994
(51) Int. Cl.: C07H 19/04, C07H 21/02, A61K 31/70

(54) **Nukleoside und Oligonukleotide mit 2'-Ethergruppen**
Nucleosides and oligonucleotides with 2'-ether groups
Nucléosides et oligonucléotides contenant des groupes d'éther sur les positions 2'

(30) Priorität: 12.05.1993 CH 1467/93
(43) Veröffentlichungstag der Anmeldung: 30.11.1994
(73) Patentinhaber: Novartis AG, 4058 Basel (CH); Novartis-Erfindungen Verwaltungsgesellschaft m.b.H., 1235 Wien (AT)
(72) Erfinder: Martin, Pierre, Dr., CH-4310 Rheinfelden (CH)

(56) Entgegenhaltungen:
- WO-A-91/06556
- CHEMICAL ABSTRACTS, vol. 64, no. 3, 31. Januar 1966, Columbus, Ohio, US; abstract no. 3659a, S.M. ZHENODAROVA 'Isolation and properties of pyridinium and barium salts of O-butoxyethylidene derivatives of uridine 3'-phosphate' & IZV. AKAD. NAUK SSSR, SER. KHIM. 1965 Seiten 1667 - 9
- ANTI-CANCER DRUG DESIGN Bd. 6 , 1991 Seiten 585 - 607 P.D.COOK 'Medicinal chemistry of antisense oligonucleotides'

## Beschreibung

Die Erfindung betrifft Ribo-Nukleosidanaloge, deren 2'-OH Gruppe mit Polyol-Derivaten verethert ist, ein Verfahren zu deren Herstellung, Oligonukleotide mit diesen Nukleosiden und die Verwendung der Nukleoside zur Herstellung von Oligonukleotiden mit gleichen oder verschiedenen Nukleosideinheiten im Molekül.

Nukleoside und Oligonukleotide haben als antivirale Wirkstoffe bzw. wegen ihrer Fähigkeit zur Wechselwirkung mit Nukleinsäuren ("Antisense"-Oligonukleotide) und der damit verbundenen biologischen Aktivität breites Interesse gefunden, siehe zum Beispiel Uhlmann, E., Peyman, A., Chemical Reviews 90:543-584 (1990). Zur Bereitstellung von Nukleosiden mit neuen Eigenschaften oder zur Verbesserung der Wechselwirkung von Antisense-Oligonukleotiden mit natürlichen Nukleinsäuren sowie ihrer Stabilität gegenüber Nukleasen sind die Zuckerreste von Nukleosiden (bzw. der Nukleotideinheiten in Oligonukleotiden), oder die internukleotidische Phosphatbindung in Oligonukleotiden in unterschiedlichster Weise modifiziert worden, siehe zum Beispiel Marquez, V.E., Lim, M.I., Medicinal Research Reviews 6:1-40 (1986), Hélène, C., Toulmé, J.J., Biochimica et Biophysica Acta 1049:99-125 (1990), Englisch, U., Gauss, D.H., Angewandte Chemie 103:629-646 (1991), Matteucci, M.D., Bischofberger, N., Annual Reports in Medicinal Chemistry 26:87-296 (1991). In Cook, P.D., Anti-Cancer Drug Design 6:585-607 (1991) und WO 91/06556 werden Nukleoside beschrieben, die an der 2'-OH-Gruppe des Zuckers modifiziert sind. Die beschriebenen Modifikationen führen zu erhöhter Nukleaseresistenz; je länger der Alkylrest wird, umso höher wird die Nukleaseresistenz. Dabei wird mit kleinen Alkylresten wie Methyl, Ethyl oder Propyl eine schwache Erhöhung der Bindungsaffinität beobachtet, bei längeren Ketten sinkt die Bindungsaffinität aber drastisch ab. Nukleoside mit Polyol-Derivaten als Seitenketten an der 2'-OH Gruppe sind unbekannt und wurden bis anhin nie in Oligonukleotide eingebaut. Überraschenderweise erhöhen die erfindungsgemässen Modifikationen die Bindungsaffinität zur komplementären RNA nicht nur bei kürzeren sondern auch bei längeren Ketten. Dieses Ergebnis war aufgrund der publizierten Daten nicht zu erwarten. In Analogie zu den 2'-OH-modifizierten Oligoribonukleotiden zeichnen sich die erfindungsgemässen Verbindungen ebenfalls durch eine markante Nukleaseresistenz aus. Zusätzlich weisen Oligonukleotide, welche die erfindungsgemässen Nukleoside enthalten, eine erhöhte zelluläre Aufnahme auf und verfügen demzufolge über eine verbesserte Bio-Verfügbarkeit und Aktivität in vivo.

Ein Gegenstand der Erfindung sind Verbindungen der Formel I 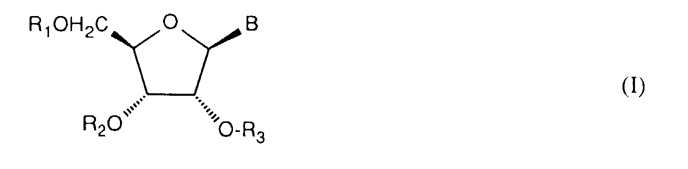 worin R₁ und R₂ unabhängig voneinander für Wasserstoff oder eine Schutzgruppe stehen oder R₁ diese Bedeutungen hat und R₂ für einen eine phosphorhaltige Nukleotid-Brückengruppe bildenden Rest steht; B einen Purin- oder Pyrimidinrest oder ein Analoges davon bedeutet; und R₃ einen Rest der Formel Ia, Ib oder Ic bedeutet 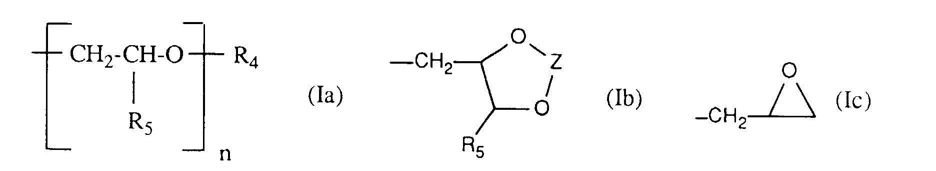 worin
- R₄: Wasserstoff, C₁-C₂₁-Alkyl, C₂-C₂₁-Alkenyl, C₂-C₂₁-Alkinyl oder -C(=O)-Alkyl;
- R₅: Wasserstoff, C₁-C₁₀-Alkyl, -CH₂-O-R₆ oder einen Rest der Formel Ib;
- R₆: Wasserstoff, C₁-C₂₂-Alkyl, C₃-C₂₁-Alkenyl, teilweise oder vollständig mit Fluor substituiertes C₁-C₁₀-Alkyl oder -[(CH₂)₂-O]ₘ-R₇;
- R₇: Wasserstoff oder C₁-C₂₁-Alkyl;
- Z: -(CH₂)ₚ- oder -(CH₂-CH₂-O)_{q}-CH₂CH₂-, wobei Z im Fall von -CH₂- unsubstituiert oder mit einem oder mehreren C₁-C₁₀-Alkyl, C₅-C₆-Cycloalkyl oder unsubstituiertem oder mit C₁-C₄-Alkyl substituiertem Phenyl unabhängig voneinander substituiert sein kann;
- n: eine Zahl von 1 bis 12;
- m: eine Zahl von 1 bis 4;
- p: eine Zahl von 1 bis 10; und
- q: eine Zahl von 1 bis 4 bedeuten;
wobei R₄ für den Fall n=1 und R₅=Wasserstoff nicht Wasserstoff bedeutet.

In einer bevorzugten Ausführungsform bedeutet R₄ Wasserstoff, C₁-C₂₁-Alkyl, C₂-C₂₁-Alkenyl oder C₂-C₂₁-Alkinyl. In einer bevorzugteren Ausführungsform bedeutet R₄ H, C₁-C₈-Alkyl, C₂-C₈-Alkenyl oder C₂-C₈-Alkinyl. In einer besonders bevorzugten Ausführungsform bedeutet R₄ H oder C₁-C₄-Alkyl. Beispiele für R₄ sind Methyl, Ethyl, n- oder i-Propyl, n, i- und t-Butyl, die Isomeren von Pentyl, Hexyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Undecyl, Vinyl, Allyl, But-3-en-1-yl, But-2-en-1-yl, Pentenyl, Octenyl, Dodecenyl, Octadecenyl, Ethinyl, Prop-1-in-1-yl, Prop-1-in-3-yl, But-1-in-1- oder -4-yl. Besonders bevorzugte Reste R₄ sind H, Methyl, Ethyl, n- oder i-Propyl oder n-Butyl. Die Zahlenwerte n betragen bevorzugt 1 bis 8, besonders bevorzugt 1 bis 6, insbesondere 1 bis 3.

In einer bevorzugten Ausführungsform bedeutet R₅ Wasserstoff, C₁-C₅-Alkyl oder -CH₂-O-R₆, besonders bevorzugt Wasserstoff, Methyl oder -CH₂-OH, -CH₂-O-C₁-C₂₂-Alkyl oder -CH₂-O-[(CH₂)₂-O]ₘ-C₁-C₁₀-alkyl, worin m eine Zahl von 1 bis 4 bedeutet.

In einer bevorzugten Ausführungsform stellen R₁ und R₂ Wasserstoff dar.

Schutzgruppen und Verfahren zur Derivatisierung der Hydroxylgruppen mit solchen Schutzgruppen sind in der Zucker- und Nukleotidchemie allgemein bekannt und zum Beispiel von Greene, B.T., Protective Groups in Organic Synthesis, Wiley Interscience, New York (1991), von Sonveaux, E., Bioorganic Chemistry 14:274-325 (1986) oder von Beaucage, S.L., Iyer, R., Tetrahedron 48:2223-2311 (1992) beschrieben. Beispiele für solche Schutzgruppen sind: Benzyl, Methylbenzyl, Dimethylbenzyl, Methoxybenzyl, Dimethoxybenzyl, Brombenzyl, 2,4-Dichlorbenzyl; Diphenylmethyl, Di(methylphenyl)methyl, Di(dimethylphenyl)methyl, Di(methoxyphenyl)methyl, Di(dimethoxyphenyl)methyl, Triphenylmethyl, Tris-4,4',4"-tert.butylphenylmethyl, Di-p-anisylphenylmethyl, Tri(methylphenyl)methyl, Tri(dimethylphenyl)methyl, Methoxyphenyl(diphenyl)methyl, Di(methoxyphenyl)phenylmethyl, Tri(methoxyphenyl)methyl, Tri(dimethoxyphenyl)methyl; Triphenylsilyl, Alkyldiphenylsilyl, Dialkylphenylsilyl und Trialkylsilyl mit 1 bis 20, bevorzugt 1 bis 12 und besonders bevorzugt 1 bis 8 C-Atomen in den Alkylgruppen, zum Beispiel Trimethylsilyl, Triethylsilyl, Tri-n-propylsilyl, i-Propyl-dimethylsilyl, t-Butyl-dimethylsilyl, t-Butyl-diphenylsilyl, n-Octyl-dimethylsilyl, (1,1,2,2-Tetramethylethyl)-dimethylsilyl; -(C₁-C₈-Alkyl)₂Si-O-Si(C₁-C₈-Alkyl)₂-, worin Alkyl zum Beispiel Methyl, Ethyl n- und i-Propyl, n-, i- oder t-Butyl bedeutet; C₂-C₁₂-, besonders C₂-C₈-Acyl, wie zum Beispiel Acetyl, Propanoyl, Butanoyl, Pentanoyl, Hexanoyl, Benzoyl, Methylbenzoyl, Methoxybenzoyl, Chlorbenzoyl und Brombenzoyl; R_{S1}-SO₂-, worin R_{S1} C₁-C₁₂-Alkyl, besonders C₁-C₆-Alkyl, C₅- oder C₆-Cycloalkyl, Phenyl, Benzyl, C₁-C₁₂- und besonders C₁-C₄-Alkylphenyl, oder C₁-C₁₂- und besonders C₁-C₄-Alkylbenzyl, oder Halogenphenyl oder Halogenbenzyl bedeutet, zum Beispiel Methyl-, Ethyl-, Propyl-, Butyl-, Phenyl-, Benzyl-, p-Brom-, p-Methoxy- und p-Methylphenylsulfonyl; unsubstituiertes oder mit F, Cl, Br, C₁-C₄-Alkoxy, Tri-(C₁-C₄-Alkyl)silyl oder C₁-C₄-Alkylsulfonyl substituiertes C₁-C₁₂-, bevorzugt C₁-C₈-Alkoxycarbonyl, zum Beispiel Methoxy-, Ethoxy-, n- oder i-Propoxy- oder n-, i- oder t-Butoxycarbonyl, 2-Trimethylsilylethoxycarbonyl, 2-Methylsulfonylethoxycarbonyl, Allyloxycarbonyl oder unsubstituiertes oder wie für Alkoxycarbonyl substituiertes Phenyloxycarbonyl oder Benzyloxycarbonyl, zum Beispiel Methyl- oder Methoxy- oder Chlorphenyloxycarbonyl oder -benzyloxycarbonyl, sowie 9-Fluorenylmethyloxycarbonyl. Sofern R₁ und/oder R₂ Alkyl bedeuten, kann es mit F, Cl, Br, C₁-C₄-Alkoxy, Phenyloxy, Chlorphenyloxy, Methoxyphenyloxy, Benzyloxy, Methoxybenzyloxy oder Chlorphenyloxy substituiert sein. Bei R₁ und R₂ in Formel I kann es sich um gleiche oder verschiedene Schutzgruppen handeln.

In einer besonders bevorzugten Ausführungsform stellen R₁ und R₂ als Schutzgruppen Benzyl, Methylbenzyl, Dimethylbenzyl, Methoxybenzyl, Dimethoxybenzyl, halogeniertes Benzyl, insbesondere Brombenzyl; Diphenylmethyl, Di(methylphenyl)methyl, Di(dimethylphenyl)methyl, Di(methoxyphenyl)methyl, Di(methoxyphenyl)(phenyl)methyl, Triphenylmethyl, Tris-4,4',4"-tert.butylphenylmethyl, Di-p-anisylphenylmethyl, Tri(methylphenyl)methyl, Tri(dimethylphenyl)methyl, Tri(methoxyphenyl)methyl, Tri(dimethoxyphenyl)methyl; Trimethylsilyl, Triethylsilyl, Tri-n-propylsilyl, 1-Propyldimethylsilyl, t-Butyl-dimethylsilyl, t-Butyl-diphenylsilyl, n-Octyl-dimethylsilyl, (1,1,2,2-Tetramethylethyl)-dimethylsilyl, -(CH₃)₂Si-O-Si(CH₃)₂-, -(i-C₃H₇)₂Si-O-Si(i-C₃H₇)₂-; Acetyl, Propanoyl, Butanoyl, Pentanoyl, Hexanoyl, Benzoyl, Methylbenzoyl, Methoxybenzoyl, Chlorbenzoyl und Brombenzoyl; Methyl-, Ethyl-, Propyl-, Butyl-, Phenyl-, Benzyl-, p-Brom-, p-Methoxy- und p-Methylphenylsulfonyl; Methoxy-, Ethoxy-, n- oder i-Propoxy- oder n-, i- oder t-Butoxycarbonyl, oder Phenyloxycarbonyl, Benzyloxycarbonyl, Methyl- oder Methoxy- oder Chlorphenyloxycarbonyl oder -benzyloxycarbonyl oder 9-Fluorenylmethyloxycarbonyl dar. Dabei bedeuten R₁ und R₂ vorteilhafterweise gleiche Schutzgruppen.

R₂ kann als phosphorhaltiger eine Nukleotid-Brückengruppe bildender Rest der Formel P1 oder P2 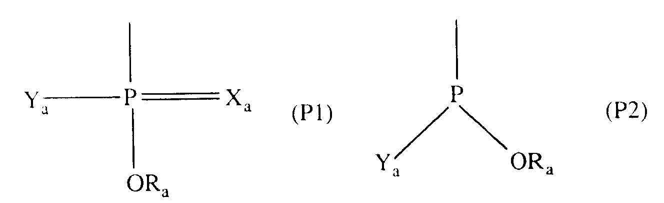 entsprechen, worin Yₐ Wasserstoff, C₁-C₁₂-Alkyl, C₆-C₁₂-Aryl, C₇-C₂₀-Aralkyl, C₇-C₂₀-Alkaryl, -OR_{b}, -SR_{b}, -NH₂, Primäramino, Sekundäramino, O^{⊖}M^{⊕} oder S^{⊖}M^{⊕} darstellt; Xₐ Sauerstoff oder Schwefel bedeutet; Rₐ Wasserstoff, M^{⊕}, C₁-C₁₂-Alkyl, C₂-C₁₂-Alkenyl, C₆-C₁₂-Aryl, oder die Gruppe RₐO- für N-Heteroaryl-N-yl mit 5 Ringgliedern und 1 bis 3 N-Atomen steht; R_{b} Wasserstoff, C₁-C₁₂-Alkyl oder C₆-C₁₂-Aryl bedeutet; und M^{⊕} für Na^{⊕}, K^{⊕}, Li^{⊕} , NH₄^{⊕} steht oder Primär-, Sekundär-, Tertiär- oder Quartenärammonium darstellt; wobei Alkyl, Aryl, Aralkyl und Alkaryl in Yₐ, Rₐ und R_{b} unsubstituiert oder mit Alkoxy, Alkylthio, Halogen, -CN, -NO₂, Phenyl, Nitrophenyl, oder Halogenphenyl substituiert ist.

Yₐ enthält als Primäramino bevorzugt 1 bis 12 und besonders bevorzugt 1 bis 6 C-Atome, und als Sekundäramino bevorzugt 2 bis 12 und besonders bevorzugt 2 bis 6 C-Atome.

Bei dem Primäramino und Sekundäramino kann es sich zum Beispiel um Reste der Formel R_{c}R_{d}N handeln, worin R_{c} für H steht oder unabhängig die Bedeutung von R_{d} hat, und R_{d} C₁-C₂₀-, bevorzugt C₁-C₁₂- und besonders bevorzugt C₁-C₆-Alkyl, C₁-C₂₀-, bevorzugt C₁-C₁₂- und besonders bevorzugt C₁-C₆-Aminoalkyl, C₁-C₂₀-, bevorzugt C₁-C₁₂- und besonders bevorzugt C₁-C₆-Hydroxyalkyl; Carboxyalkyl oder Carbalkoxyalkyl, wobei die Carbalkoxygruppe 2 bis 8 C-Atome enthält und die Alkylgruppe 1 bis 6, bevorzugt 1 bis 4 C-Atome; C₂-C₂₀-, bevorzugt C₂-C₁₂- und besonders bevorzugt C₂-C₆-Alkenyl; Phenyl, Mono- oder Di-(C₁-C₄-Alkyl- oder -Alkoxy)phenyl, Benzyl, Mono- oder Di-(C₁-C₄-Alkyl- oder -Alkoxy)benzyl; oder 1,2-, 1,3- oder 1,4-Imidazolyl-C₁-C₆-Alkyl darstellt, oder R_{c} und R_{d} zusammen Tetra- oder Pentamethylen, 3-Oxa-1,5-pentylen, -CH₂-NRₑ-CH₂CH₂- oder -CH₂CH₂-NR₁₉-CH₂CH₂- darstellen, worin Rₑ für H oder C₁-C₄-Alkyl steht. Die Aminogruppe im Aminoalkyl kann mit ein oder zwei C₁-C₄-Alkyl oder -Hydroxyalkylgruppen substituiert sein. Die Hydroxylgruppe im Hydroxyalkyl kann mit C₁-C₄-Alkyl verethert sein.

Unter Primär-, Sekundär-, Tertiär- und Quartärammonium ist für Yₐ im Zusammenhang mit der Definition von M^{⊕} ein Ion der Formel R_{f}R_{g}RₕRᵢN^{⊕} zu verstehen, bei dem R_{f} C₁-C₂₀-, bevorzugt C₁-C₁₂- und besonders bevorzugt C₁-C₆-Alkyl, -Aminoalkyl, -Hydroxyalkyl; Carboxyalkyl oder Carbalkoxyalkyl, wobei die Carbalkoxygruppe 2 bis 8 C-Atome enthält und die Alkylgruppe 1 bis 6, bevorzugt 1 bis 4 C-Atome; C₂-C₂₀-, bevorzugt C₂-C₁₂- und besonders bevorzugt C₂-C₆-Alkenyl; Phenyl, Mono- oder Di-(C₁-C₄-Alkyl- oder -Alkoxy)phenyl, Benzyl, Mono- oder Di-(C₁-C₄-Alkyl- oder -Alkoxy)benzyl; oder 1,2-, 1,3- oder 1,4-Imidazolyl-C₁-C₆-Alkyl darstellt, und R_{g}, Rₕ und Rᵢ unabhängig voneinander Wasserstoff sind oder die Bedeutung von R_{f} haben, oder R_{f} und R_{g} zusammen Tetra- oder Pentamethylen, 3-Oxa-1,5-pentylen, -CH₂-NRₑ-CH₂CH₂- oder -CH₂CH₂-NRₑ-CH₂CH₂-darstellen, worin Rₑ für H oder C₁-C₄-Alkyl steht, und Rₕ und Rᵢ unabhängig voneinander die Bedeutung von R_{f} haben. Die Aminogruppe im Aminoalkyl kann mit ein oder zwei C₁-C₄-Alkyl oder -Hydroxyalkylgruppen substituiert sein. Die Hydroxylgruppe im Hydroxyalkyl kann mit C₁-C₄-Alkyl verethert sein.

Beispiele für Carboxyalkyl sind Carboxymethyl, Carboxyethyl, Carboxypropyl und Carboxybutyl, und Beispiele für Carbalkoxyalkyl sind diese mit Methyl oder Ethyl veresterten Carboxyalkylgruppen. Beispiele für Alkenyl sind Allyl, But-1-en-3-yl oder -4-yl, Pent-3- oder 4-en-1-yl oder -2-yl, Hex-3- oder -4- oder -5-en-1-yl oder -2-yl. Beispiele für Alkyl- und Alkoxyphenyl beziehungsweise -benzyl sind Methylphenyl, Dimethylphenyl, Ethylphenyl, Diethylphenyl, Methylbenzyl, Dimethylbenzyl, Ethylbenzyl, Diethylbenzyl, Methoxyphenyl, Dimethoxyphenyl, Ethoxyphenyl, Diethoxyphenyl, Methoxybenzyl, Dimethoxybenzyl, Ethoxybenzyl, Diethoxybenzyl. Beispiele für Imidazolylalkyl, in dem die Alkylgruppe bevorzugt 2 bis 4 C-Atome enthält, sind 1,2-, 1,3- oder 1,4-Imidazolylethyl oder -n-propyl oder -n-butyl. R₁₉ stellt bevorzugt H, Methyl oder Ethyl dar.

Bevorzugte Beispiele für Primäramino und Sekundäramino sind Methyl-, Ethyl-, Dimethyl-, Diethyl-, Di-i-Propyl, Mono- oder Di-(1-hydroxy-eth-2-yl)-, Phenyl- und Benzylamino, Acetylamino und Benzoylamino sowie Piperidinyl, Piperazinyl und Morpholinyl.

Bevorzugte Beispiele für Primär- und Sekundärammonium sind Methyl-, Ethyl-, Dimethyl-, Diethyl-, Di-i-Propyl-, Mono- oder Di-(1-hydroxy-eth-2-yl)-, Phenyl- und Benzylammonium.

Beispiele für Yₐ, Rₐ und R_{b} als Alkyl sind Methyl, Ethyl und die Isomeren von Propyl, Butyl, Pentyl, Hexyl, Heptyl und Octyl; Beispiele für Yₐ, Rₐ und R_{b} als Aryl sind Phenyl und Naphthyl; Beispiele für Rₐ als Alkenyl sind Allyl und (C₁-C₄-Alkyl)CH=CH-CH₂-; Beispiele für Yₐ als Aralkyl sind Phenyl-CₙH₂ₙ- mit n gleich einer Zahl von 1 bis 6, besonders Benzyl; Beispiele für Yₐ als Alkaryl sind Mono-, Di- und Tri(C₁-C₄-alkyl)phenyl. Bevorzugte Substituenten sind Chlor, Brom, Methoxy, -NO₂, -CN, 2,4-Dichlorphenyl und 4-Nitrophenyl. Beispiele für R_{b} sind 2,2,2-Trichlorethyl, 4-Chlorphenyl, 2-Chlorphenyl und 2,4-Dichlorphenyl; und Beispiele für R_{b}O- als N-Heteroaryl sind Pyrrol-N-yl, Triazol-N-yl und Benztriazol-N-yl.

In einer besonders bevorzugten Ausführungsform bedeutet Rₐ β-Cyanoethyl und stellt Yₐ Di(i-propylamino) dar.

Wenn B einen Purinrest oder ein Analoges davon darstellt, so kann es sich um einen Rest der Formel II, IIa, IIb, IIc, IId, IIe oder IIf handeln, 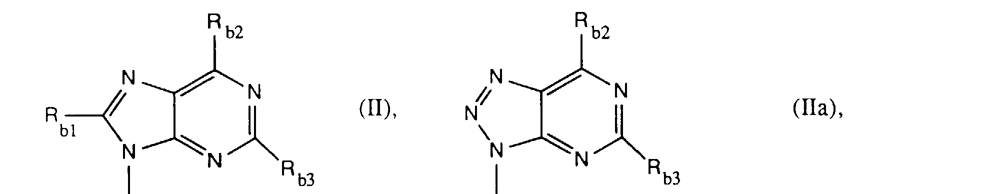 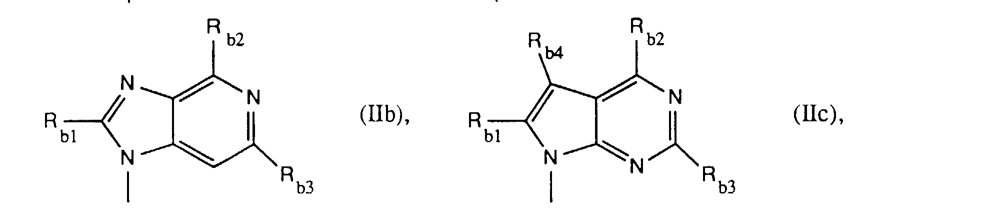 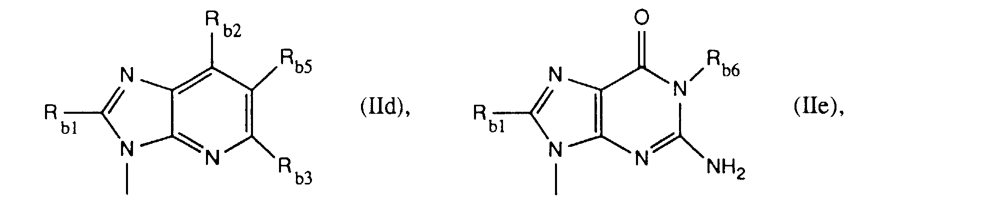 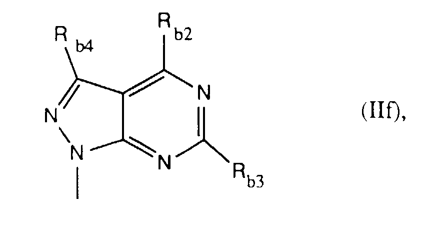 worin R_{b1} für H, Cl, Br, OH oder -O-C₁-C₁₂-Alkyl steht, und R_{b2}, R_{b3} und R_{b5} unabhängig voneinander H, OH, SH, NH₂, NHNH₂, NHOH, NHO-C₁-C₁₂-Alkyl, -N=CH-N(C₁-C₁₂-Alkyl)₂, -N=CH-N-Cycloalkyl, F, Cl, Br, C₁-C₁₂-Alkyl,- Hydroxy-C₁-C₁₂-alkyl, Amino-C₁-C₁₂-alkyl, C₁-C₁₂-Alkoxy, Benzyloxy, C₁-C₁₂-Alkylthio, wobei die Hydroxyl- und Aminogruppen unsubstituiert oder mit einer Schutzgruppe substituiert sind, Phenyl, Benzyl, Primäramino mit 1 bis 20 C-Atomen oder Sekundäramino mit 2 bis 30 C-Atomen bedeuten, R_{b4} Wasserstoff, CN oder -C≡C-R_{b7}, R_{b6} und R_{b7} Wasserstoff oder C₁-C₄-Alkyl darstellen.

Geeignete Schutzgruppen sind zuvor erwähnt worden. Bevorzugte Schutzgruppen sind C₁-C₈-Acylgruppen, wie zum Beispiel Acetyl, Propionyl, Butyroyl und Benzoyl. R_{b6} steht bevorzugt für H oder Methyl.

Das Primäramino enthält bevorzugt 1 bis 12 und besonders bevorzugt 1 bis 6 C-Atome, und das Sekundäramino bevorzugt 2 bis 12 und besonders bevorzugt 2 bis 6 C-Atome.

Einige Beispiele für Alkyl, Alkoxy, Alkylthio, Hydroxyalkyl und Aminoalkyl, die bevorzugt 1 bis 6 C-Atome enthalten, sind Methyl, Ethyl und die Isomeren von Propyl, Butyl, Pentyl, Hexyl, Heptyl, Octyl, Nonyl, Decyl, Undecyl und Dodecyl, sowie entsprechende Alkoxy-, Alkylthio-, Hydroxyalkyl- und Aminoalkylreste. Das Alkyl, Alkoxy, Alkylthio, Hydroxyalkyl und Aminoalkyl enthält besonders bevorzugt 1 bis 4 C-Atome. Bevorzugte Alkyl-, Alkoxy-, Alkylthio-, Hydroxyalkyl- und Aminoalkylreste sind Methyl, Ethyl, n-und i-Propyl, n-, i- und t-Butyl, Methoxy, Ethoxy, Methylthio und Ethylthio, Aminomethyl, Aminoethyl, Hydroxymethyl und Hydroxyethyl.

Bei dem Primäramino und Sekundäramino kann es sich zum Beispiel um Reste der Formel Rₐ₁Rₐ₂N handeln, worin Rₐ₁ für H steht oder unabhängig die Bedeutung von Rₐ₂ hat, und Rₐ₂ C₁-C₂₀-, bevorzugt C₁-C₁₂- und besonders bevorzugt C₁-C₆-Alkyl, -Aminoalkyl, -Hydroxyalkyl; Carboxyalkyl oder Carbalkoxyalkyl, wobei die Carbalkoxygruppe 2 bis 8 C-Atome enthält und die Alkylgruppe 1 bis 6, bevorzugt 1 bis 4 C-Atome; C₂-C₂₀-, bevorzugt C₂-C₁₂- und besonders bevorzugt C₂-C₆-Alkenyl; Phenyl, Mono- oder Di-(C₁-C₄-Alkyl- oder -Alkoxy)phenyl, Benzyl, Mono- oder Di-(C₁-C₄-Alkyl- oder -Alkoxy)benzyl; oder 1,2-, 1,3- oder 1,4-Imidazolyl-C₁-C₆-Alkyl darstellt, oder Rₐ₁ und Rₐ₂ zusammen Tetra- oder Pentamethylen, 3-Oxa-1,5-pentylen, -CH₂-NRₐ₃-CH₂CH₂- oder -CH₂CH₂-NRₐ₃-CH₂CH₂- darstellen, worin Rₐ₃ für H oder C₁-C₄-Alkyl steht. Die Aminogruppe im Aminoalkyl kann mit ein oder zwei C₁-C₄-Alkyl oder -Hydroxyalkylgruppen substituiert sein. Die Hydroxylgruppe im Hydroxyalkyl kann mit C₁-C₄-Alkyl verethert sein.

Beispiele für Alkyl sind zuvor angegeben worden. Beispiele für Aminoalkyl sind Aminomethyl, Aminoethyl, 1-Aminoprop-2-yl oder -3-yl, 1-Amino-but-2-yl oder -3-yl oder -4-yl, N-Methyl- oder N,N-Dimethyl- oder N-Ethyl- oder N,N-Diethyl- oder N-2-Hydroxyethyl- oder N,N-Di-2-hydroxyethylaminomethyl oder -aminoethyl oder -aminopropyl oder -aminobutyl. Beispiele für Hydroxyalkyl sind Hydroxymethyl, 1-Hydroxy-eth-2-yl, 1-Hydroxy-prop-2- oder -3-yl, 1-Hydroxy-but-2-yl, -3-yl oder -4-yl. Beispiele für Carboxyalkyl sind Carboxymethyl, Carboxyethyl, Carboxypropyl und Carboxybutyl, und Beispiele für Carbalkoxyalkyl sind diese mit Methyl oder Ethyl veresterten Carboxyalkylgruppen. Beispiele für Alkenyl sind Allyl, But-1-en-3-yl oder -4-yl, Pent-3-oder 4-en-1-yl oder -2-yl, Hex-3- oder -4- oder -5-en-1-yl oder -2-yl. Beispiele für Alkyl- und Alkoxyphenyl beziehungsweise -benzyl sind Methylphenyl, Dimethylphenyl, Ethylphenyl, Diethylphenyl, Methylbenzyl, Dimethylbenzyl, Ethylbenzyl, Diethylbenzyl, Methoxyphenyl, Dimethoxyphenyl, Ethoxyphenyl, Diethoxyphenyl, Methoxybenzyl, Dimethoxybenzyl, Ethoxybenzyl, Diethoxybenzyl. Beispiele für Imidazolylalkyl, in dem die Alkylgruppe bevorzugt 2 bis 4 C-Atome enthält, sind 1,2-, 1,3- oder 1,4-Imidazolylethyl oder -n-propyl oder -n-butyl. Rₐ₃ stellt bevorzugt H, Methyl oder Ethyl dar.

Bevorzugte Beispiele für Primäramino und Sekundäramino sind Methyl-, Ethyl-, Dimethyl-, Diethyl-, Allyl-, Mono- oder Di-(1-hydroxy-eth-2-yl)-, Phenyl- und Benzylamino, Acetylamino, Isobutyrylamino und Benzoylamino.

In einer bevorzugten Ausführungsform stellt R_{b1} Wasserstoff dar. In einer anderen bevorzugten Ausführungsform stellt R_{b5} Wasserstoff dar. In einer weiteren bevorzugten Ausführungsform bedeuten R_{b2} und R_{b3} unabhängig voneinander H, F, Cl, Br, OH, SH, NH₂, NHOH, NHNH₂, Methylamino, Dimethylamino, Benzoylamino, Isobutyrylamino, Methoxy, Ethoxy und Methylthio.

Einige Beispiele für Analoge der Purinreihe sind neben Purin Xanthin, Hypoxanthin, Adenin, N-Methyladenin, N-Benzoyladenin, 2-Methylthioadenin, 2-Aminoadenin, 6-Hydroxypurin, 2-Amino-6-chlorpurin, 2-Amino-6-methylthiopurin, Guanin, N-Isobutyrylguanin. Besonders bevorzugt sind Adenin, 2-Aminoadenin und Guanin, sowie deren basengeschützte Derivate.

Wenn B in Formel I einen Pyrimidinrest darstellt, so handelt es sich bevorzugt um einen Uracil-, Thymin- oder Cytosinrest der Formel III, IIIa, IIIb oder IIIc 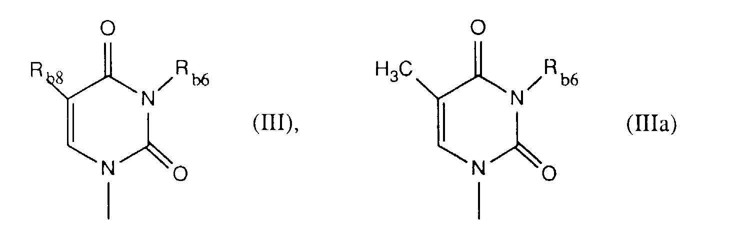 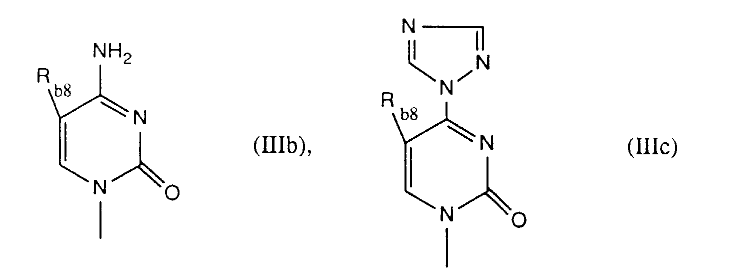 worin R_{b6} H oder C₁-C₄-Alkyl und R_{b8} H, OH, SH, NH₂, NHNH₂, NHOH, NHO-C₁-C₁₂-Alkyl, -N=CH-N(C₁-C₁₂-Alkyl)₂, -N=CH-N-Cycloalkyl, F, Cl, Br, C₁-C₁₂-Alkyl, Hydroxy-C₁-C₁₂-alkyl, Amino-C₁-C₁₂-alkyl, C₁-C₁₂-Alkoxy, Benzyloxy, C₁-C₁₂-Alkylthio, wobei die Hydroxyl- und Aminogruppen unsubstituiert oder mit einer Schutzgruppe substituiert sind, Phenyl, Benzyl, Primäramino mit 1 bis 20 C-Atomen, Sekundäramino mit 2 bis 30 C-Atomen, C₁-C₁₂-Alkenyl oder C₁-C₁₂-Alkinyl bedeuten, und die NH₂-Gruppe in Formel IIIb unsubstituiert oder mit C₁-C₆-Alkyl, Benzoyl oder einer Schutzgruppe substituiert ist, sowie die Dihydroderivate der Reste der Formeln III, IIIa, IIIb und IIIc. Bevorzugt stellt R_{b8} in Formel III H, C₁-C₆-Alkyl oder -Hydroxyalkyl, C₂-C₆-Alkenyl oder -Alkinyl, F, Cl, Br, NH₂, Benzoylamino, Mono- oder Di-C₁-C₆-alkylamino dar. Bevorzugt stellt R_{b8} in Formel IIIb und IIIc H, C₁-C₆-Alkyl oder -Alkoxy oder -Hydroxyalkyl, C₂-C₆-Alkenyl oder -Alkinyl, F, Cl, Br, NH₂, Benzoylamino, Mono- oder Di-C₁-C₆-alkylamino dar.

R_{b6} steht bevorzugt für H oder Methyl. R_{b8} in Formel III bedeutet bevorzugt H, F, Cl, Br, NH₂, NHCH₃, N(CH₃)₂, C₁-C₄-Alkyl, C₂-C₄-Alkenyl oder C₂-C₄-Alkin-1-yl. R_{b8} in Formel IIIb und IIIc stellt bevorzugt H, C₁-C₄-Alkyl, besonders Methyl, C₂-C₄-Alkenyl, besonders Vinyl oder C₂-C₄-Alkin-1-yl, besonders 1-Propin-1-yl, oder NH₂, NHCH₃ oder (CH₃)₂N dar.

Einige Beispiele für Pyrimidinanaloge sind Uracil, Thymin, Cytosin, 5-Fluoruracil, 5-Chloruracil, 5-Bromuracil, Dihydrouracil, 5-Methylcytosin, 5-Propinthymin und 5-Propincytosin.

Ein weiterer Gegenstand der Erfindung ist ein Verfahren zur Herstellung von Verbindungen der Formel I, 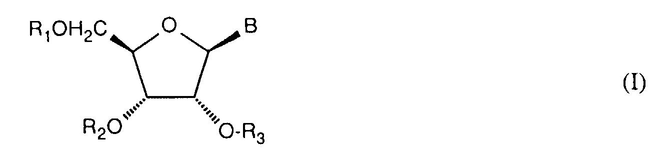 worin R₁ und R₂ unabhängig voneinander für Wasserstoff oder eine Schutzgruppe stehen oder R₁ diese Bedeutungen hat und R₂ für einen eine phosphorhaltige Nukleotid-Brückengruppe bildenden Rest steht; B einen Purin- oder Pyrimidinrest oder ein Analoges davon bedeutet; und
(a) R₃ einen Rest der Formel Ia bedeutet 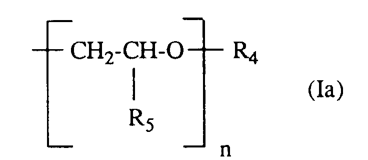 worin R₄ Wasserstoff, C₁-C₂₁-Alkyl, C₂-C₂₁-Alkenyl, C₂-C₂₁-Alkinyl oder -C(=O)-Alkyl;
   R₅ Wasserstoff, C₁-C₁₀-Alkyl, -CH₂-O-R₆ oder einen Rest der Formel Ib; R₆ Wasserstoff, C₁-C₂₂-Alkyl, C₃-C₂₁-Alkenyl, teilweise oder vollständig mit Fluor substituiertes C₁-C₁₀-Alkyl oder -[(CH₂)₂-O]ₘ-R₇; R₇ Wasserstoff oder C₁-C₂₁-Alkyl; n eine Zahl von 1 bis 12; und m eine Zahl von 1 bis 4 bedeuten; wobei R₄ für den Fall n=1 und
   R₅=Wasserstoff nicht Wasserstoff ist,
   das dadurch gekennzeichnet ist, dass man eine Verbindung der Formel IVa 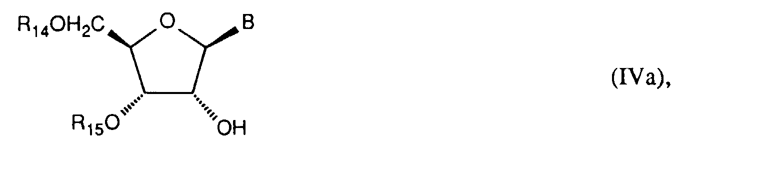 worin R₁₄ und R₁₅ für gleiche oder verschiedene Schutzgruppen stehen und B einen Purin- oder Pyrimidinrest oder ein Analoges davon bedeutet, wobei funktionelle Gruppen im Basenrest B durch Schutzgruppen geschützt sind, in einem inerten Lösungsmittel mit einer Verbindung der Formel A umsetzt 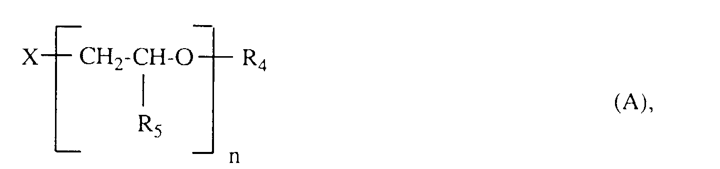 worin R₄, R₅ und n die oben genannten Bedeutungen haben und X Cl, Br, I, Tosyl-O oder Mesyl-O bedeutet;
(b) R₃ einen Rest der Formel Ic bedeutet 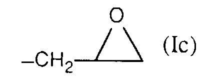 das dadurch gekennzeichnet ist, dass man eine Verbindung der Formel IVa in einem inerten Lösungsmittel mit einer Verbindung der Formel B umsetzt 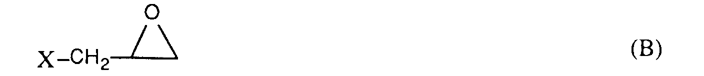 worin X Cl, Br, I, Tosyl-O oder Mesyl-O bedeutet;
(c) R₃ einen Rest der Formel Ib bedeutet 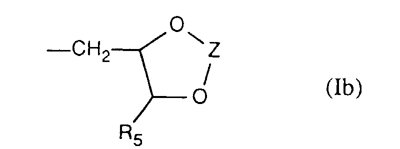 worin R₅ Wasserstoff, C₁-C₁₀-Alkyl, -CH₂-O-R₆ oder einen Rest der Formel Ib; R₆ Wasserstoff, C₁-C₂₂-Alkyl, C₃-C₂₁-Alkenyl, teilweise oder vollständig mit Fluor substituiertes C₁-C₁₀-Alkyl oder -[(CH₂)₂-O]ₘ-R₇; R₇ Wasserstoff oder C₁-C₂₁-Alkyl; Z -(CH₂)ₚ- oder -(CH₂-CH-O)_{q}-CH₂CH₂-, wobei Z im Fall von -CH₂- unsubstituiert oder mit einem oder mehreren C₁-C₁₀-Alkyl, C₅-C₆-Cycloalkyl oder unsubstituiertem oder mit C₁-C₄-Alkyl substituiertem Phenyl unabhängig voneinander substituiert sein kann; m eine Zahl von 1 bis 4; p eine Zahl von 1 bis 10; und q eine Zahl von 1 bis 4 bedeuten; das dadurch gekennzeichnet ist, dass man das in (b) erhaltene Epoxid in Gegenwart von H₂O und BF₃ öffnet und mit einer Verbindung der Formel X'-(CH₂)_{q}-X' oder X'-(CH₂-CH-O)ᵣ-X', worin X' Cl, Br, I, Tosyl-O oder Mesyl-O bedeutet, zu einem neuen Ring schliesst;
(d) R₃ einen Rest der Formel Ia darstellt,
   das dadurch gekennzeichnet ist, dass man das in (b) erhaltene Epoxid in Gegenwart von R₆OH und BF₃ umsetzt und gegebenenfalls die freiwerdende Hydroxylgruppe in einen Ether oder Ester überführt;
(e) R₃ einen Rest der Formel Ia darstellt,
   das dadurch gekennzeichnet ist, dass man das in (b) erhaltene Epoxid in Gegenwart von BF₃ und beispielsweise NaBH₄ hydriert und gegebenenfalls die freiwerdende Hydroxylgruppe in einen Ether oder Ester überführt;
(f) R₃ einen Rest der Formel Ia darstellt,
   das dadurch gekennzeichnet ist, dass man das in (b) erhaltene Epoxid mit einem entsprechenden Grignard-Reagenz umsetzt und gegebenenfalls die freiwerdende Hydroxylgruppe in einen Ether oder Ester überführt; oder
g) R₃ einen Rest der Formel Ia, Ib oder Ic bedeutet,
   das dadurch gekennzeichnet ist, dass man eine Verbindung der Formel IVb 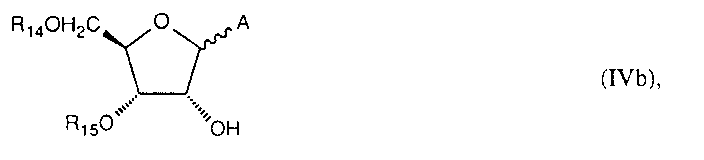 worin R₁₄ und R₁₅ die oben erwähnte Bedeutung haben und A für eine Abgangsgruppe, bevorzugt Alkoxy, Acyloxy, Mesyl-O, Tosyl-O und besonders bevorzugt OCH₃, OCOCH₃ und Benzoyloxy steht, nach einem der in (a) bis (f) beschriebenen Verfahren an der 2'-OH Gruppe substituiert und dann in an sich bekannter Weise den Basenrest B durch Substitution einführt [E. Lukevics, A. Zablocka, Nucleoside Synthesis, Ellis Horwood, New York (1991)]; gegebenenfalls die Schutzgruppen R₁₄ und R₁₅ abspaltet und den die phosphorhaltige Nukleotid-Brückengruppe bildenden Rest einführt.

Verbindungen der Formel I mit R₅ gleich Wasserstoff und n=1 erhält man auch durch Umsetzen einer Verbindung der Formel IVa mit einer Verbindung der Formel X-CH₂-CO-OR, wenn man anschliessend die CO-Gruppe zu einer CH₂OH-Gruppe reduziert und diese dann alkyliert.

Die Verbindungen der Formeln IVa und IVb, A und B sind bekannt, teilweise käuflich oder nach bekannten beziehungsweise analogen Verfahren herstellbar.

Inerte Lösungsmittel sind zum Beispiel Kohlenwasserstoffe, Halogenkohlenwasserstoffe, alkylierte Carbonsäureamide und Lactame, Ether, Nitrile wie Acetonitril, Dialkylsulfone oder -sulfoxide oder cyclische Sulfone und Sulfoxide.

Die Reaktionstemperaturen bei den Verfahrensstufen (a) bis (g) liegen zwischen -50 bis 200°C, bevorzugt zwischen 0 bis 90°C.

Die Umsetzungen werden vorteilhaft in Gegenwart von Basen durchgeführt, zum Beispiel Alkalimetallhydriden, -alkoholaten, -hydroxiden, -carbonaten, Trialkylaminen oder Diazabicycloundecen.

Die Reduktion kann zum Beispiel katalytisch oder mit Borhydriden vorgenommen werden.

Die Isolierung der Verbindungen der Formel I und deren Reinigung erfolgt nach an sich bekannten Verfahren wie zum Beispiel Fällung oder Kristallisation und Filtration und chromatographischen Methoden.

Aus den Verbindungen der Formel I können Oligonukleotide aufgebaut werden, die auf Grund ihrer Wechselwirkung mit Nukleinsäuren wertvolle biologische Aktivitäten aufweisen, und als pharmazeutische Wirkstoffe oder als Diagnostika verwendet werden können.

Ein weiterer Gegenstand der Erfindung ist die Verwendung der Verbindungen der Formel I zur Herstellung von Oligonukleotiden, die gleiche oder verschiedene Monomereinheiten von Verbindungen der Formel I enthalten, jedoch mindestens eine Monomereinheit von Verbindungen der Formel I in Kombination mit Monomereinheiten von anderen natürlichen oder synthetischen Nukleosiden, wobei die Oligonukleotide 2 bis 200 Monomereinheiten enthalten. Bevorzugt enthalten die Oligonukleotide 2 bis 100, besonders bevorzugt 2 bis 50, und insbesondere bevorzugt 4 bis 30 Monomereinheiten. Bevorzugt sind Oligonukleotide, die gleiche oder verschiedene Monomereinheiten von Verbindungen der Formel I enthalten. Bevorzugt sind ferner Oligonukleotide, die zusätzlich Monomereinheiten von synthetischen oder natürlichen Nukleosiden enthalten, die sich von D-Ribose oder 2-Desoxyribose ableiten.

Ein weiterer Gegenstand der Erfindung sind Oligonukleotide der Formel V

5'-U-(O-Y-O-V-)ₓO-Y-O-W-3' (V),

worin x eine Zahl von 0 bis 200 und Y eine Nukleotid-Brückengruppe bedeuten, U, V und W je für sich gleiche oder verschiedene Reste von natürlichen oder synthetischen Nukleosiden darstellen und mindestens einer der Reste U, V und/oder W einen Rest der Formel VI 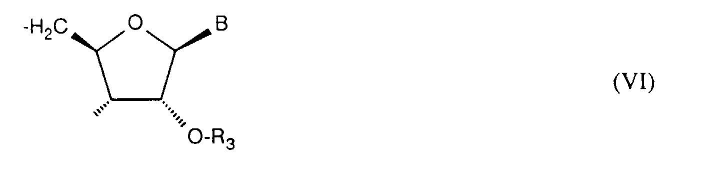 bedeutet, und B und R₃ die für die Verbindungen der Formel I angegebenen Bedeutungen haben einschliesslich der Bevorzugungen und Beispiele.

Eine bevorzugte Brückengruppe Y ist die in natürlichen Oligonukleotiden vorkommende Gruppe -P(O)O^{⊖}-. Beispiele für weitere Brückengruppen sind -P(O)S^{⊖}-, -P(S)S^{⊖}-, -P(O)R₁₆-, P(O)NR₁₇R₁₈, oder -CH₂-, worin R₁₆ H oder C₁-C₆-Alkyl darstellt und R₁₇ und R₁₈ unabhängig voneinander die Bedeutung von R₁₆ haben. In Formel V steht x bevorzugt für eine Zahl von 0 bis 100, besonders bevorzugt für eine Zahl von 1 bis 50 und insbesondere bevorzugt für eine Zahl von 3 bis 29. Die Reste der Formel VI können endständig oder in der Nukleotidsequenz gebunden sein, wobei alle oder mehrere, zum Beispiel 2 bis 5 Reste der Formel VI aufeinander folgen können, oder die Reste der Formel VI können zwischen Resten von natürlichen oder synthetischen Nukleosiden gebunden sein, oder es können Mischformen dieser Verteilungen in der Nukleotidsequenz vorliegen.

Eine ganz besonders bevorzugte Ausführungsform sind Oligonukleotide der Formel V, worin x eine Zahl von 2 bis 50, bevorzugt 2 bis 30 darstellt, Y für die Gruppe -P(O)O^{⊖}-steht, U, V und W je für sich gleiche oder verschiedene Reste eines natürlichen Nukleosids bedeuten und mindestens einer der Reste U, V oder W der Formel VI entspncht. Als natürliche Nukleoside kommen Adenosin, Cytidin, Guanosin, Uridin, 2-Aminoadenin, 5-Methylcytosin, 2'-Desoxyadenosin, 2'-Desoxycytidin, 2'-Desoxyguanosin und Thymidin in Frage. Als natürliche Nukleosidbasen sind besonders Adenin, Cytosin, Guanin, Thymin und Uracil zu nennen. Die Reste der Formel VI können endständig oder in der Nukleotidsequenz gebunden sein, wobei alle oder mehrere, zum Beispiel 2 bis 5 gleiche oder verschiedene Reste der Formel VI aufeinander folgen können, oder gleiche oder verschiedene Reste der Formel VI zwischen Resten von natürlichen Nukleosiden gebunden sind, oder Mischformen dieser Verteilungen in der Nukleotidsequenz vorliegen. In einer anderen bevorzugten Ausführungsform von Oligonukleotiden der Formel V entsprechen sämtliche Reste U, V und W gleichen oder verschiedenen Resten der Formel VI. Bevorzugt stellt x eine Zahl von 3 bis 29 dar und bevorzugt sind insgesamt 1 bis 12 Reste der Formel VI enthalten.

Die Herstellung der erfindungsgemässen Oligonukleotide kann in an sich bekannter Weise nach verschiedenen Verfahren in gegebenenfalls automatisierten und zusammen mit Verfahrensvorschriften käuflichen DNA-Synthesizern erfolgen. Im Falle der Brückengruppe -P(O)O^{⊖}- kann zum Beispiel das Phosphortriesterverfahren, das Phosphittriesterverfahren oder das H-Phosphonatverfahren angewendet werden, die dem Fachmann geläufig sind. Beim Phosphittriesterverfahren kann man zum Beispiel so vorgehen, dass man die Nukleoside der Formel I, worin R₁ und R₂ je H bedeuten, mit einem Schutzgruppenreagenz, zum Beispiel 4,4'-Dimethoxytriphenylmethyl-chlorid zu einem Nukleosid der Formel D umsetzt 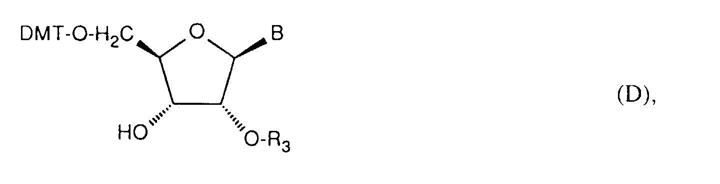 und die Verbindung der Formel D mit Hilfe eines "linkers", zum Beispiel Bernsteinsäureanhydrid, an ein festes Trägermaterial bindet, zum Beispiel an Controlled Pore Glass (CPG), das langkettige Alkylaminogruppen enthält. In einem separaten Verfahren wird die Hydroxylgruppe der Verbindung der Formel D derivatisiert, zum Beispiel zu einem Phosphoramidit unter Verwendung von R'OP[N(i-Propyl)₂)]₂ zu einer Verbindung der Formel E 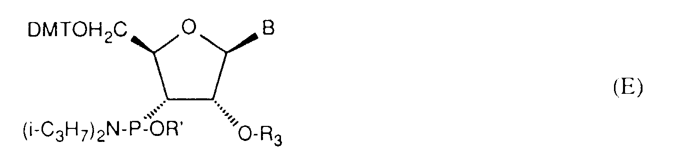 wobei R' zum Beispiel β-Cyanoethyl darstellt.

Nach dem Abspalten der Schutzgruppe wie zum Beispiel der DMT-Gruppe des an den Träger gebundenen Materials koppelt man unter Abspaltung von -N(i-C₃H₇)₂ mit der Verbindung der Formel D, blockiert eventuell vorhandene freie Hydroxylgruppen (capping) und oxidiert dann das gebildete Phosphit zum Phosphat. Nach dem Entschützen des Dimeren wiederholt man den Reaktionszyklus mit einer Verbindung der Formel E, bis man ein Oligomer mit der gewünschten Anzahl an Monomereinheiten synthetisiert hat, und löst das Produkt vom Trägermaterial ab. Auf diese Weise erhält man Oligonukleotide, in denen sämtliche Reste U, V und W gemäss Formel V aus Resten der Formel VI bestehen. Auf diese Weise sind auch Oligonukleotide mit beliebigen Monomereinheiten in beliebiger Sequenz herstellbar, je nach Verwendung synthetischer, natürlicher und erfindungsgemässer Nukleosidbausteine in den einzelnen Reaktionszyklen.

Die erfindungsgemässen Verbindungen der Formel I, worin R₁ und R₂ je H bedeuten, weisen antivirale und antiproliferative Eigenschaften auf und können demgemäss als Arzneimittel Verwendung finden. Die erfindungsgemässen Oligonukleotide weisen zudem eine hohe Stabilität gegenüber einem Abbau durch Nukleasen auf. Besonders überraschend ist ihre ausgezeichnete Paarung mit komplementären Nukleinsäuresträngen, vor allem vom RNA-Typ. Zusätzlich zeigen sie eine unerwartet hohe zelluläre Aufnahme. Die erfindungsgemässen Oligonukleotide eignen sich daher besonders für die Antisense-Technologie, das heisst zur Inhibition der Expression unerwünschter Proteinprodukte durch die Bindung an geeignete komplementäre Nukleotidsequenzen von mRNA (EP 266,099, WO 87/07300 und WO 89/08146). Sie können zur Behandlung von Infektionen und Krankheiten zum Beispiel durch die Blockierung der Expression von bioaktiven Proteinen auf der Stufe der Nukleinsäuren (zum Beispiel Onkogene) eingesetzt werden. Die erfindungsgemässen Oligonukleotide eignen sich auch als Diagnostika und können als Gensonden zum Nachweis von viralen Infektionen oder genetisch bedingten Krankheiten durch selektive Interaktion auf der Stufe von einzel- oder doppelsträngigen Nukleinsäuren verwendet werden ("gene probes"). Im besonderen - bedingt durch die erhöhte Stabilität gegenüber Nukleasen - ist eine diagnostische Anwendung nicht nur in vitro sondern auch in vivo (zum Beispiel Gewebeproben, Blutplasma und Blutserum) möglich. Solche Verwendungsmöglichkeiten sind zum Beispiel in der WO 91/06556 beschrieben.

Ein weiterer Gegenstand der Erfindung betrifft die Verwendung der erfindungsgemässen Oligonukleotide als Diagnostika zum Nachweis von viralen Infektionen oder genetisch bedingten Krankheiten.

Ein anderer Gegenstand der Erfindung betrifft auch die erfindungsgemässen Nukleoside der Formeln I sowie der Oligonukleotide der Formel V zur Anwendung in einem therapeutischen Verfahren zur Behandlung von Krankheiten bei Warmblütern einschliesslich des Menschen durch Inaktivierung von Nukleotidsequenzen im Körper. Die Dosierung bei Verabreichung an Warmblüter von etwa 70 kg Körpergewicht kann zum Beispiel 0,01 bis 1000 mg pro Tag betragen. Die Verabreichung erfolgt vorzugsweise in Form pharmazeutischer Präparate parenteral, zum Beispiel intravenös oder intraperitoneal.

Ein weiterer Gegenstand der Erfindung betrifft ein pharmazeutisches Präparat, enthaltend eine wirksame Menge eines Nukleosids der Formeln I oder eines Oligonukleotids der Formeln V alleine oder zusammen mit anderen Wirkstoffen, ein pharmazeutisches Trägermaterial vorzugsweise in einer signifikanten Menge und gegebenenfalls Hilfsstoffe.

Man kann die pharmakologisch wirksamen erfindungsgemässen Nukleoside und Oligonukleotide in Form von parenteral verabreichbaren Präparaten oder von Infusionslösungen verwenden. Solche Lösungen sind vorzugsweise isotonische wässrige Lösungen oder Suspensionen, wobei diese zum Beispiel bei lyophilisierten Präparaten, welche die Wirksubstanz allein oder zusammen mit einem Trägermaterial, zum Beispiel Mannit, enthalten, vor Gebrauch hergestellt werden können. Die pharmazeutischen Präparate können sterilisiert sein und/oder Hilfsstoffe, zum Beispiel Konservier- Stabilisier-, Netz- und/oder Emulgiermittel, Löslichkeitsvermittler, Salze zur Regulierung des osmotischen Drucks und/oder Puffer enthalten. Die pharmazeutischen Präparate, die gewünschtenfalls weitere pharmakologisch wirksame Stoffe wie zum Beispiel Antibiotka enthalten können, werden in an sich bekannter Weise, zum Beispiel mittels konventioneller Lösungs- oder Lyophilisierungsverfahren, hergestellt, und enthalten etwa 0,1 % bis 90 %, insbesondere von etwa 0,5 % bis etwa 30 %, zum Beispiel 1 % bis 5 % Aktivstoff(e).

Die nachfolgenden Beispiele erläutern die Erfindung. Den ¹H-NMR-Spektren liegt die Nummerierung der Kohlenstoffatome in den folgenden cyclischen Kohlenstoffgerüsten zu Grunde:
Ausgangsverbindungen:

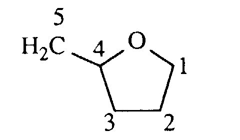

Nukleoside (Beispiele):

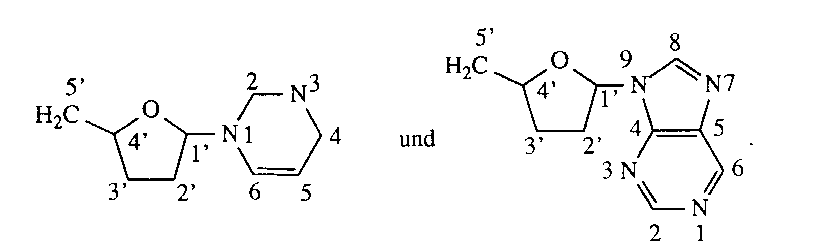

Verwendete Abkürzungen im Text und in den Formeln:
- DMF: Dimethylformamid
- ClBnCl₂: 2,4-Dichlorbenzylchlorid
- Bn: Benzyl
- Ac: Acetyl
- φ: Phenyl
- BSA: N,N-Bistrimethylsilylacetamid
- DBU: Diazabicyclo[5.4.0]undec-7-en
- BOM-Cl: Benzyloxymethylchlorid
- DMTCl: 4,4'-Dimethoxytritylchlorid
- THF: Tetrahydrofuran

### A) Herstellung von Nukleosid-Analogen

Beispiel A1: Zu einer Vorlage von 13,5 g NaH in 130 ml DMF werden bei 60°C 28,0 g 1-Methylribose zugetropft. Nach Ende der H₂-Entwicklung werden 110,0 g ClBnCl₂ zugetropft. Das Reaktionsgemisch wird 16 Stunden bei 25°C weitergerührt. Um noch vorhandenes NaH zu zerstören tropft man vorsichtig Methanol zu und giesst das Reaktionsgemisch dann auf Eis/Wasser. Der klumpige Niederschlag wird abfiltriert und mit Acetonitril gut nachgewaschen. Man erhält die Verbindung (A1). 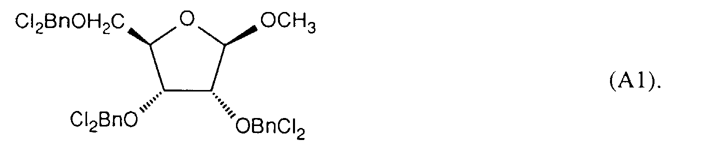 ¹H-NMR (250 MHz, CDCl₃): Das H-C(1)-Proton erscheint bei 5,0 ppm als Singulett.
MS: 638 (M⁺)

Beispiel A2: 65,9 g des in Beispiel A1 hergestellten Produktes werden in 600 ml Methylenchlorid gelöst und auf 0°C gekühlt. Dann tropft man 121 ml SnCl₄ in 800 ml Methylenchlorid zu und lässt bei 3°C stehen. Nach 26 Stunden werden nochmals 2 ml SnCl₄ zugegeben. Nach total 35 Stunden wird die Reaktionslösung vorsichtig auf 700 ml einer gesättigten NaHCO₃-Lösung gegossen. Nach Verdünnung mit 400 ml Methylenchlorid wird der Sn-haltige Niederschlag abfiltriert. Die organische Phase des Filtrats wird mit MgSO₄ getrocknet und zur Verbindung (A2) eingedampft. 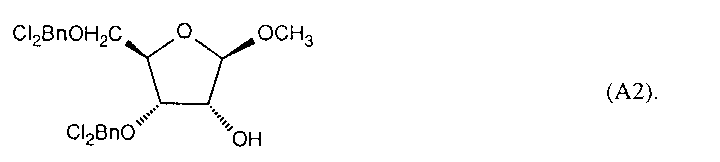 ¹H-NMR (250 MHz, CDCl₃): Das H-C(1)-Proton erscheint bei 4,90 ppm als Doublett mit J=5 Hz.

Beispiel A3: 125,9 g des in Beispiel A2 erhaltenen Produktes werden in 1 l Pyridin gelöst. Bei 20°C werden 25,5 g Acetanhydrid und 1 g 4-Dimethylaminopyridin zugegeben. Anschliessend wird 17 Stunden gerührt. Das Reaktionsgemisch wird in 1 l Wasser aufgenommen, mit konzentrierter Salzsäure angesäuert und mit Essigsäureethylester extrahiert. Der Extrakt wird mit MgSO₄ getrocknet und eingedampft. Schliesslich wird der Rückstand mit Hexan zur Kristallisation gebracht. Man erhält die Verbindung (A3). 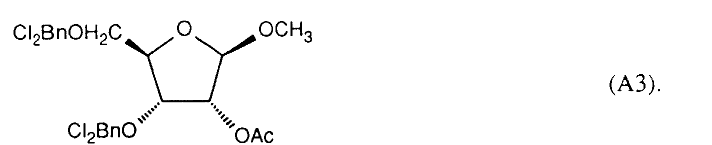 ¹H-NMR (250 MHz, CDCl₃): 5,15 [d, J=4,5 Hz,H-C(1)]; 3,50 (s,OCH₃); 2,17 (s, OCOCH₃);
MS: 522 (M⁺)
[α]_{Na(D)}=87,4±1,0°, CHCl₃ (0,998%)

Beispiel A4: 24 g Thymin werden in 100 ml 1,2-Dichlorethan aufgeschlämmt. Nach Zugabe von 116,4 g BSA wird unter Rückfluss erhitzt bis eine klare Lösung entsteht. Danach wird auf 50°C abgekühlt und es werden 50 g des in Beispiel A3 hergestellten Produktes sowie 27,5 g Trifluormethansulfonsäuretrimethylsilylester zugegeben. Es wird während 20 Stunden bei 70°C gerührt und anschliessend auf 300 ml NaHCO₃-Lösung gegossen und gerührt. Nach Extraktion mit Dichlorethan wird mit MgSO₄ getrocknet und eingedampft. Schliesslich wird der Rückstand mit Methanol zur Kristallisation gebracht. Man erhält die Verbindung (A4). 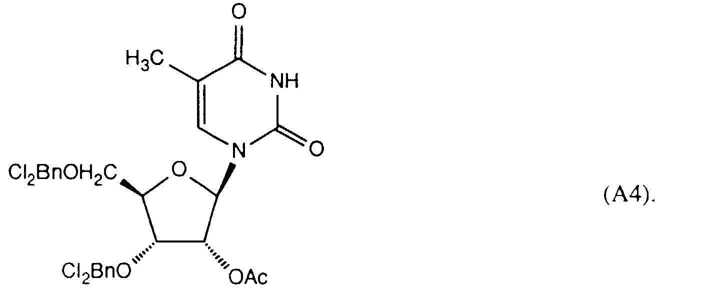 ¹H-NMR (250 MHz, CDCl₃): 8,25 (s, NH; 6,10 [d, J=4,5 Hz, H-C(1')]; 2,13 (s,OCOCH₃); 1,66 (s,CH₃)
MS: 616 (M⁺)

Beispiel A5: 85 g des in Beispiel A4 hergestellten Produktes werden in 850 ml Acetonitril suspendiert. Es werden bei Raumtemperatur 24,2 g DBU und 24,9 g BOM-Cl zugetropft. Nach 20 Stunden Rühren wird das Reaktionsgemisch auf Wasser gegossen und mit Essigsäureethylester extrahiert. Der Extrakt wird mit MgSO₄ getrocknet und eingedampft. Man erhält die Verbindung (A5). 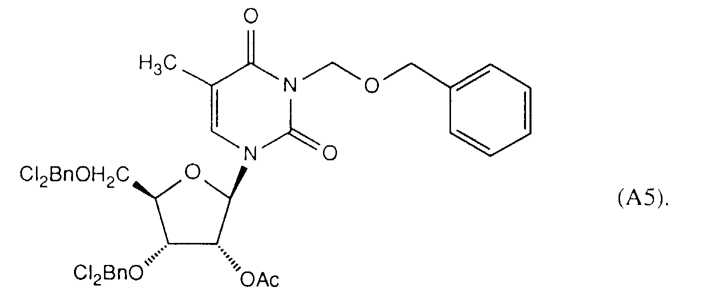 ¹H-NMR (250 MHz, CDCl₃): 6,05 [d, J=4,5 Hz, H-C(1')]; 5,5 (AB, CH₂); 5,37 [dd, H-C(2')]; 2,13 (s, OCOCH₃); 1,55 (s, CH₃)
MS: 736 (M⁺)

Beispiel A6: 106 g des in Beispiel A5 hergestellten Produktes werden in 1 l THF suspendiert. Es werden 26 g einer 30 %igen NaOCH₃/CH₃OH-Lösung zugetropft. Nach 2,5 Stunden Rühren wird die Reaktionslösung auf Wasser gegossen, mit gesättigter wässriger Kochsalzlösung versetzt und mit Essigsäureethylester extrahiert. Nach Trocknung mit MgSO₄ wird der Extrakt eingedampft. Man erhält die Verbindung (A6). 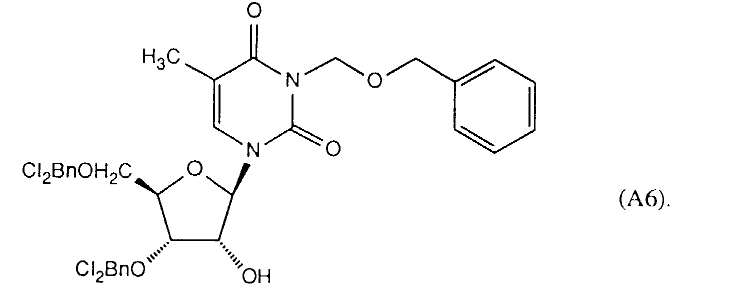 ¹H-NMR (250 MHz, CDCl₃): 5,93 [d, J=5 Hz, H-C(1')]; 5,5 (AB, CH₂); 3,03 (d, J=6,5 Hz, OH); 1,72 (s, CH₃)
MS: 694 (M⁺)

Beispiel A7: 20,3 g des in Beispiel A6 erhaltenen Produktes werden in 200 ml THF gelöst. Nach Zugabe von 0,73 g NaH wird 30 min gekocht. Anschliessend werden 2,89 g 2-Chlorethylmethylether zugegeben und es wird 24 Stunden weitergekocht. Anschliessend werden nochmals 0,5 g NaH und 1,7 g 2-Chlorethylmethylether zugegeben und weitergekocht. Nach insgesamt 32 Stunden wird das Reaktionsgemisch auf Wasser gegossen und mit Essigsäureethylester extrahiert. Der Extrakt wird mit MgSO₄ getrocknet und eingedampft. Der Rückstand wird an Kieselgel mit Toluol/Essigsäureethylester (4:1) chromatographiert. Man erhält die Verbindung (A7). 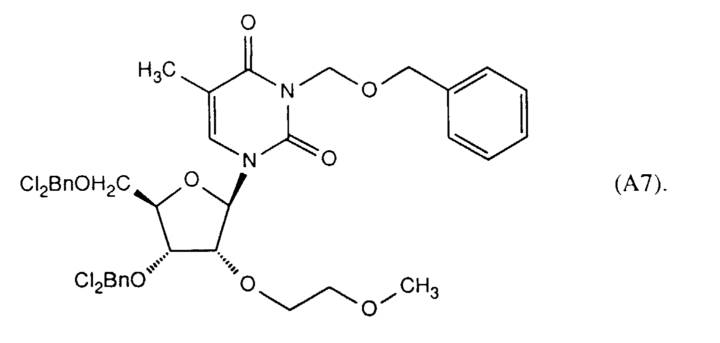 ¹H-NMR (250 MHz, CDCl₃): 7,65 [s, H-C(6)]; 5,93 [s,H-C(1')]; 5,5 (s, CH₂); 3,33 (s, OCH₃); 1,6 (s, CH₃).
MS: 752 (M⁺).

Beispiel A8: 79,4 g des in Beispiel A6 erhaltenen Produktes werden in 800 ml THF gelöst. Nach Zugabe von 3,3 g NaH wird kurz aufgekocht und anschliessend werden bei 40°C 21 g Bromessigsäuremethylester zugetropft. Das Reaktionsgemisch wird insgesamt 27 Stunden bei 60°C gerührt, wobei nach 16 Stunden und nach 20 Stunden je 1 g NaH und 2 ml Bromessigsäuremethylester zugegeben werden. Schliesslich wird das Reaktionsgemisch auf Wasser gegossen und mit Essigsäureethylester extrahiert. Der Extrakt wird mit MgSO₄ getrocknet und eingedampft. Dabei erhält man die Verbindung (A8). 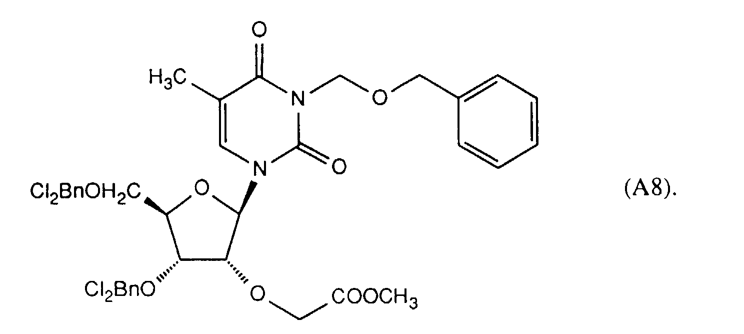 ¹H-NMR (250 MHz, CDCl₃): 7,70 [s, H-C(6)]; 5,92 [s, H-C(1')]; 5,48 (AB, CH₂); 3,75 (s, OCH₃); 1,58 (s, CH₃).
MS: 766 (M⁺).

### Beispiel A9:

a) 37 g des gemäss Beispiel A8 gewonnenen Produktes werden in 400 ml THF gelöst. Man gibt bei 20°C portionsweise 1,5 g LiBH₄ zu und rührt 1 Stunde lang. Danach giesst man das Reaktionsgemisch vorsichtig auf 500 ml Wasser und neutralisiert mit 32 ml 2 N wässriger Salzsäure. Nach Extraktion mit Essigsäureethylester und Eindampfen erhält man die Verbindung (A9). 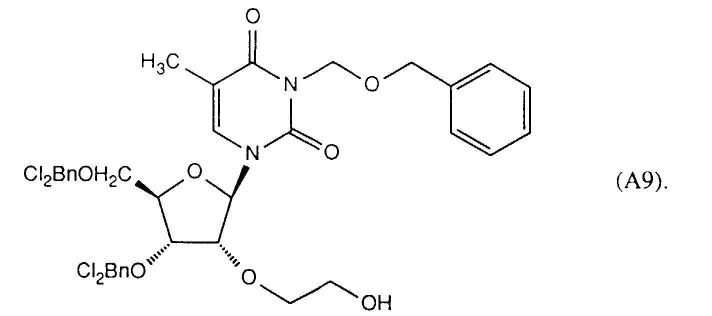 ¹H-NMR (250 MHz, CDCl₃): 7,65 [s,H-C(6)]; 5,96 [s,H-C(1')]; 5,50 (AB, CH₂); 2,57 (breites s, OH); 1,60 (s, CH₃).
   MS: 738 (M⁺).
b) Die Verbindung (A9) wird mit CH₃J in Gegenwart von NaH in THF bei 70°C methyliert. Nach 8 Stunden wird wie in Beispiel A7 beschrieben aufgearbeitet. Man erhält die Verbindung (A7).

Beispiel A10: 20,0 g des in Beispiel A7 hergestellten Produktes werden in 200 ml THF gelöst und über 2 g Pd/C (5%) bei 25°C und unter Normaldruck 4,5 Stunden hydriert (H₂-Aufnahme 102 %). Nach Filtration und Eindampfen des Filtrats wird der Rückstand in 170 ml Methanol gelöst und mit einer 30%igen NaOCH₃/CH₃OH-Lösung auf einen pH-Wert von 11 eingestellt. Nach 24 Stunden giesst man auf 250 ml Wasser, säuert mit 2 N wässriger Salzsäure an und extrahiert mit Essigsäureethylester. Der Extrakt wird mit MgSO₄ getrocknet und eingedampft. Man erhält die Verbindung (A10). 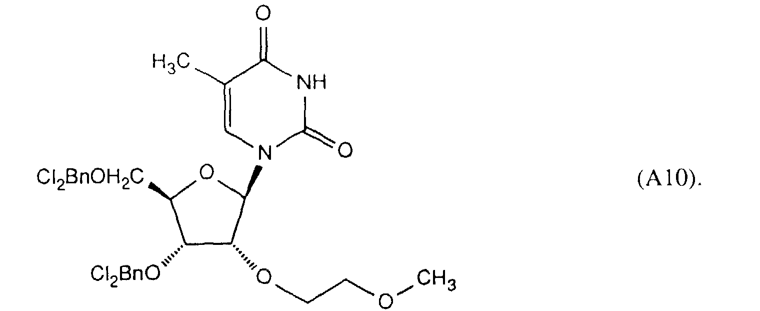 ¹H-NMR (250 MHz, CDCl₃): 8,75 (s, NH); 7,65 [s, H-C(6)]; 5,97 [s, H-C(1')]; 3,33 (s, OCH₃); 1,60 (s, CH₃).
MS: 632 (M⁺).

Beispiel A11: 15 g des in Beispiel A10 hergestellten Produktes werden in 250 ml Methanol in Gegenwart von 9,25 g wasserfreiem Natriumacetat über 5 g Pd/C (5%) bei 50°C und unter Normaldruck hydriert. Nach 46 Stunden wird das Hydriergemisch filtriert und eingedampft. Der Rückstand wird zur Entfernung von Salzen über eine kleine Fritte mit Kieselgel chromatographiert (Essigsäureethylester/Methanol 9:1). Man erhält die Verbindung (A11). 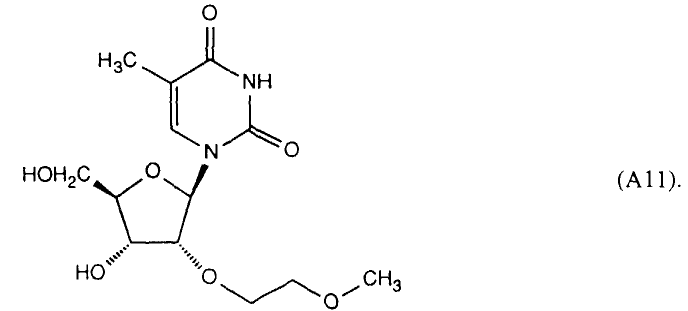 ¹H-NMR (250 MHz, CDCl₃): 11,5 (s, NH); 7,95 [s, H-C(6)]; 6,00 [d, J=6 Hz, H-C(1')]; 5,33 (breites s, OH); 5,20 (d, OH); 3.32 (s, OCH₃); 1,92 (s, CH₃).
MS: 316 (M⁺).

Beispiel A12: 0,45 g des in Beispiel A11 hergestellten Produktes werden zweimal in Pyridin aufgenommen und eingedampft. Man nimmt erneut in 18 ml Pyridin auf und gibt nun der Reihe nach zu: 0,2 g Triethylamin, 0,55 g DMTCl und 25 mg 4-Dimethylamino-pyridin. Nach 16 Stunden Rühren bei Raumtemperatur wird das Reaktionsgemisch mit 50 ml Essigsäureethylester verdünnt und auf 50 ml Wasser gegossen. Die organische Phase wird mit MgSO₄ getrocknet und eingeengt. Den Rückstand chromatographiert man über Kieselgel (Hexan/Essigsäureethylester/Triethylamin 86:10:4). Man erhält die Verbindung (A12). 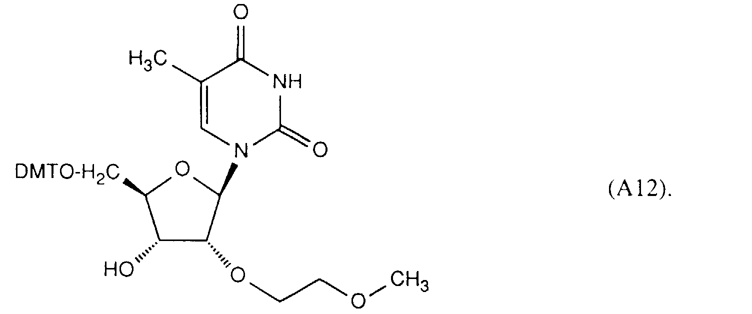 ¹H-NMR (250 MHz, CDCl₃): 7,62 [s, H-C(6)]; 6,02 [d, J=4 Hz, H-C(1')]; 3.38 (s, OCH₃); 1,36 (s, CH₃).

Beispiel A13: 330 mg des in Beispiel A12 erhaltenen Produktes werden zu einer Vorlage aus 110 mg Diisopropylammoniumtetrazolid, 178 mg 2-Cyanoethyl-N,N,N',N'-tetraisopropylphosphordiamidit und 6 ml Methylenchlorid gegeben. Das Reaktionsgemisch wird 17 Stunden bei Raumtemperatur gerührt und danach auf eine gesättigte wässrige NaHCO₃-Lösung gegossen. Die organische Phase wird mit MgSO₄ getrocknet und eingedampft. Den Rückstand chromatographiert man über Kieselgel (Ethanol/Essigsäureethylester 4:1 mit 1 % Zusatz von Triethylamin). Der erhaltenen Schaum wird in 1 ml Methyl-t-butylether gelöst und bei 0°C in Pentan getropft. Man erhält die Verbindung (A13) (Diastereoisomere, 1:1). 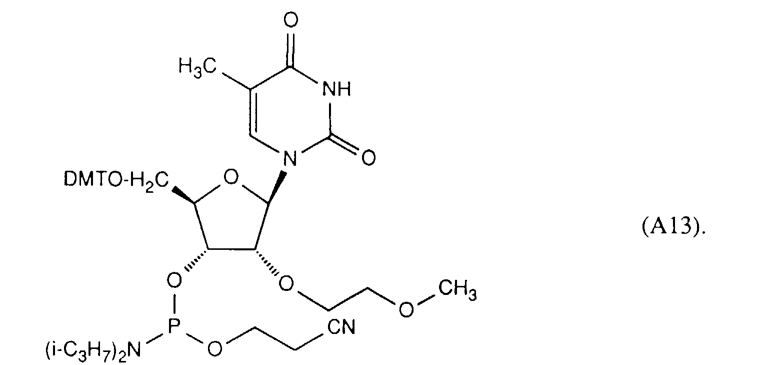 ¹H-NMR (250 MHz, CDCl₃): 7,70 [s, H-C(6)] und 7,63 [s, H-C(6)]; 6,08 [d, J=4 Hz, H-C(1)]; 6,02 [d, J=4 Hz, H-C(1')].

Beispiel 14: 24,2 g des in Beispiel A2 erhaltenen Produktes werden in 250 ml THF gelöst. Es werden 8,76 g Bromessigsäuremethylester und 1,38 g NaH zugegeben, und nun rührt man das Reaktionsgemisch 3,5 Stunden bei 60°C. Nach erneuter Zugabe von 0,14 g NaH und 0,53 ml Bromessigsäuremethylester wird weiter 3 Stunden bei 60°C gerührt. Danach giesst man die Suspension auf 300 ml Wasser, neutralisiert mit 2 N wässriger Salzsäure und extrahiert mit Essigsäureethylester. Der Extrakt wird über MgSO₄ getrocknet und eingedampft. Man erhält die Verbindung (A14). 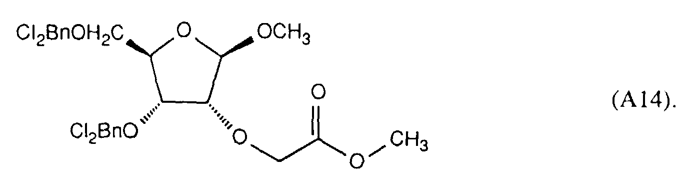 ¹H-NMR (250 MHz, CDCl₃): 5,05 [d, J=4 Hz, H-C(1')]; 3,50 [s, OCH₃] und 3,75 [s, OCH₃].
MS: 552 (M⁺).

Beispiel A15: 5,0 g des in Beispiel A14 erhaltenen Produktes werden in 60 ml Acetonitril gelöst. Nun gibt man 4,88 g bisilyliertes Thymin zu und tropft unter Rühren bei 60°C 2,6 g Trifluormethansulfonsäuretrimethylsilylester ein. Nach 3,5 Stunden Rühren wird die Reaktionslösung abgekühlt, auf eine gesättigte wässrige NaHCO₃-Lösung gegossen und verrührt. Nun wird mit Essigsäureethylester extrahiert, die organische Phase mit MgSO₄ getrocknet und eingedampft. Der Rückstand wird über Kieselgel chromatographiert (Toluol/Essigsäureethylester 1:1). Man erhält die Verbindung (A15). 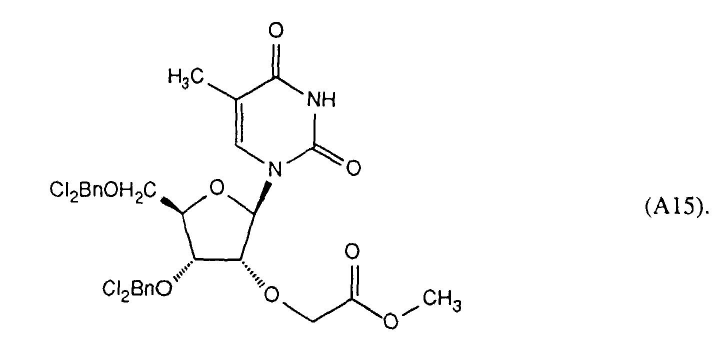 ¹H-NMR (250 MHz, CDCl₃): 8,36 (s, NH); 7,70 [s, H-C(6)]; 5,94 [d, J=1,5Hz, H-C(1')]; 3,73 (s, OCH₃); 1,58 [s, CH₃].
MS: 646 (M⁺).
b) Die Umsetzung mit Benzyloxymethylchlorid ergibt die Verbindung (A8).

Beispiel A16: Analog der vorstehenden Arbeitsvorschriften werden weitere 2'-OH-Modifikationen vorgenommen und in Tabelle 1 aufgeführt.

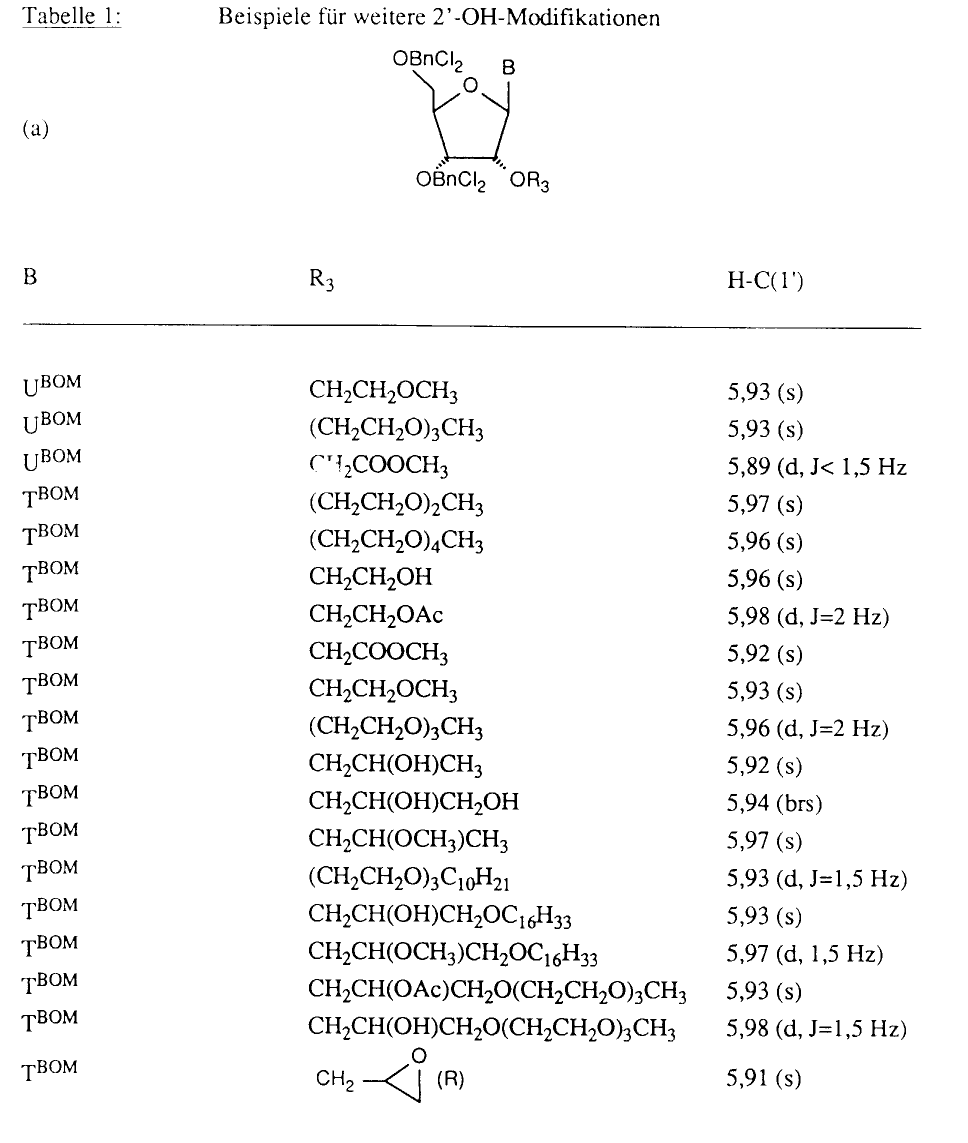

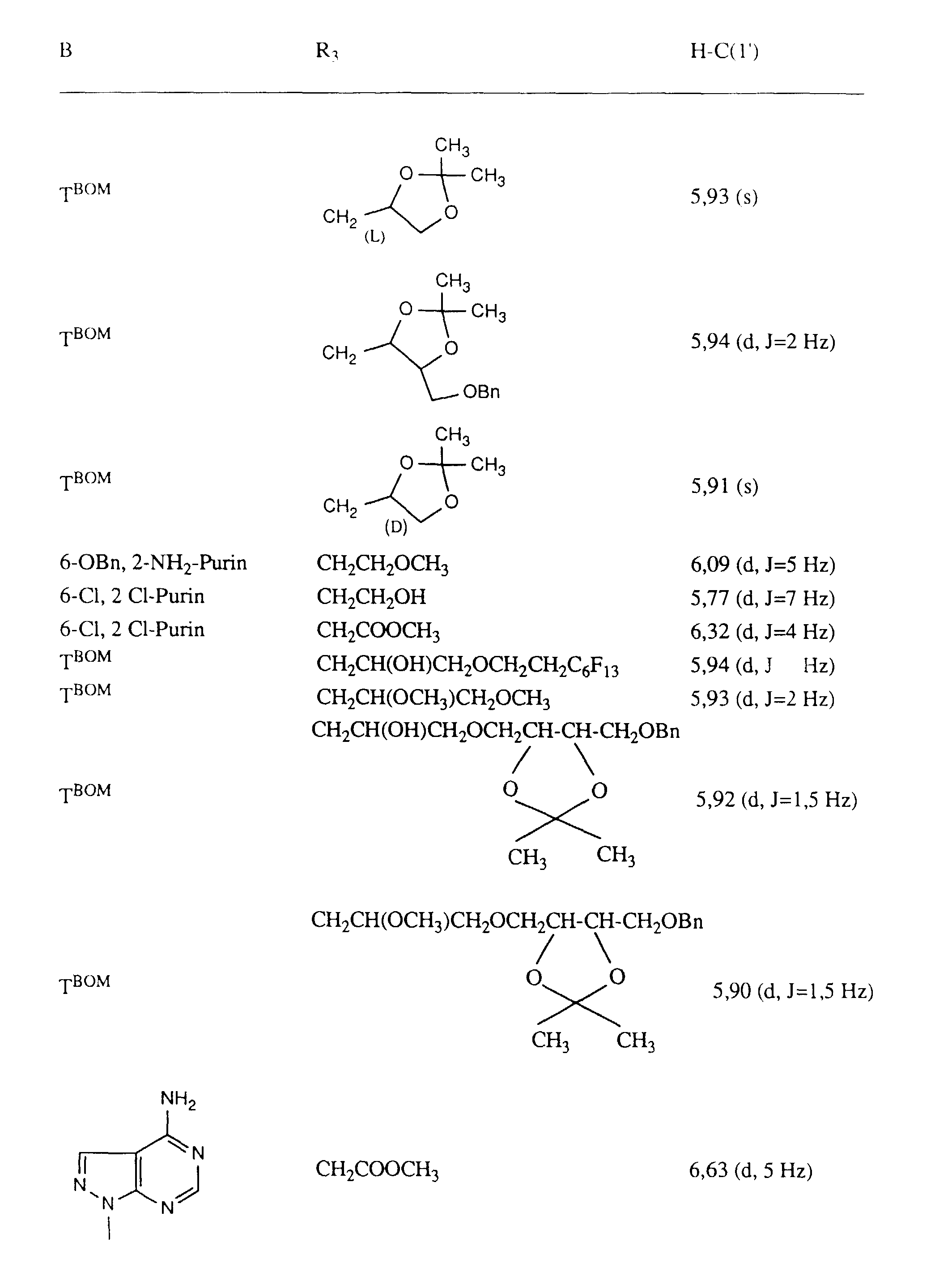

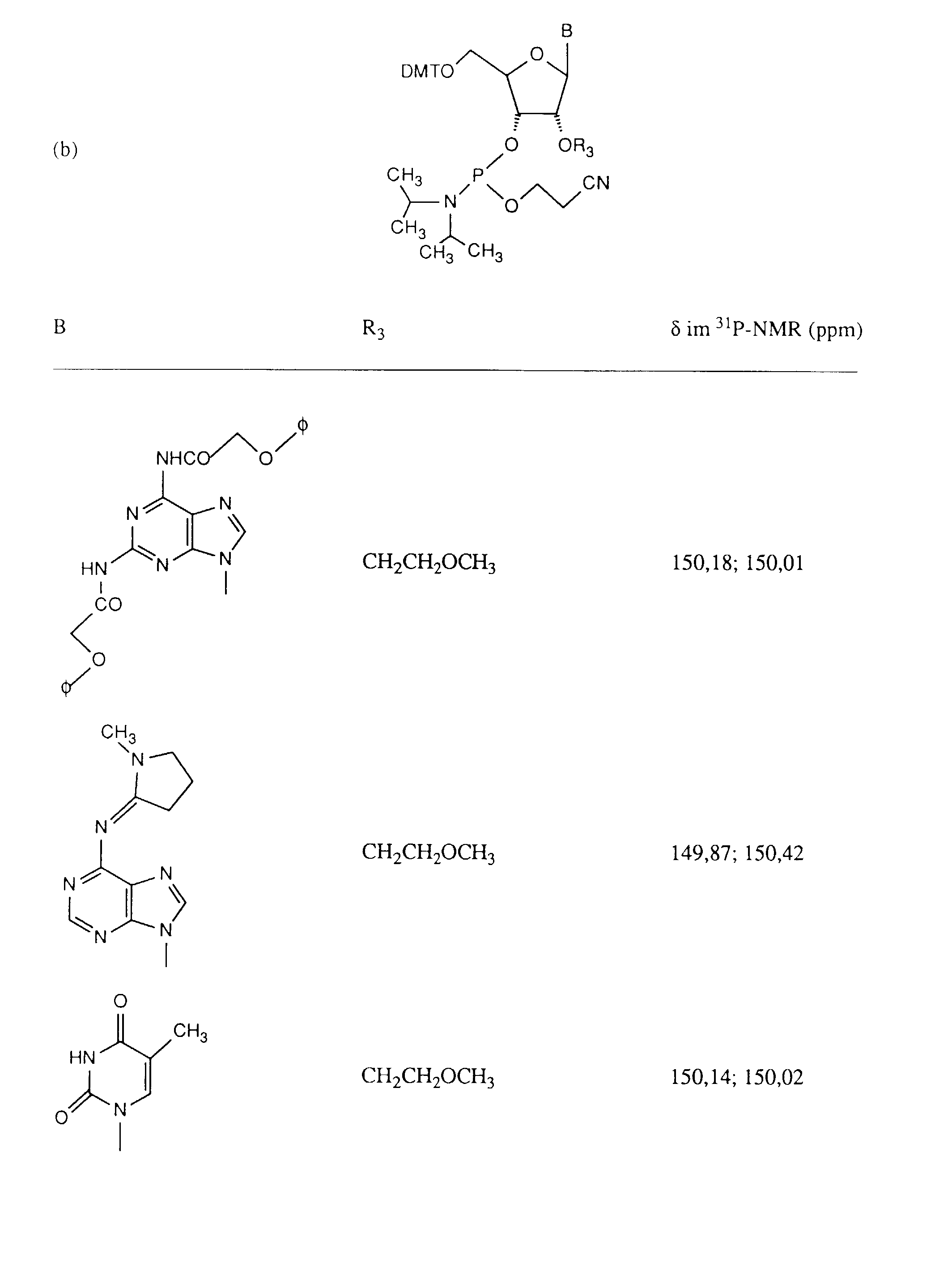

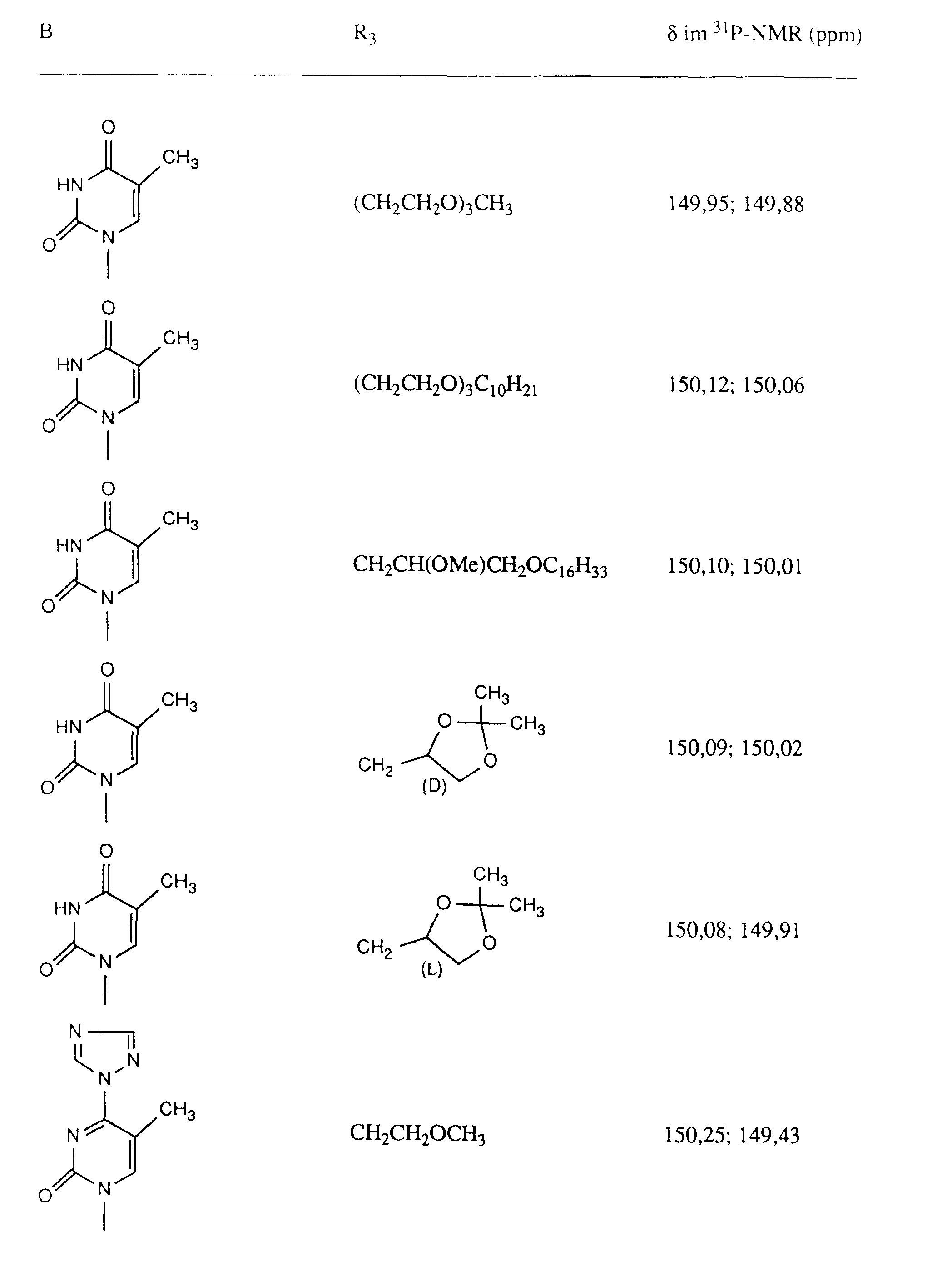

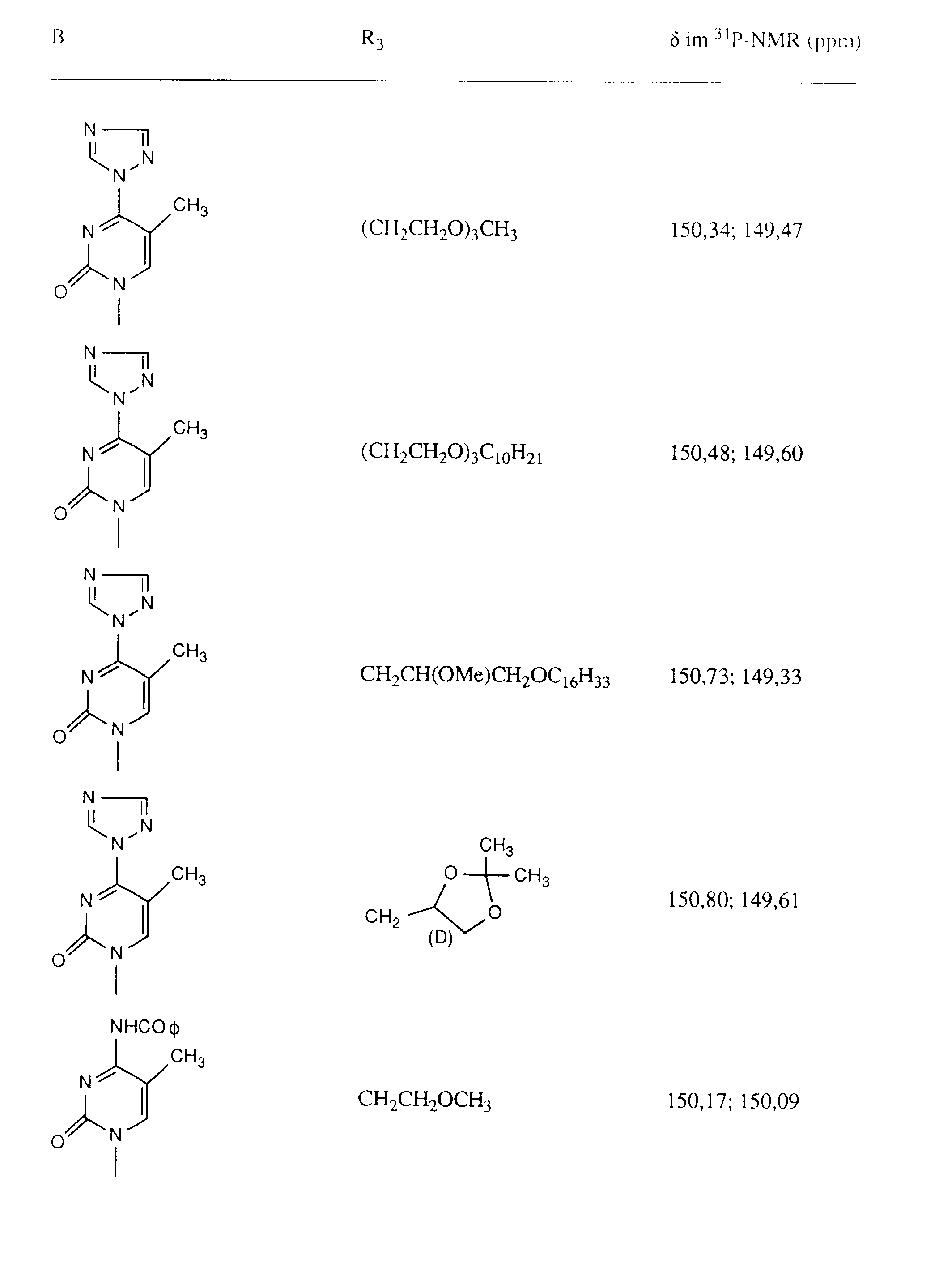

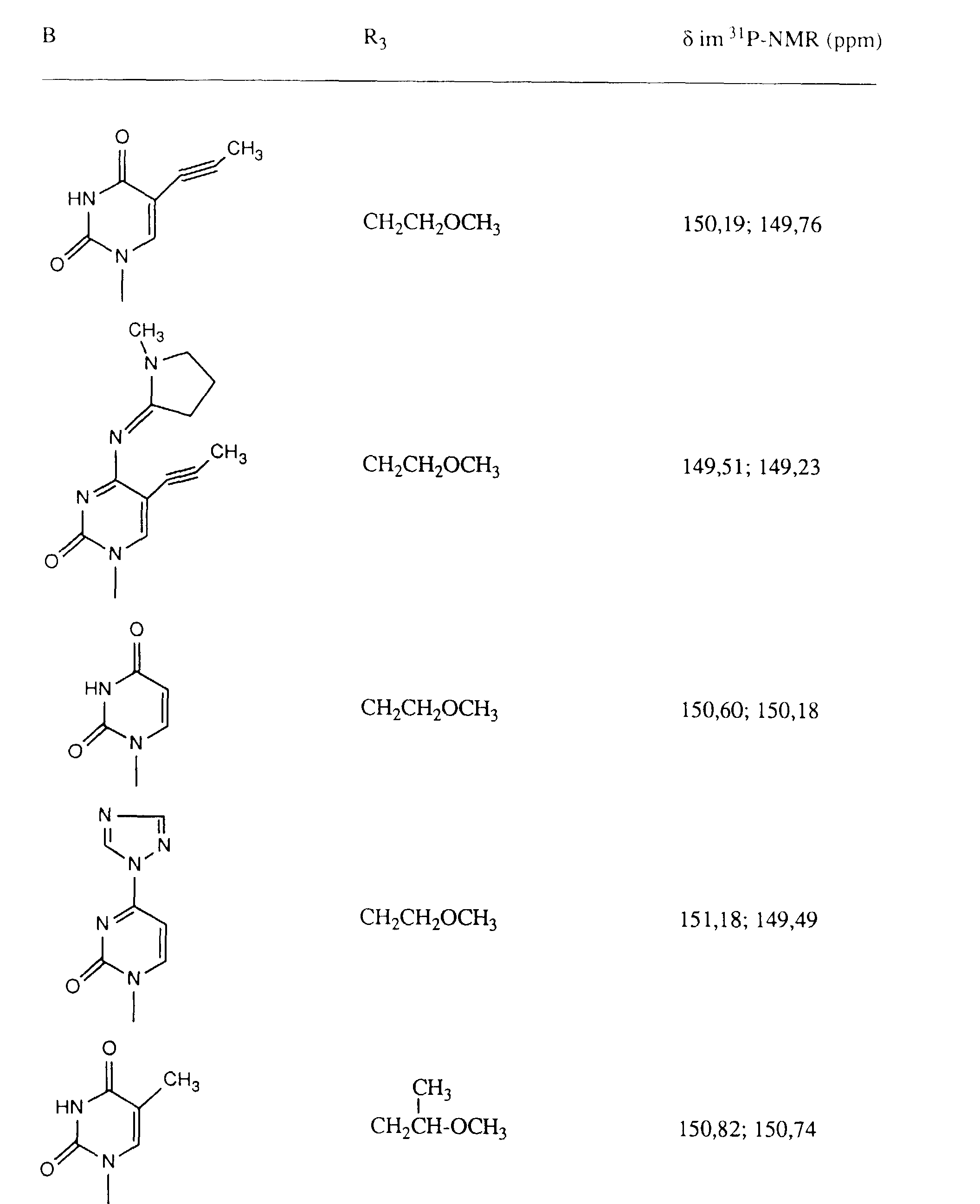

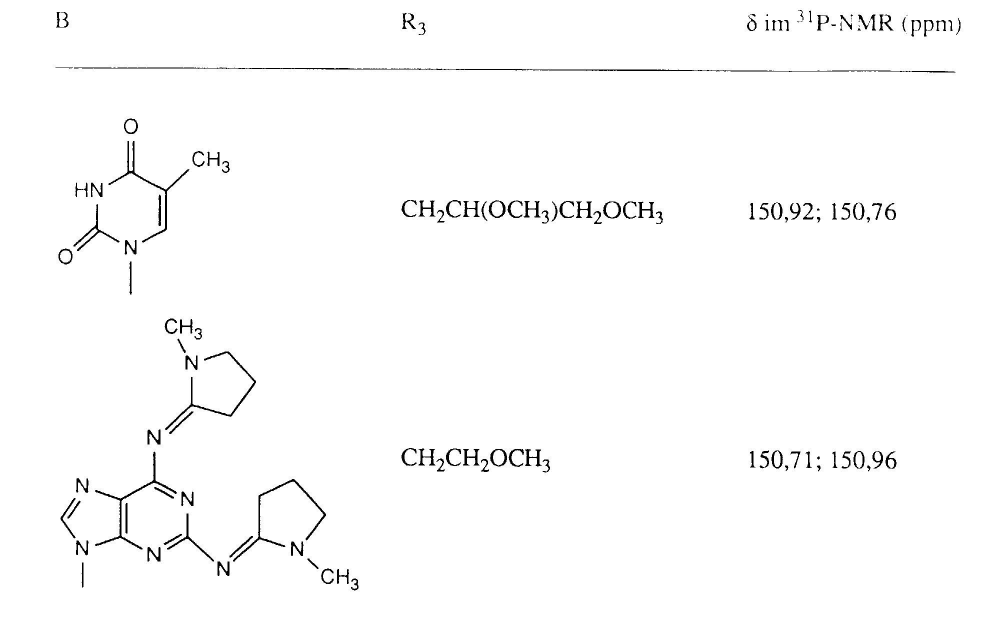

Beispiel A17: 5,0 g des in Beispiel A6 hergestellten Produktes werden in 50 ml THF gelöst. Nach Zugabe von 0,21 g NaH wird das Reaktionsgemisch während 45 Minuten auf 60°C erwärmt, dann mit 1,57 g Diethylenglykol-chlorethyl-methylether versetzt und schliesslich total 54 Stunden bei 60°C weitergerührt. Nach der 8. und 42. Stunde gibt man erneut 0,05 g NaH und 0,4 g des Diethylenglykol-chlorethyl-methylethers zu. Nach dem Abkühlen des Gemisches wird auf Wasser gegossen und mit Essigsäureethylester extrahiert. Nach Trocknen über MgSO₄ und Eindampfen des Extraktes erhält man die Verbindung (A17), welche noch an Kieselgel mit Hexan/Essigsäureethylester (1:1) chromatographiert wird. 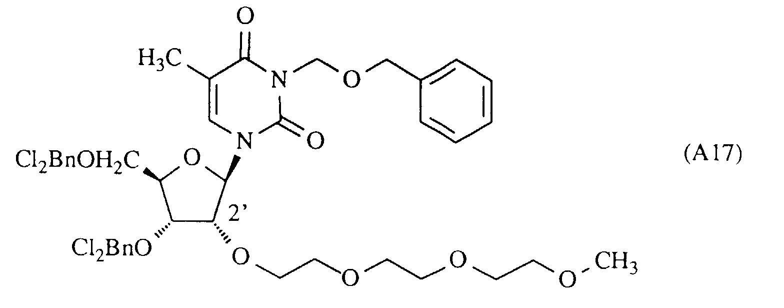 ¹H-NMR (250 MHz, CDCl₃): 1,60(s, CH₃); 3,33 (s, OCH₃); 5,47 (AB, CH₂); 5,93 [d, H-C(1')]; 7,65 [s, H-C(6)].
MS: 840 (M⁻).

Beispiel A18: 3,2 g des in Beispiel A17 hergestellten Produktes wird in 35 ml Tetrahydrofuran über 0,6 g Pd/C (5%) bei Normaldruck und 22°C hydriert. Nach 1 Stunde (H₂-Aufnahme: 96%) wird das Hydriergemisch klarfiltriert und eingedampft. Der Rückstand wird in 30 ml Methanol gelöst und mit 3,5 ml einer 30%igen Lösung von NaOCH₃ in Methanol versetzt. Nach 24 Stunden Rühren bei 20°C wird die Reaktionslösung auf Wasser gegossen und mit Essigsäureethylester extrahiert. Nach Trocknen über MgSO₄ und Eindampfen des Extraktes erhält man die Verbindung (A18). 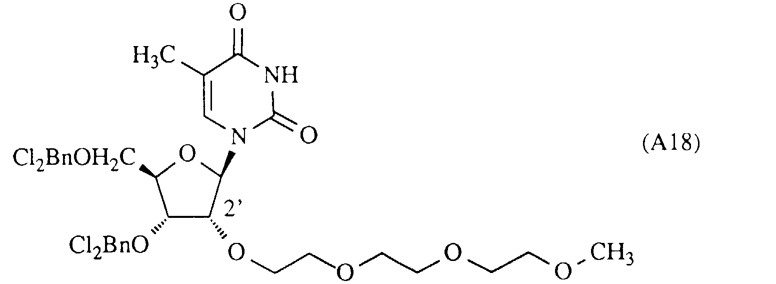 ¹H-NMR (250 MHz, CDCl₃): 1,60 (s, CH₃); 3,55 (s, OCH₃); 5,96 [d, J=2 Hz, H-C(1')]; 7,62 [s, H-C(6)]; 8,96 (s, NH).
MS: 720/722/724 (M⁺).

Beispiel A19: 2,3 g des in Beispiel A18 hergestellten Produktes werden in 75 ml Methanol gelöst und unter Zugabe von 1,15 g Natriumacetat und 1,0 g Pd/C (5%) bei 35°C hydriert. Nach 43 Stunden wird der Katalysator abfiltriert und das Filtrat eingeengt. Der Rückstand wird zur Entfernung von Salzen mit Essigsäureethylester/Methanol (4:1) über Kieselgel filtriert. Man erhält die Verbindung (A19). 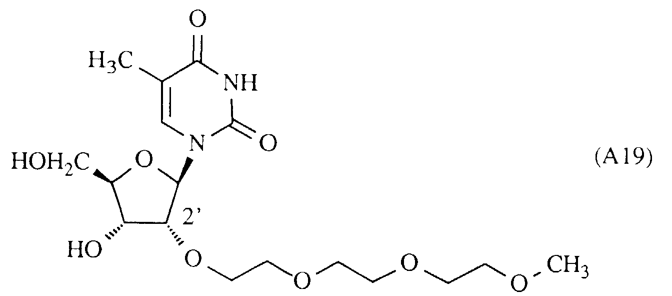 ¹H-NMR [250 MHz, Methanol (D₄)]: 1,72 (s, CH₃); 3,22 (s, OCH₃); 5,80 [d, J=5Hz,H-C(1')]; 7,78 [s, H-C(6)].
MS: 404 (M).

Beispiel A20: 400 mg des in Beispiel A19 hergestellten Produktes werden in 8 ml absolutem Pyridin gelöst. Bei 20°C gibt man 0.34 g DMTCl zu und rührt 20 Stunden lang. Dann wird mit 30 ml Methylenchlorid verdünnt und auf Wasser gegossen. Die organische Phase wird ein zweitesmal mit Wasser gewaschen und anschliessend getrocknet (MgSO₄) und eingedampft. Der Rückstand wird an Kieselgel mit Essigsäureethylester/Triethylamin (97:3) chromatographiert. Man erhält die Verbindung (A20). 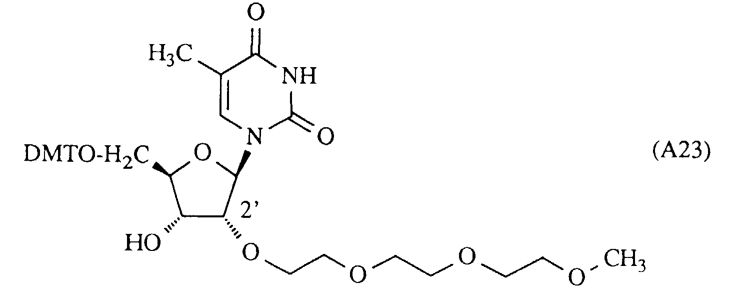 ¹H-NMR (250 MHz, CDCl₃; alle Signale sind breit): 1,40 (s, CH₃); 3,35 (s, OCH₃); 3,70 (s, 2 x OCH₃); 6,02 (s, H-C(1'); 7,66 [s, H-C(6)].
MS: 705 (M-H).

Beispiel A21: Zur Vorlage von 0,12 g Diisopropylammoniumtetrazolid und 0,20 g Cyanoethyltetraisopropylphosphoramidit in 2,5 ml absolutem Methylenchlorid werden 0,39 g des in Beispiel A20 hergestellten Produktes in 2,5 ml Methylenchlorid zugetropft. Nach 28 Stunden Rühren bei 20°C wird die Reaktionslösung auf eine gesättigte wässrige NaHCO₃-Lösung gegossen und mit Methylenchlorid extrahiert. Der eingedampfte Rückstand wird an Kieselgel mit Essigsäureethylester/Triethylamin (97:3) chromatographiert. Man erhält die Verbindung (A21). 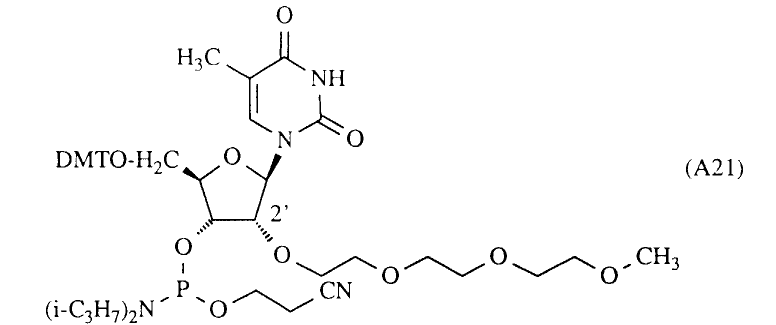 ¹H-NMR (250 MHz, CDCl₃): Die Protonen von H-C(1') erscheinen als Doublett bei 6,02 und 6,07 ppm.
³¹P-NMR (CDCl₃): 149,95 und 149,88 ppm

Beispiel A22: 21,4 g Uracil werden in 250 ml Dichlorethan suspendiert. Nach Zugabe von 116 g BSA wird auf 80° erwärmt. Nach 30 Minuten tritt Lösung ein. Man kühlt auf 40°C ab und gibt 50,0 g des in Beispiel A3 hergestellten Produktes, gelöst in 350 ml Dichlorethan, sowie 27,4 g SnCl₄ zu. Anschliessend wird die Lösung 5 Stunden auf 80°C gehalten. Nach dem Abkühlen wird auf gesättigte NaHCO₃-Lösung gegossen, die entstehende, 2-phasige Suspension filtriert und die organische Phase abgetrennt. Die wässrige Phase wird nochmals mit CH₂Cl₂ exträhiert. Die gesammelten Extrakte werden über MgSO₄ getrocknet und eingedampft. Der Rückstand wird in Acetonitril digeriert. Man erhält die Verbindung (A22). 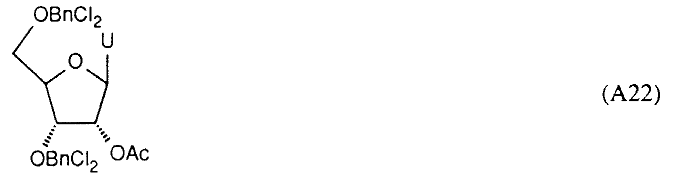 NMR (250 MHz, CDCl₃): 2.22 (s, OAc), 5.35 (t, H-C(2')), 6,07 (d, J=4 Hz, H-C(1')), 5.53 und 7.23 (je d, J=8 Hz, H-C(5) und H-C(6)), 8.57 (s, NH).
MS: 602

Beispiel A23: Zur Vorlage von 60 ml THF und 1,47 g NaH (100 %) werden bei 60°C 35,6 g des in Beispiel A4 hergestellten Produktes, in 300 ml THF gelöst, gegeben und 1 Stunde weiter erwärmt. Dann werden 14,0 g R-(-)Glycidyltosylat portionenweise eingetragen. Nach 2,5 Stunden werden erneut 0,1 g NaH zugegeben. Nach 4 Stunden Erwärmen wird abgekühlt, auf Eis gegossen und mit Essigsäureethylester extrahiert. Der eingedampfte Extrakt wird chromatographiert (Kieselgel, Toluol/Essigsäureethylester 9:1). Man erhält Verbindung (A23). 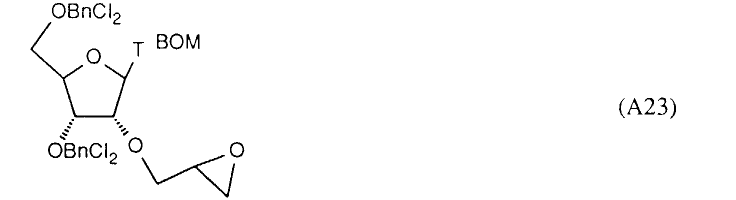 NMR (250 MHz, CDCl₃): 1.59 (s, CH₃), 5.48 (AB, CH₂), 5.92 (s, 1,5 Hz, H-C(1')), 7.62 (s, H-C(6)).
FAB-MS: 751 (M+H)

Beispiel A24: 5,1 g des in Beispiel A23 hergestellten Produktes und 4,93 g 1-Hexadecanol werden in 50 ml CH₂Cl₂ gelöst. Dazu werden 0,96 g Bortrifluoriddiethyletherat (d = 1,13 g/ml) zugegeben. Diese Lösung wird 19 Stunden bei Raumtemperatur gerührt, dann auf H₂O gegossen und mit CH₂Cl₂ extrahiert. Der eingedampfte Extrakt wird über Kieselgel chromatographiert (Essigsäureethylester/Hexan 1:1). Man erhält die Verbindung (A24). 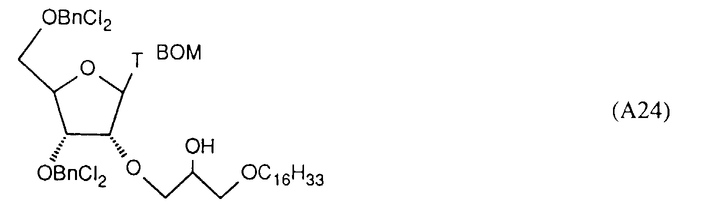 NMR (250 MHz, CDCl₃): 1.60 (s, CH₃), 5.47 (AB, CH₂), 5.93 (d, J=1,5 Hz, H-C(1')), 7,63 (s, H-C(6)).
FAB-MS: 1027 (M+Cl)⁻

Beispiel A25: 3,45 g des in Beispiel A24 hergestellten Produktes werden in 35 ml THF mit 0,15 g NaH (100 %) 45 Minuten auf 60°C erwärmt. Dann werden 0,63 g MeI zugegeben und 1,5 Stunden weiter erwärmt. Nach dem Abkühlen wird das Reaktionsgemisch auf Wasser gegossen und mit Essigsäureethylester extrahiert. Der eingedampfte Rückstand wird in Toluol über eine kurze Kieselgel-Säule filtriert. Man erhält die Verbindung (A25). 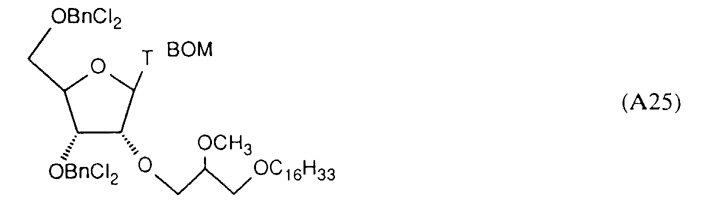 NMR (250 MHz, CDCl₃): 1.59 (s, CH₃), 5.45 (AB, CH₂), 5.93 (d, J=1,5 Hz, H-C(1')), 7.63 (s, H-C(6)).
MS (DCl): 1024 (M+NH₄)⁺

Beispiel A26: 3,3 g des in Beispiel A25 hergestellten Produktes werden in 70 ml THF über 0,7 g Pd/C hydriert (33 Stunden, H₂-Aufnahme 104 %). Das Reaktionsgemisch wird filtriert und eingedampft. Der Rückstand wird in 10 ml THF und 10 ml Methanol gelöst und mit NaOCH₃/HOCH₃-Lösung (30 %) versetzt. Nach 24 Stunden Rühren bei Raumtemperatur wird ca. bis zur Hälfte des Volumens eingeengt, dann auf Wasser gegossen und mit Essigsäureethylester extrahiert. Der eingedampfte Extrakt wird über Kieselgel chromatographiert (Essigsäureethylester/Toluol 1:2). Man erhält die Verbindung (A26). 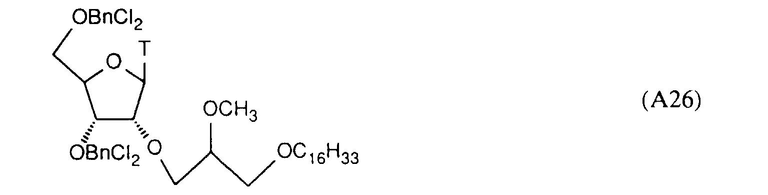 NMR (250 MHz, CDCl₃): 1.62 (s, CH₃), 3.40 (s, OCH₃), 5.98 (d, J=2 Hz, H-C(1')), 7.62 (s, H-C(6)), 8.57 (s, NH).
MS (DCl): 886

Beispiel A27: 1,21 g des in Beispiel A26 hergestellten Produktes werden mit 50 ml Methanol und 5 ml Toluol über 0,2 g Pd/C (10%) und 0,258 g MgO hydriert (2 Stunden, H₂-Aufnahme 220 ml). Das Reaktionsgemisch wird filtriert, eingedampft und über Kieselgel filtriert (Entfernung von Mg-Salzen). Man erhält die Verbindung (A27). 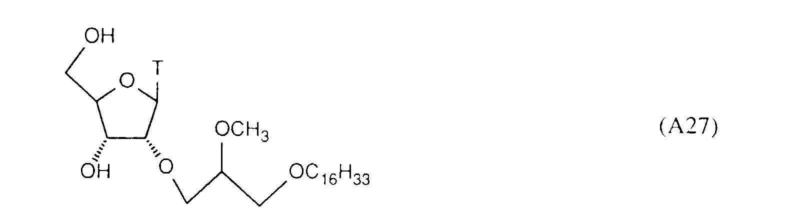 NMR (250 MHz, DMSO-D₆): 1.72 (s, CH₃), 5.05 (d, OH), 5.18 (t, OH), 5.84 (d, J=6 Hz, H-C(1')), 7.77 (s, H-C(6)).
FAB-MS: 569 (M-H).

Beispiel A28: 0,83 g des in Beispiel A28 hergestellten Produktes werden in 50 ml Pyridin gelöst und eingedampft. Der Rückstand wird mit 8 ml Pyridin und 0,6 g DMT-Chlorid versetzt und 44 Stunden bei Raumtemperatur gerührt. Die Lösung wird auf H₂O gegossen und mit Essigsäureethylester extrahiert. Der eingedampfte Rückstand wird über Kieselgel chromatographiert (Toluol/Essigsäureethylester 1:1 + 1 % NEt₃-Zusatz). Man erhält die Verbindung (A28). 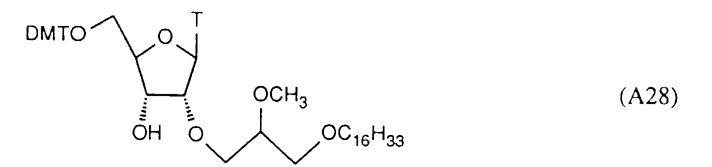 NMR (250 MHz, CDCl₃): 1.35 (s, CH₃), 3.47 (s, OCH₃), 3.80 (s, OCH₃), 6.03(d, J=4 Hz, H-C(1')), 7.64 (s, H-C(6)), 8.20 (s, NH).
FAB-MS: 873 (M+H).

Beispiel A29: 0,95 g des in Beispiel A28 hergestellten Produktes werden mit 10 ml CH₂Cl₂, 0,245 g Diisopropylammoniumtetrazolid und 0,39 g Cyanethyltetraisopropylphosphordiamidit versetzt. Nach 3 Tagen Rühren bei Raumtemperatur wird das Reaktionsgemisch auf gesättigte NaHCO₃-Lösung gegossen und mit CH₂Cl₂ extrahiert. Der eingedampfte Rückstand wird über Kieselgel chromatographiert (Toluol/Essigsäureethylester 2:1 + 1 % NEt₃-Zusatz). Man erhält die Verbindung (A29). 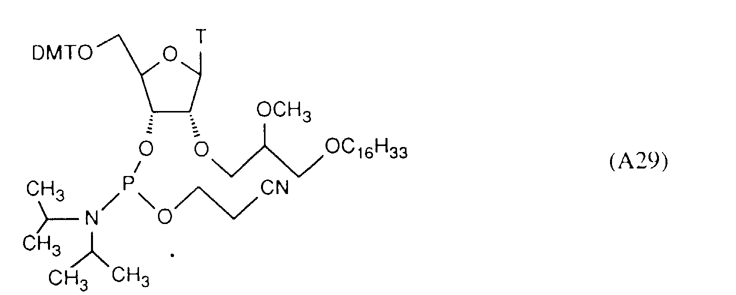 ¹H-NMR (250 MHz, CDCl₃): 5.97 und 6.05 (je d, J=4 Hz, je H-C(1)), 7.62 und 7.70 (je s, je H-C(6)).
P-NMR (250 MHz, CDCl₃): 150,012 und 150,102.
FAB-MS: 1071 (M-H).

Beispiel A30: 15,6 g Hypoxanthin und 46,6 g BSA werden in 300 ml Dichlorethan unter Rückfluss erhitzt. Nach 30 Minuten tritt Lösung ein. Es werden 30,0 g des in Beispiel A3 hergestellten Produktes sowie 16,5 g TMS-Triflat zugegeben und weitere 16 Stunden gekocht. Nach dem Abkühlen wird das Reaktionsgemisch auf gesättigte NaHCO₃-Lösung gegossen und die entstehende Suspension filtriert. Das Filtrat wird mit CH₂Cl₂ extrahiert. Die organische Phase wird über MgSO₄ getrocknet und eingedampft. Der Rückstand wird chromatographiert (Methanol/Essigsäureethylester 1:1). Man erhält die Verbindung (A30). 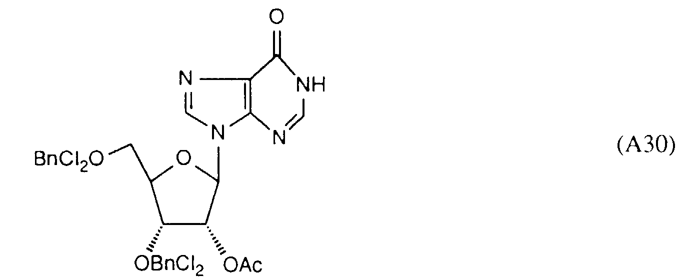 ¹H-NMR (250 MHz, CDCl₃): 6.22 (d, 3 Hz, H-C(1')), 8.14 und 8.37 (je s, H-C(2) und H-C(8)).
FAB-MS: 625 M-H

Beispiel A31: 3,8 g des in Beispiel A30 hergestellten Produktes werden in 150 ml CH₂Cl₂ vorgelegt. Unter Kochen wird dazu ein Gemisch aus 4,8 ml SOCl₂ und 2,4 ml DMF getropft. Nach 3 Stunden Weiterkochen werden erneut 1,2 ml SOCl₂ und 0,6 ml DMF zugegeben. Nach insgesamt 5,5 Stunden Kochen wird das Reaktionsgemisch auf eine gesättigte NaHCO₃-Lösung eingetragen. Nach 20 Minuten Rühren wird mit CH₂Cl₂ extrahiert. Der Extrakt wird über MgSO₄ getrocknet und eingedampft. Man erhält die Verbindung (A31). 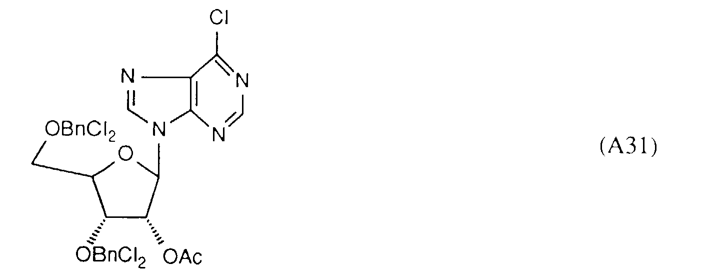 NMR (250 MHz, CDCl₃): 5.83 (dd, H-C(2')), 6.33 (d, J=3 Hz, H-C(1')), 8.47 und 8.73 (je s, H-C(2) und H-C(8)).
MS: 664

Beispiel A32: 30,4 g des in Beispiel A31 hergestellten Produktes werden in 200 ml Methanol und 100 ml THF gelöst. Dazu werden 16,9 g 30%-ige NaOMe/Methanol getropft. Nach 3 Stunden Rühren wird auf H₂O gegossen und mit Essigsäureethylester extrahiert. Der Extrakt wird über MgSO₄ getrocknet und eingedampft. Man erhält die Verbindung (A32). 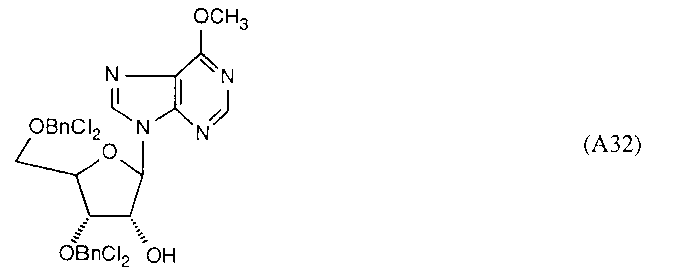 NMR (250 MHz, CDCl₃): 4.20 (s, OCH₃), 6.10 (d, J=6 Hz, H-C(1')), 8.17 und 8.48 (je s, H-C(2) und H-C(8)).
MS: 598

Beispiel A33: Zu 25,4 g des in Beispiel A32 hergestellten Produktes in 250 ml THF werden 1,17 g NaH (100 %) gegeben. Unter Kochen werden 6,76 g 2-Bromethylmethylether zugetropft. Nach 6 Stunden Weiterkochen werden erneut 0,5 g NaH und 1,9 ml Bromid zugegeben. Nach 23 Stunden wird das Reaktionsgemisch abgekühlt und auf H₂O gegossen. Nach Extraktionen mit Essigsäureethylester wird eingedampft und der Rückstand chromatographiert. (Kieselgel, Hexan/Aceton 2:1). Man erhält die Verbindung (A33). 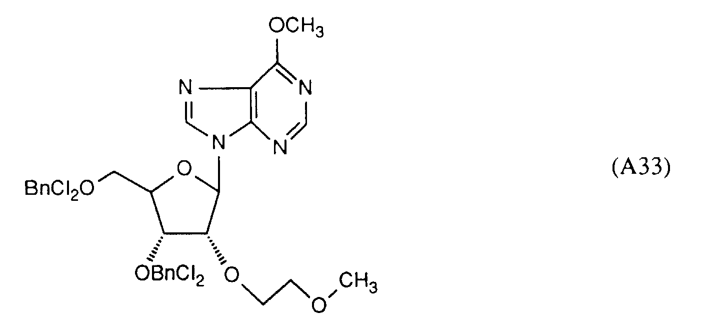 ¹H-NMR (250 MHz, CDCl₃): 3.27 (OCH₃), 4.20 (OCH₃), 6.27 (d, J=5 Hz, H-C(1')), 8.24 und 8.52 (je s, H-C(2) und H-C(8)).
MS(DCI): 656

Beispiel A34: 2,2 g des in Beispiel A33 hergestellten Produktes wird in 45 ml Methanol mit 0,66 g Pd/C (10 %) und mit Zusatz von 402 ml MgO hydriert. Nach 13 Stunden (H₂-Aufnahme: 485,5 ml H₂) wird das Gemisch klarfiltriert und eingedampft. Zur Entfernung von Magnesiumsalzen wird über Kieselgel chromatographiert (Essigsäureethylester/Methanol 9:1). Man erhält die Verbindung (A34). 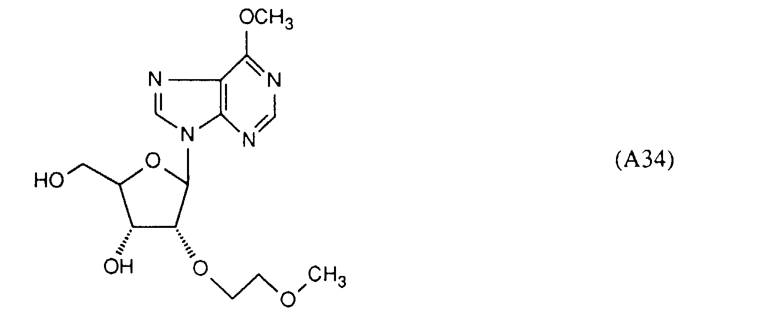 NMR (250 MHz, DMSO(-d₆)): 4.4 (t, OH), 5.23 (OH), 5.94 (d, J=6,5 Hz, H-C(1')), 8.40 und 8.55 (je s, H-C(2) und H-C(8)).
FAB MS: 339 (M-H)⁻.

Beispiel A35: 2,46 g des in Beispiel A34 hergestellten Produktes werden in 80 ml Methanol gelöst und in einen Autoklaven verbracht. Es werden 20 g NH₃ aufgepresst und die Apparatur 12 Stunden auf 120°C gehalten (Innendruck: 17 bar). Das Reaktionsgemisch wird eingedampft und an Kieselgel chromatographiert (Essigsäureethylester/Methanol). Man erhält die Verbindung (A35). 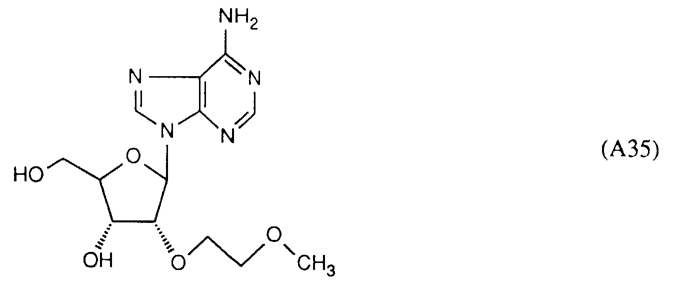 NMR (250 MHz, DMSO-D₆): 5.32 (d, OH), 5.60 (t, OH), 6.15 (d, J=6 Hz, H-C(1')), 8.33 und 8.57 (je s, H-C(2) und H-C(8)).
MS (DCI): 326 (M+H).

Beispiel A36: 0,48 g des in Beispiel A35 erhaltenen Produktes werden in 5 ml Methanol gelöst und mit 0,28 g N-Methyl-2,2-dimethoxypyrrolidin versetzt. Nach 18-stündigem Rühren wird eingedampft. Der Rückstand wird zweimal mit Pyridin versetzt und eingedampft. Der verbliebene Rückstand wird in 10 ml Pyridin gelöst und mit 0,5 g DMT-chlorid 40 Stunden bei Raumtemperatur gerührt. Dann wird auf H₂O gegossen und mit CH₂Cl₂ extrahiert. Der eingedampfte Extrakt wird über Kieselgel chromatographiert (Essigsäureethylester/Methanol 9:1). Man erhält die Verbindung (A36). 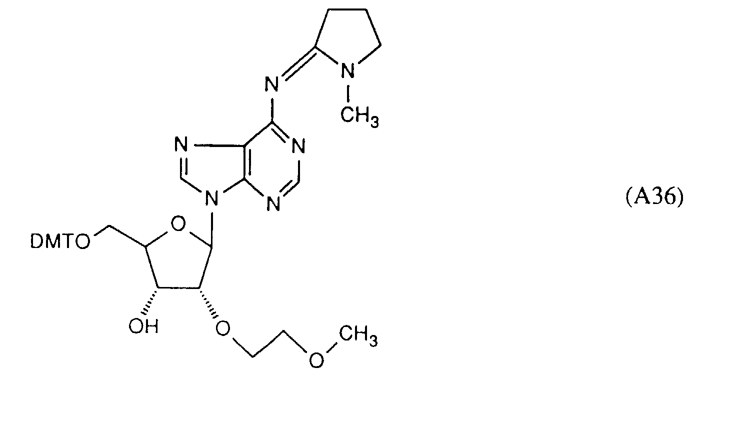 NMR (250 MHz, CDCl₃): 3.09 (s, NMe), 3.72 (s, OCH₃), 6.08 (d, J=6 Hz, H-C(1')), 7.98 und 8.43 (je s, H-C(2) und H-C(8)).
FAB MS: 709 (M+H)

Beispiel A37: Zu 0,38 g Cyanethyltetraisopropylphosphordiamidit und 0,24 g Diisopropylammoniumtetrazolid in 6 ml CH₂Cl₂ werden 0,63 g des in Beispiel A36 erhaltenen Tritylderivates in 6 ml CH₂Cl₂ gegeben. Nach 40-stündigem Rühren wird das Reaktionsgemisch auf gesättigte NaHCl₃-Lösung gegossen und mit CH₂Cl₂ extrahiert. Der eingedampfte Extrakt wird chromatographiert (Essigsäureethylester + 1 % NEt₃). Man erhält die Verbindung (A37). 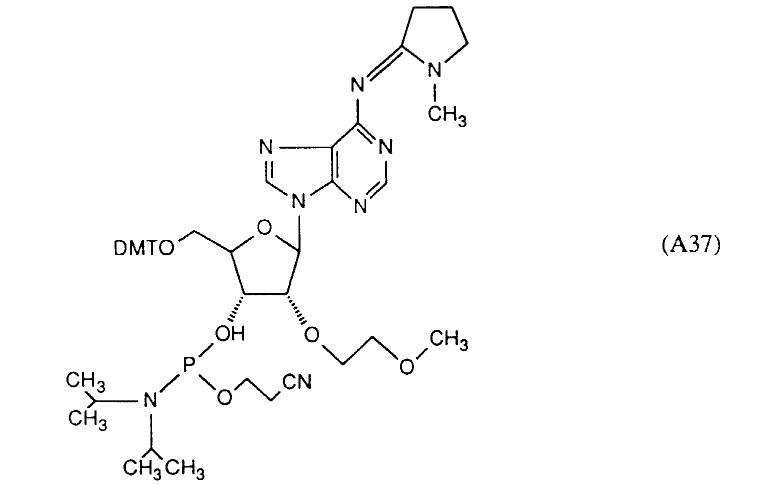 ³¹P-NMR (250 MHz, CDCl₃): 149.8 und 150.4.
FAB-MS: 909 (M+H)

Beispiel A38: 7,1 g des in Beispiel A23 hergestellten Epoxids wird in 70 ml THF gelöst, mit 0,43 g NaBH₄ versetzt und bei Raumtemperatur gerührt. Dazu werden 0,5 g BF₃·OEt₂ zugegeben. Nach insgesamt 26 Stunden wird auf Wasser gegossen und mit Essigsäureethylester extrahiert. Nach Eindampfen erhält man die Verbindung (A38). 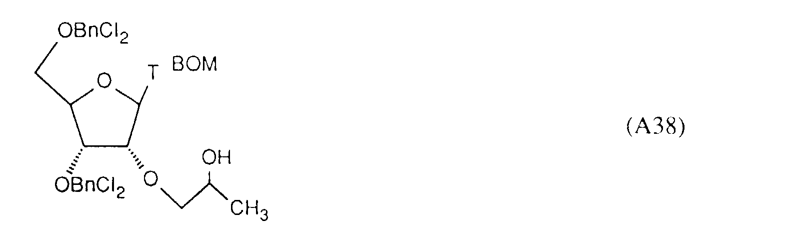 NMR (250 MHz, CDCl₃): 1.16 (d, J=7,5 Hz, CH₃), 1.61 (s, CH₃), 5.48 (AB, CH₂), 5.92 (d, J=1 Hz, H-C(1')), 7.69 (s, H-C(6)).
FAB-MS: 787 (M+Cl)⁻.

Beispiel A39: 6,9 g des in Beispiel A38 erhaltenen Alkohols werden mit 1,56 g NaH (100 %) und 1,56 g MeJ in 70 ml THF methyliert (60°C, 5 Stunden). Nach dem üblichen Aufarbeiten erhält man die Verbindung (A39). 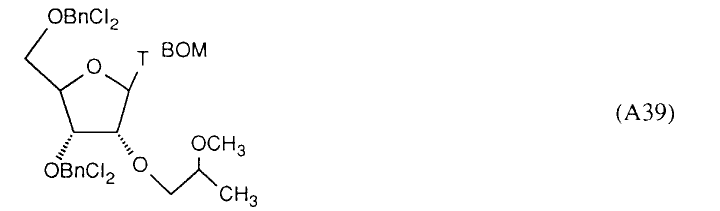 NMR (250 MHz, CDCl₃): 1.13 (d, J=7,5 Hz, CH₃), 1.60 (s, CH₃), 3.33 (s, OCH₃), 5.49 (AB, CH₂), 5.95 (A, J=2 Hz, H-C(1')), 7.64 (s, H-C(6)).
MS (DCI): 766 (M⁻).

Beispiel A40: 14,4 g des nach Beispiel A4 hergestellten Derivates werden in 150 ml THF und 0,65 g NaH (100 %) 30 Minuten auf 60°C erwärmt. Dann wird auf 25°C abgekühlt und es werden 5,92 g D-α,β-Isopropylidenglycerin-γ-tosylat zugegeben. Nach 1,5 Stunden wird noch 3 Stunden bei 60°C weitergerührt. Das abgekühlte Reaktionsgemisch wird auf Wasser gegossen, mit Essigsäureethylester extrahiert und eingedampft. Der Rückstand wird über Kieselgel (Toluol/Essigsäureethylester 4:1) chromatographiert. Man erhält die Verbindung (A40). 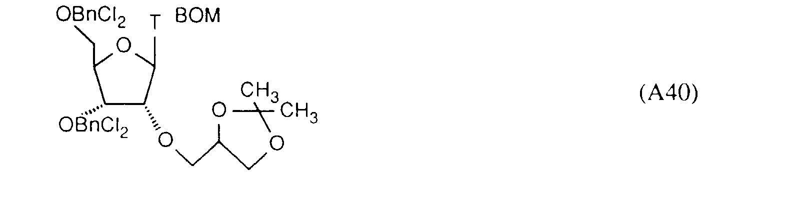 NMR (250 MHz, CDCl₃): 1.34 und 1.41 (je s, je CH₃), 1.58 (s, CH₃), 5.48 (AB, CH₂), 5.92 (d, J=1,5 Hz, H-C(1')), 7.68 (s, H-C(6)).
MS (DCI): 843 (M+Cl)⁻.

Beispiel A41: 3,13 g des nach Beispiel A13 hergestellten Phosphoramidites werden in 50 ml Acetonitril gelöst. Dazu werden 9,4 ml NEt₃ und 6,17 g 1,2,4-Triazol gegeben. Unter Rühren werden anschliessend 1,53 g POCl₃ derart zugetropft, dass 30°C nicht überschritten werden. Nach 4 Stunden Weiterrühren bei Raumtemperatur wird das Reaktionsgemisch auf 1 N NaHCO₃ (150 ml) gegossen und mit Essigsäureethylester extrahiert. Der eingedampfte Extrakt wird in wenig CH₂Cl₂ gelöst und in 100 ml n-Pentan eingerührt. Dabei fällt die Verbindung (A41) aus. 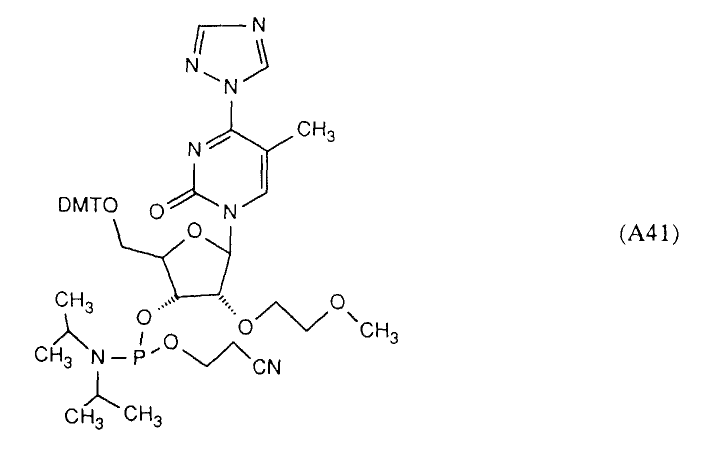 ¹H-NMR (250 MHz, CDCl₃): 6.02 und 6.06 (je d, J=1,5 Hz, H-C(1')), 8.44 und 8.47 (je s, H-C(6)), 8.07 und 9.35 (je s, triazole H).
³¹P-NMR (250 MHz, CDCl₃): 149,433 und 150,253
MS (DCI): 869 (M⁻)

Beispiel A42: 8,88 g N²-Isobutyryl-O⁶-benzylguanin [hergestellt nach analoger Vorschrift von Jenny, T.F., Schneider, K.C., Benner, S.A., Nucleosides and Nucleotides 11:1257-1261 (1992)] und 34,8 g N,O-Bis-(trimethylsilyl)acetamid werden in 130 ml Toluol suspendiert und auf 100°C erhitzt bis Lösung eintritt. Bei 50°C werden 13,0 g des nach Beispiel A3 hergestellten Ribosederivates sowie 6,33 g Trifluormethansulfonsäuretrimethylsilylester zugegeben. Die farblose Lösung wird 4 Stunden bei 100°C gerührt und nach dem Abkühlen auf 200 ml gesättigte NaHCO₃-Lösung gegossen. Nach dem Extrahieren mit Essigsäureethylester wird über MgSO₄ getrocknet und eingedampft. Der Rückstand wird über 1,2 kg Kieselgel chromatographiert (Toluol/Essigsäureethylester 4:1). Man erhält die Verbindung (A42). 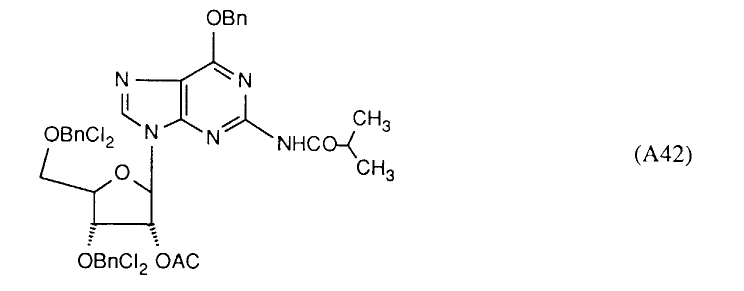 ¹H-NMR (250 MHz, CDCl₃): 1.26 (d, J=7,5 Hz, CH₃), 2.23 (s, OAc), 5.61 (AB, OCH₂), 5.78 (dd, H-C(2')), 6.19 (d, J=3 Hz, H-C(1')), 7.83 (s, NH), 8.00 (s, H-C(8)).
MS (DCI): 801 (M⁻).

Beispiel A43: 7,4 g des im Beispiel A42 hergestellten Derivates, in 80 ml Methanol gelöst, werden mit 0,83 g einer 30%igen NaOMe/Methanol-Lösung versetzt und 30 Minuten bei Raumtemperatur gerührt. Die Lösung wird auf H₂O gegossen und mit Essigsäureethylester extrahiert. Der Extrakt wird nach Trocknen über MgSO₄ eingedampft. Man erhält die Verbindung (A43). 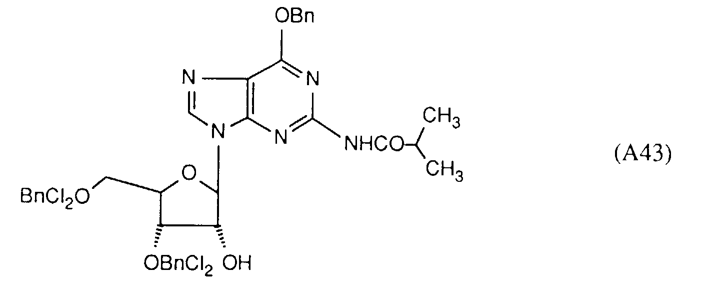 NMR (250 MHz, CDCl₃): 1.29 (d, J=7,5 Hz, CH₃), 5.61 (AB, OCH₂), 5.99 (d, J=6 Hz, H-C(1')), 7.97 (s, NH), 8.04 (s, H-C(8)).
MS (DCI): 760 (M+H)⁺

Beispiel A44: Zu 9,0 g der nach Beispiel A43 hergestellten Verbindung in 100 ml THF werden 630 mg NaH (100 %) gegeben und 15 Minuten gerührt. Dann werden 1,97 g 2-Bromethyl-methylether zugespritzt. Nach 25 Stunden werden erneut 2,0 g Bromid zugegeben. Nach total 48 Stunden Rühren wird auf Wasser gegossen und mit Essigsäureethylester extrahiert. Der eingedampfte Extrakt wird über Kieselgel chromatographiert (Toluol/Essigsäureethylester 4:1). Man erhält die Verbindung (A44). 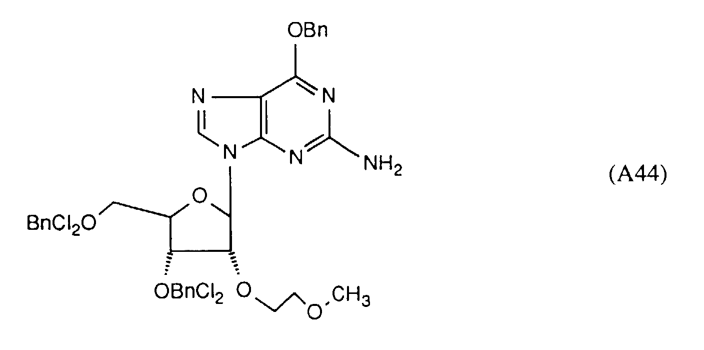 NMR (250 MHz, CDCl₃): 3.28 (s, OCH₃), 5.56 (AB, OCH₂), 6.10 (d, J=5 Hz, H-C(1')), 7.90 (s, H-C(8)).
FAB-MS: 748 (M+H)⁺.

Beispiel A45: Ausgehend von Verbindung (A3) werden weitere Basen eingeführt. Die Produkte sind in Tabelle 2 aufgeführt.

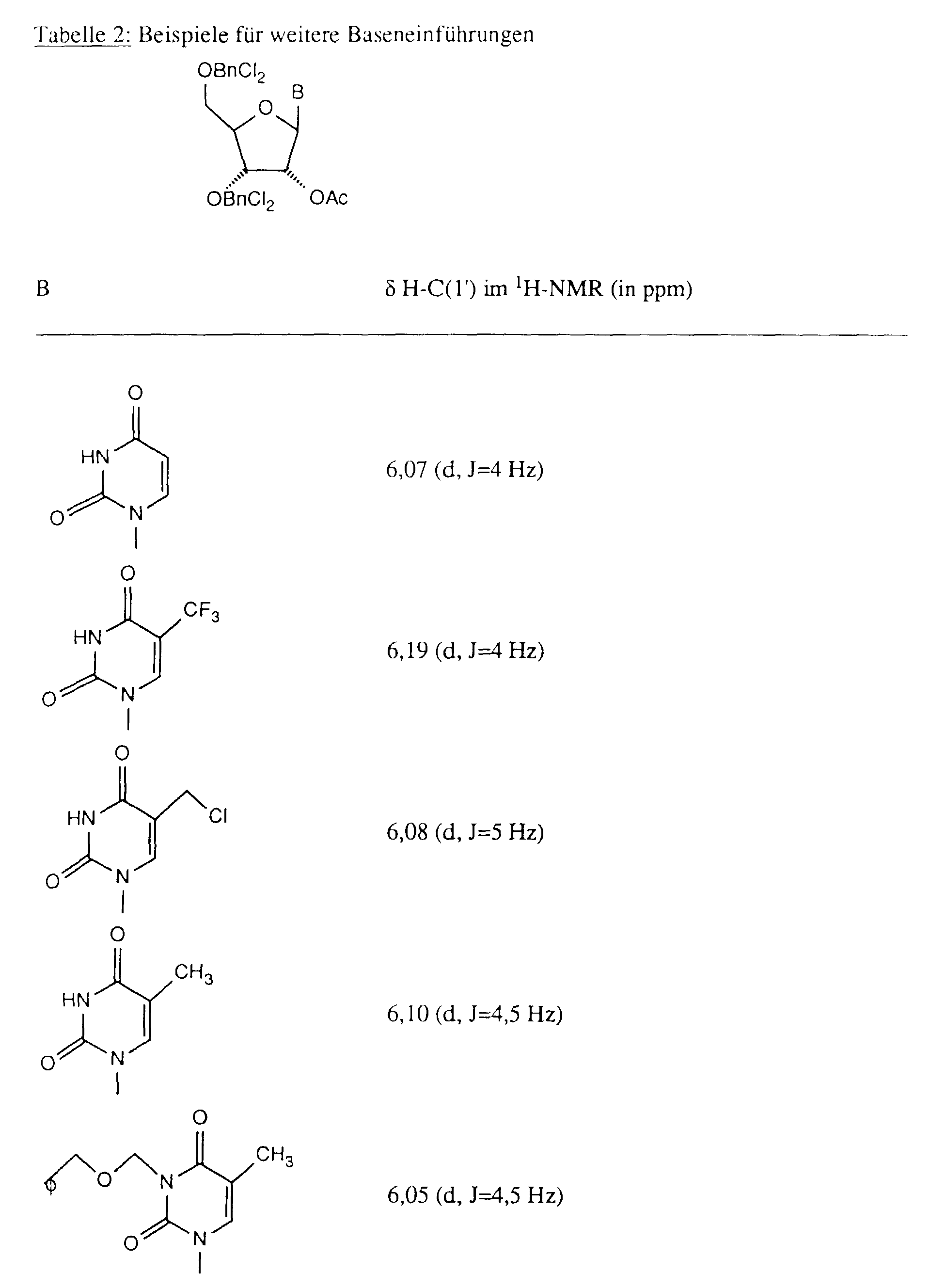

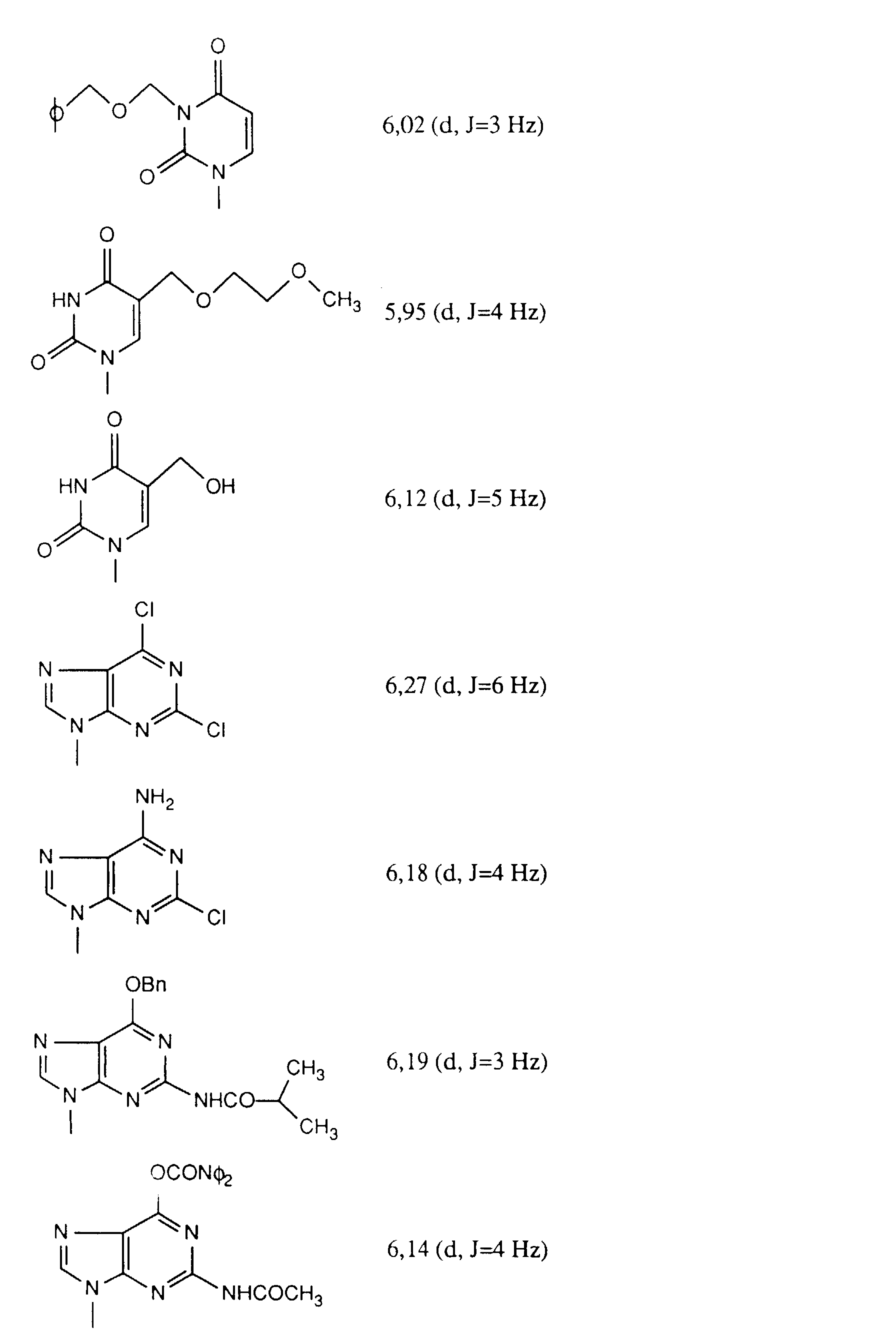

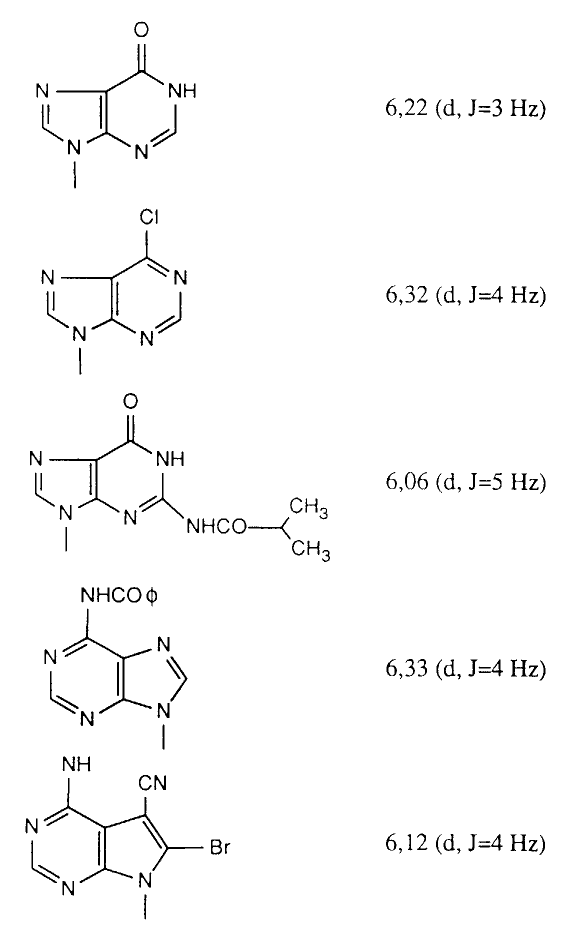

### Beispiel B: Herstellung von Oligonukleotiden

Oligonukleotide werden unter Verwendung der erfindungsgemässen dimethoxytritylierten und 3'-aktivierten [3'-(β-Cyanoethoxy-di(i-propylamino)phosphoramidit)] Nukleoside bzw. solchen natürlichen aktivierten Nukleosiden an einen festen Träger gebunden (Controlled Pore Glas, CPG) und die Synthese auf einem DNA-Synthesiser (Applied Biosystems, Modell 380 B, Standard Phosphoramiditchemie und Iodoxidation) gemäss den Standardprotokollen des Herstellers durchgeführt [vergleiche auch "Oligonucleotide synthesis a practical approach" M.J Gait; IRL Press 1984 (Oxford-Washington DC)]. Nach der Kopplung des letzten Nukleosidbausteins wird das 5'-geschützte Oligonukleotid unter gleichzeitiger Abspaltung aller übrigen Schutzgruppen durch Behandlung mit konzentriertem wässrigem Ammoniak über Nacht vom Träger abgelöst und anschliessend unter Verwendung von 50 mM Ammoniumacetatpuffer (pH 7)/Acetonitril durch "reverse-phase" HPLC gereinigt. Anschliessend wird die 5'-Dimethoxytrityl-Schutzgruppe durch 20-minütige Behandlung mit 80 %-iger wässriger Essigsäure abgespalten, das Oligonukleotid mit Ethanol ausgefällt und durch Zentrifugation isoliert. Die Reinheit des Oligonukleotids wird durch Gelelektrophorese (Polyacrylamid), seine Identität mittels matrixunterstützter Laserdesorptions-Time-of-flight Massenspektroskopie (MALDI-TOF MS) überprüft.

### Beispiel C1: Affinität; Wechselwirkung der Oligonukleotide (Antisense) mit komplementären Oligoribonukleotidsequenzen (Sense)

Die Wechselwirkung der Oligonukleotide mit den entsprechenden basenkomplementären Oligomeren der natürlichen Ribonukleotide wird durch das Aufzeichnen von UV-Schmelzkurven und den daraus ermittelten Tₘ-Werten charakterisiert. Diese Standardmethode ist zum Beispiel von Marky, L.A., Breslauer, K.J., Biopolymers 26:1601-1620 (1987) beschrieben.
Es wird eine Lösung der Oligonukleotide und der entsprechenden basenkomplementären natürlichen Oligoribonukleotide in 10 mM Phosphatpuffer, 100 mM NaCl, 0,1 mM EDTA, pH = 7,0 (c = 4·10⁻⁶ M/Oligonukleotid) hergestellt und die Änderung der Extinktion bei 260 nm in Abhängigkeit von der Temperatur (15 bis 95°C) aufgezeichnet. Aus den erhaltenen Schmelzkurven wird der Tₘ-Wert ermittelt (Tabelle 3).

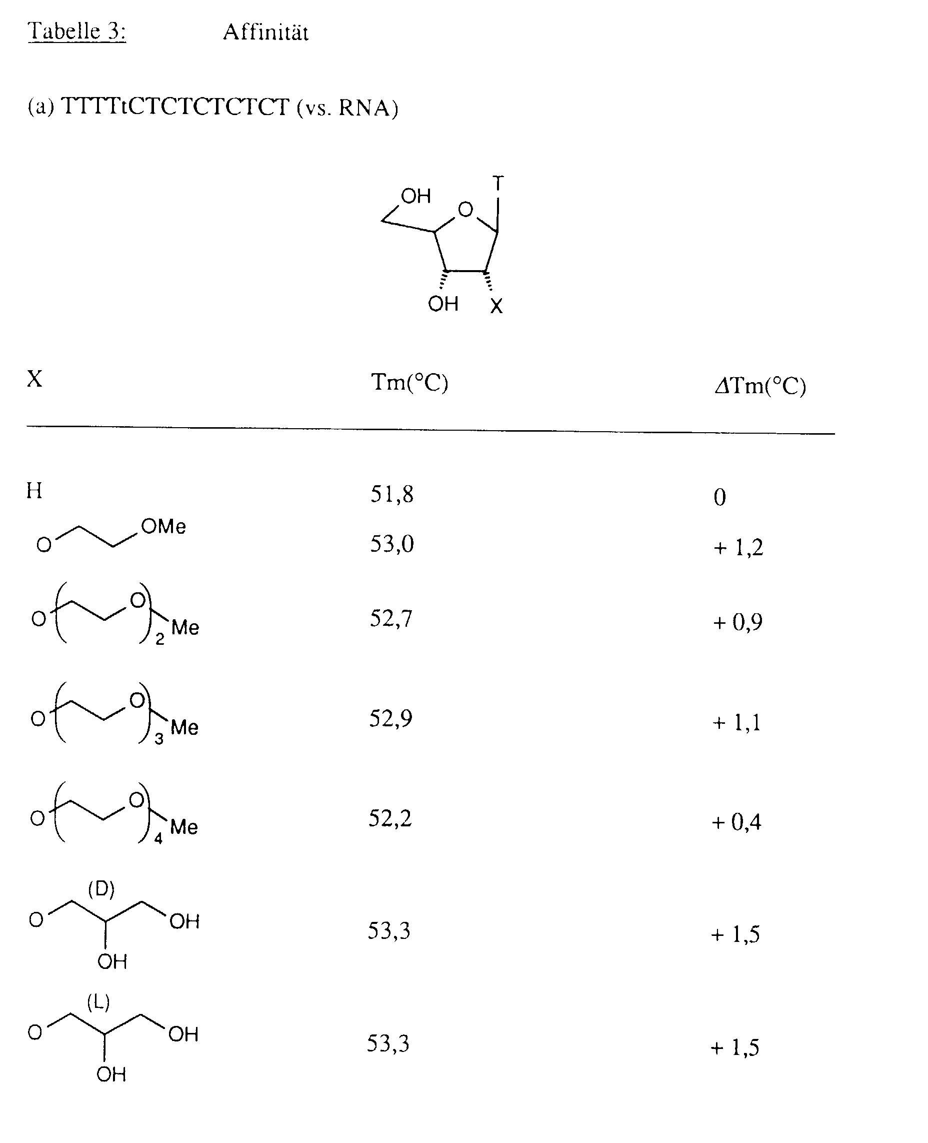

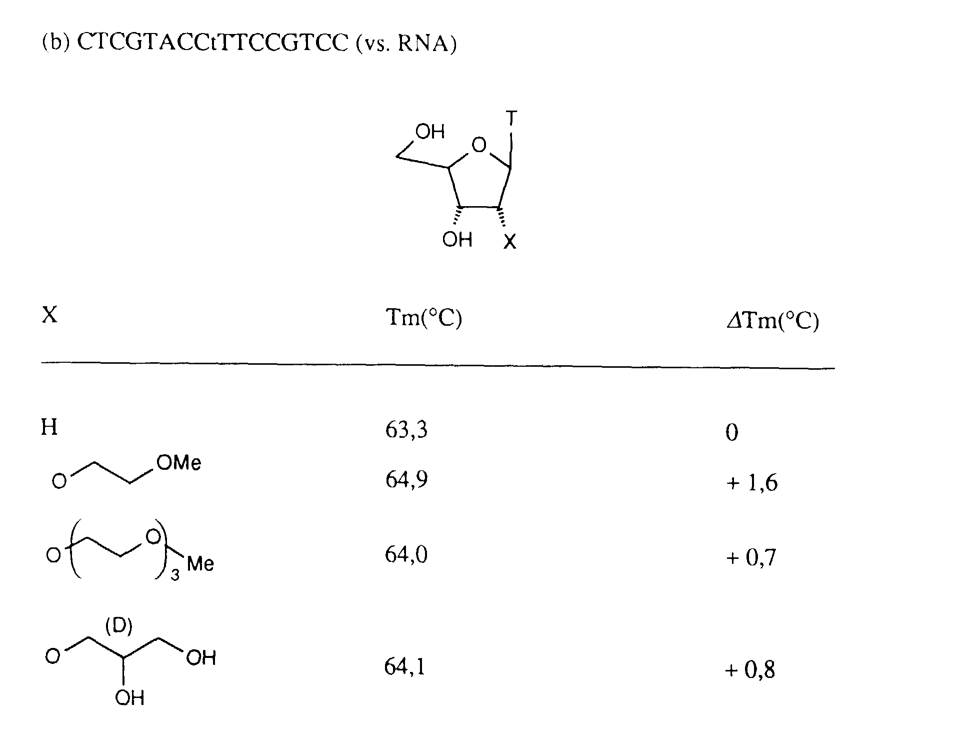

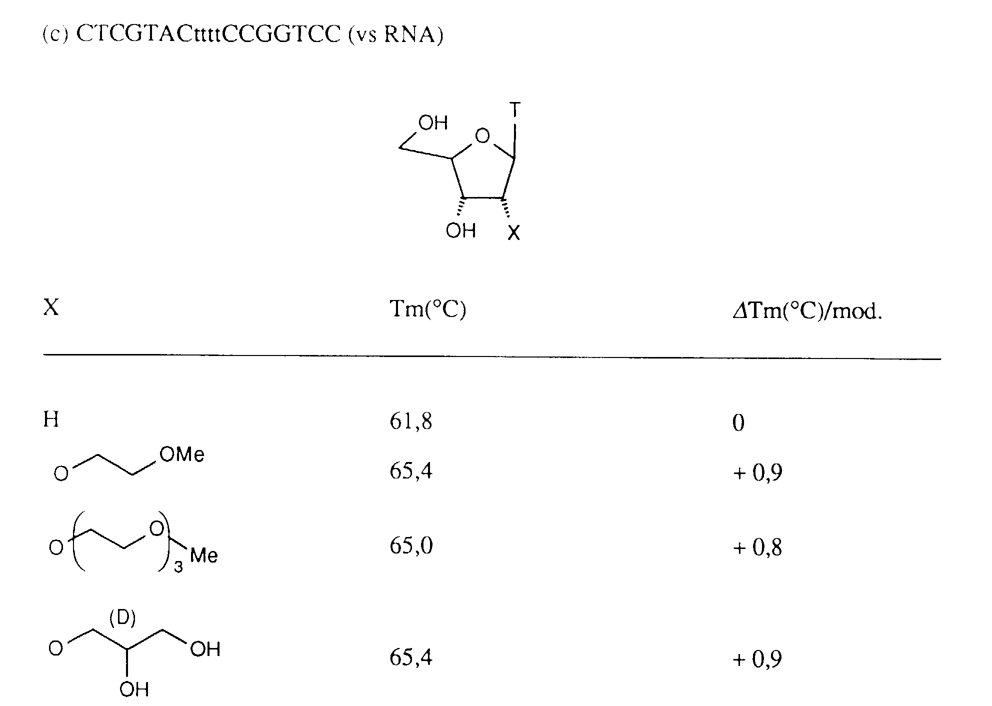

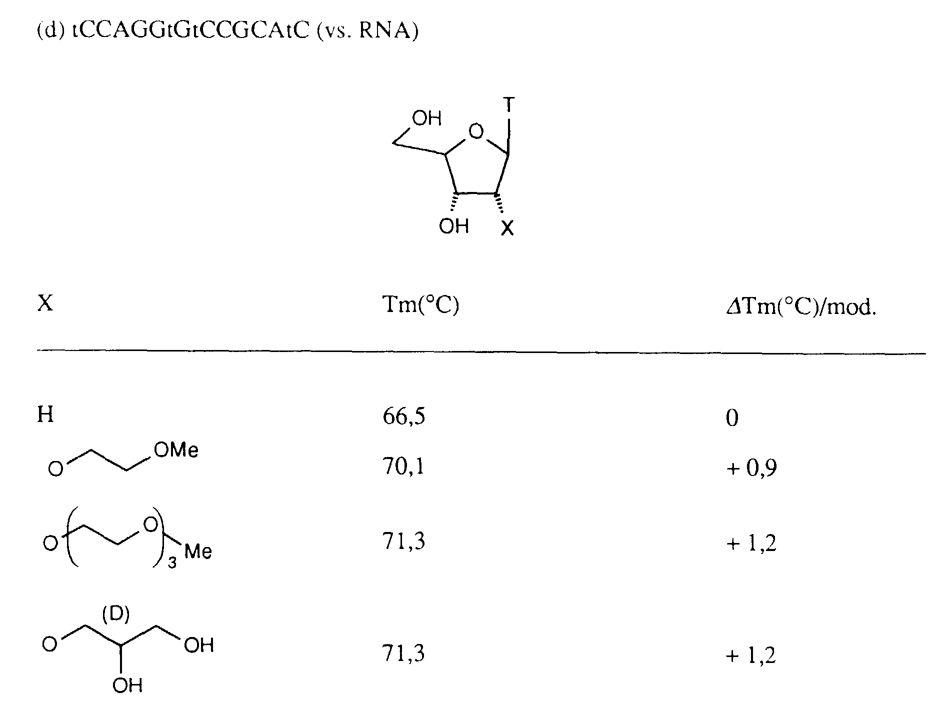

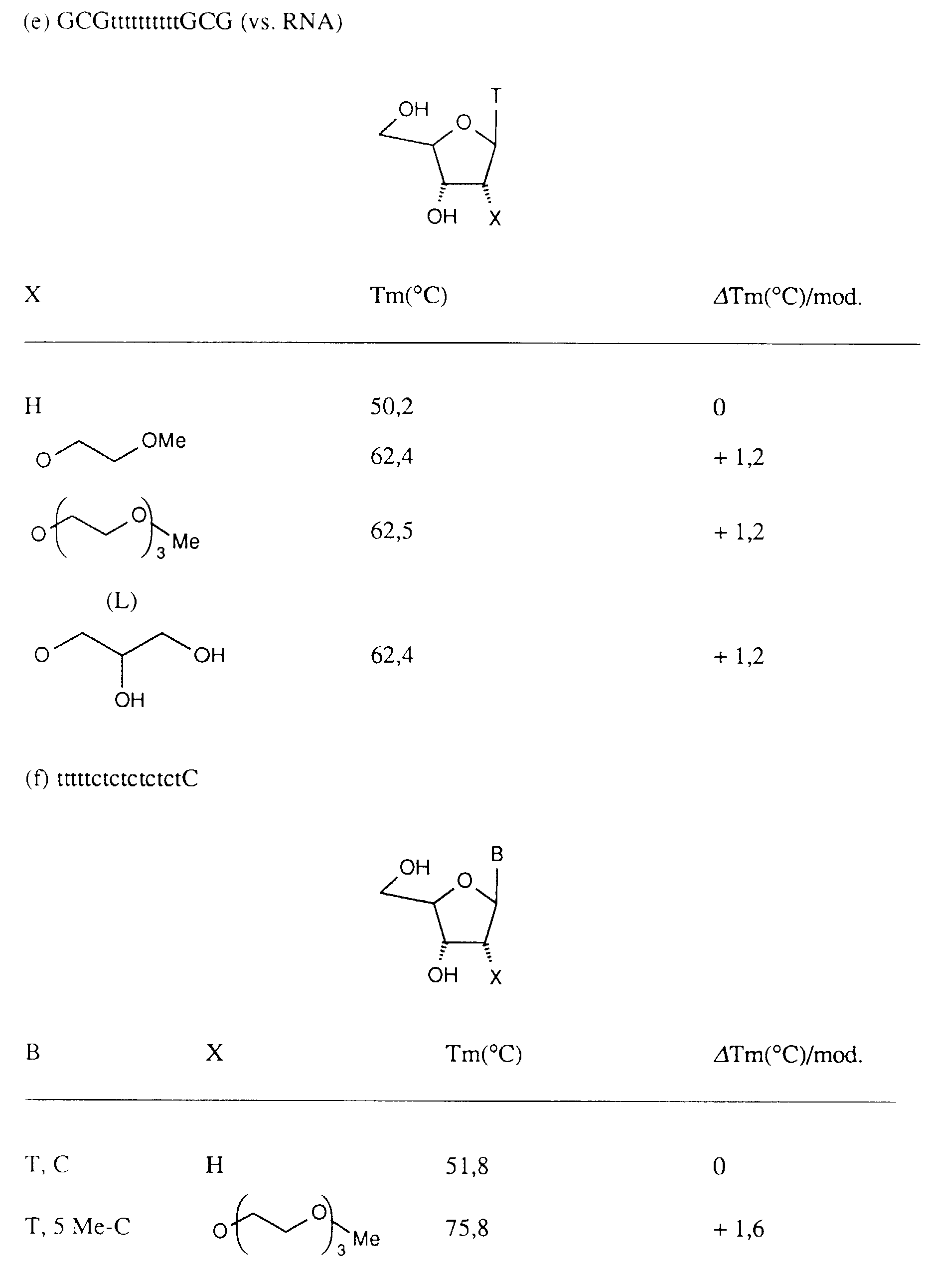

### Beispiel D2: Spezifität; Wechselwirkung des Oligonukleotids mit basenkomplementären Oligoribonukleotiden, in welche ein falsches Nukleosid (Y) eingebaut wurde

Man stellt Lösungen des Oligonukleotids mit den entsprechenden basenkomplementären Oligonukleotiden der Sequenzen r(GGA CCG GAA YGG TAC GAG) in 10 mM Phosphatpuffer, 100 mM NaCl, 0,1 mM EDTA, pH 7, (c = 4·10⁻⁶ M/Oligonukleotid) her und misst die Änderung der Extinktion bei 260 nm in Abhängigkeit von der Temperatur (15°C bis 95°C). Aus den Kurven wird der Tₘ-Wert ermittelt. Die Ergebnisse sind in der Tabelle 4 angegeben.

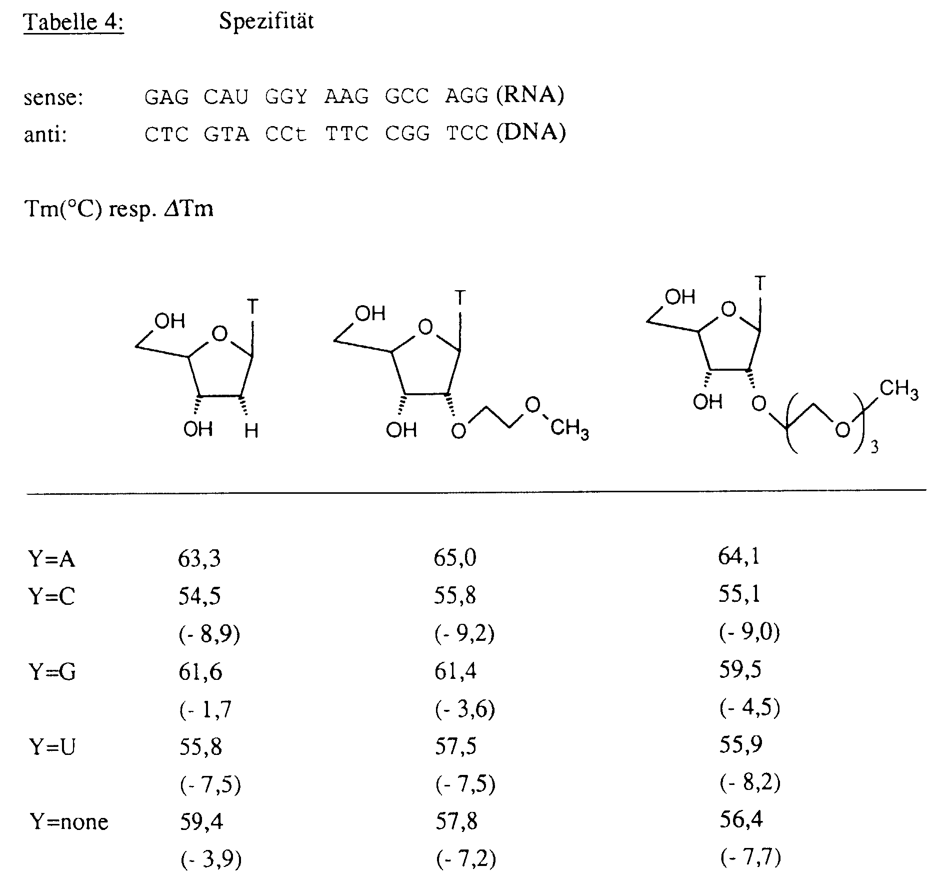

### Beispiel C3: Nucleasestabilität; Enzymatische Hydrolyse verschiedener Oligonukleotide der Sequenz d(TCC AGG TGT CCG ttt C)

Je 14 µg des synthetischen Oligonukleotids beziehungsweise des entsprechenden natürlichen Oligomers werden in 200 µl 10%-igem Hitze-inaktivierten Serum aus Kalbsföten bei 37°C inkubiert (c = 70 µg/ml). Nach 0,5; 1; 2; 4; 6, 24 und 48 Stunden werden jeweils 15 µl der Reaktionslösung durch Zugabe zu 25 µl 9 M Harnstoff und Trisborat-Puffer (pH 7) gequencht und bis zur Messung bei -20°C gelagert. Die gequenchten Reaktionslösungen werden mittels Polyacrylamid-Gelelektophorese aufgetrennt und die Spaltprodukte über den Phosphorgehalt bestimmt (Phospho-imagers-Methode). Das Verhältnis R der Summe der Konzentrationen des völlig intakten Oligonukleotids (cₙ^{(t)}) und des durch Abspaltung des natürlichen C-Bausteins vom 3'-Ende entstehenden Fragments (cₙ₋₁^{(t)}) zu einer gegebenen Zeit t zur Ausgangskonzentration des völlig intakten Oligonukleotids zum Zeitpunkt t = 0 (cₙ⁽⁰⁾) R = (cₙ^{(t)} + cₙ₋₁^{(t)})/cₙ⁽⁰⁾ wird gegen die Zeit graphisch aufgetragen. Die dabei ermittelten Halbwertszeiten τ_{1/2} - das sind jene Zeiten, für die R=0,5 - betragen

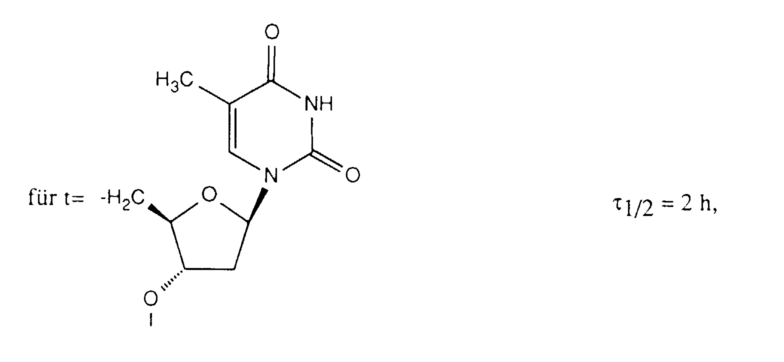

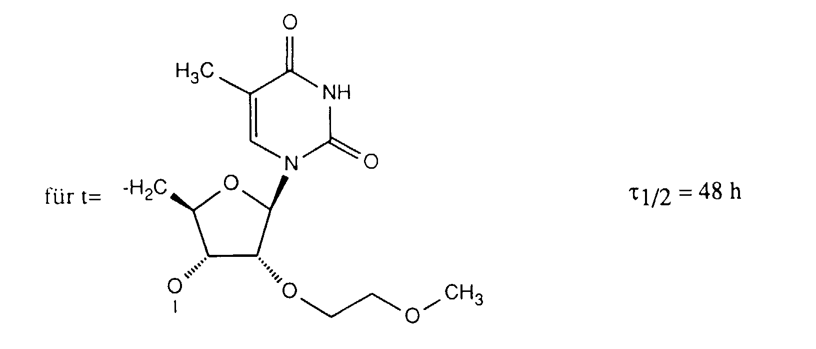

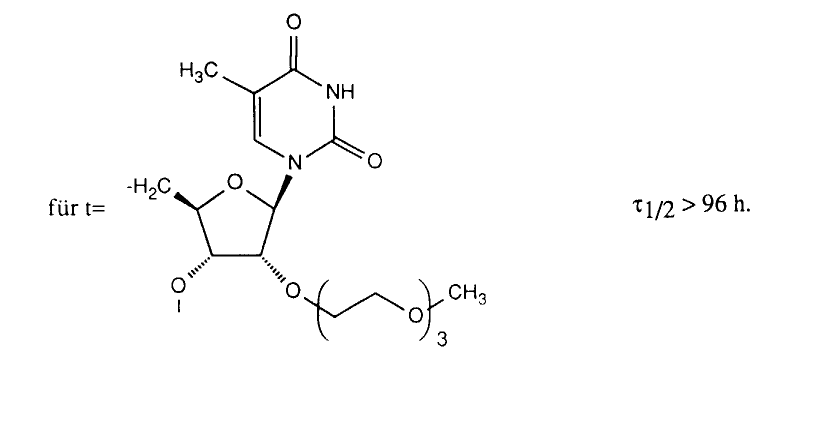

## Patentansprüche

1. Verbindungen der Formel I 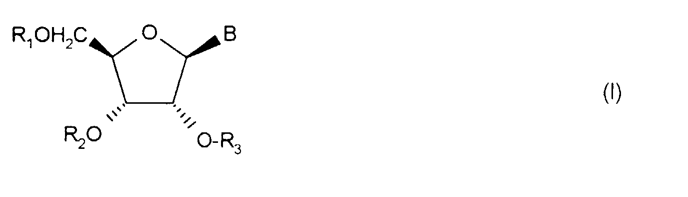 worin R₁ und R₂ unabhängig voneinander für Wasserstoff oder eine Schutzgruppe stehen oder R₁ diese Bedeutungen hat und R₂ für einen eine phosphorhaltige Nukleotid-Brückengruppe bildenden Rest steht; B einen Purin- oder Pyrimidinrest oder ein Analoges davon bedeutet; und R₃ einen Rest der Formel Ia, Ib oder Ic bedeutet 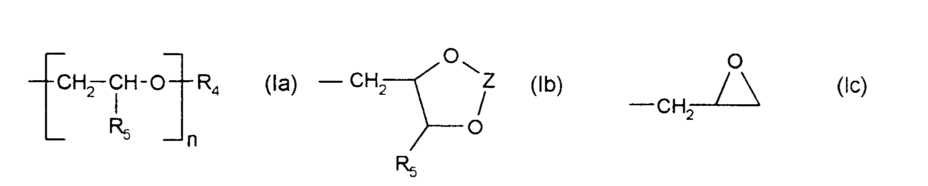 worin
R₄ Wasserstoff, C₁-C₂₁-Alkyl, C₂-C₂₁-Alkenyl, C₂-C₂₁-Alkinyl oder -C(=O)-Alkyl;
R₅ Wasserstoff, C₁-C₁₀-Alkyl, -CH₂-O-R₆ oder einen Rest der Formel Ib;
R₆ Wasserstoff, C₁-C₂₂-Alkyl, C₃-C₂₁-Alkenyl, teilweise oder vollständig mit Fluor substituiertes C₁-C₁₀-Alkyl oder -[(CH₂)₂-O]ₘ-R₇;
R₇ Wasserstoff oder C₁-C₂₁-Alkyl;
Z -(CH₂)ₚ- oder -(CH₂-CH₂-O)_{q}-CH₂CH₂-, wobei Z im Fall von -CH₂- unsubstituiert oder mit einem oder mehreren C₁-C₁₀-Alkyl, C₅-C₆-Cycloalkyl oder unsubstituiertem oder mit C₁-C₄-Alkyl substituiertem Phenyl unabhängig voneinander substituiert sein kann;
n eine Zahl von 1 bis 12;
m eine Zahl von 1 bis 4;
p eine Zahl von 1 bis 10; und
q eine Zahl von 1 bis 4 bedeuten;
wobei R₄ für den Fall n=1 und R₅=Wasserstoff nicht Wasserstoff bedeutet.

2. Verbindungen gemäss Anspruch 1, dadurch gekennzeichnet, dass R₁ und R₂ je für Wasserstoff stehen.

3. Verbindungen gemäss Anspruch 1, dadurch gekennzeichnet, dass R₁ und R₂ gleiche Schutzgruppen bedeuten.

4. Verbindungen gemäss Anspruch 1, worin B als Purinrest oder einem Analogen davon einen Rest der Formel II, IIa, IIb, IIc, IId, IIe oder IIf darstellt, 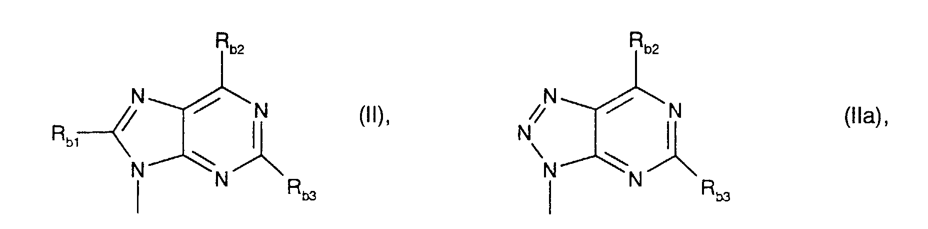 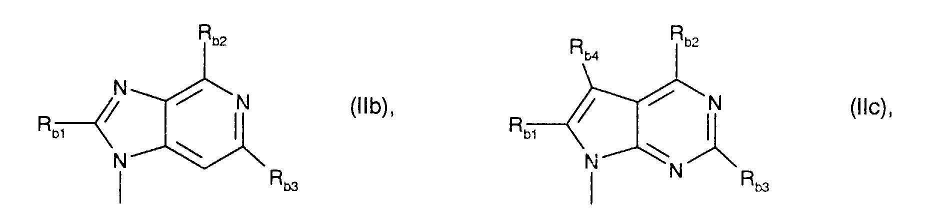 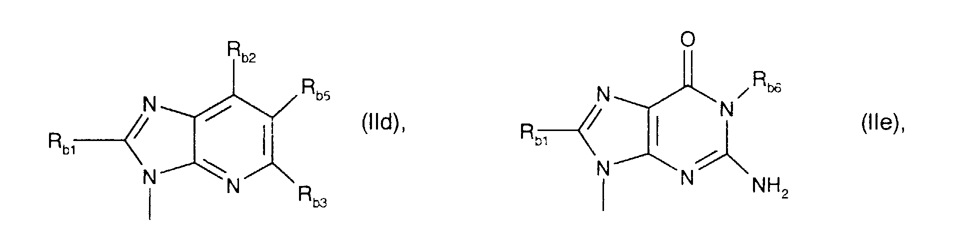 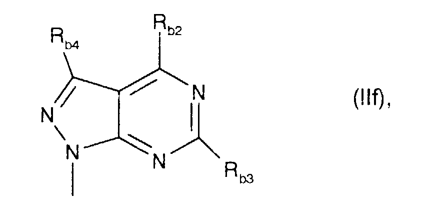 worin
R_{b1} für H, Cl, Br, OH oder -O-C₁-C₁₂-Alkyl steht, und
R_{b2}, R_{b3} und R_{b5} unabhängig voneinander H, OH, SH, NH₂, NHNH₂, NHOH, NHO-C₁-C₁₂-Alkyl, -N=CH-N(C₁-C₁₂-Alkyl)₂, -N=CH-N-Cycloalkyl, F, Cl, Br, C₁-C₁₂-Alkyl, Hydroxy-C₁-C₁₂-alkyl, Amino-C₁-C₁₂-alkyl, C₁-C₁₂-Alkoxy, Benzyloxy, C₁-C₁₂-Alkylthio, wobei die Hydroxyl- und Aminogruppen unsubstituiert oder mit einer Schutzgruppe substituiert sind, Phenyl, Benzyl, Primäramino mit 1 bis 20 C-Atomen oder Sekundäramino mit 2 bis 30 C-Atomen bedeuten,
R_{b4} Wasserstoff, CN oder -C≡C-R_{b7} sowie
R_{b6} und R_{b7} Wasserstoff oder C₁-C₄-Alkyl darstellen.

5. Verbindungen gemäss Anspruch 4, worin die Schutzgruppe für Hydroxyl- und Aminogruppen C₁-C₈-Acyl darstellt.

6. Verbindungen gemäss Anspruch 4, worin das Primäramino 1 bis 12 C-Atome und das Sekundäramino 2 bis 12 C-Atome enthält.

7. Verbindungen gemäss Anspruch 4, worin es sich bei dem Primäramino und Sekundäramino um Reste der Formel Rₐ₁Rₐ₂N handelt, worin Rₐ₁ für H steht oder unabhängig die Bedeutung von Rₐ₂ hat, und Rₐ₂ C₁-C₂₀-Alkyl, -Aminoalkyl, -Hydroxyalkyl; Carboxyalkyl oder Carbalkoxyalkyl, wobei die Carbalkoxygruppe 2 bis 8 C-Atome enthält und die Alkylgruppe 1 bis 6, bevorzugt 1 bis 4 C-Atome; C₂-C₂₀-Alkenyl; Phenyl, Mono- oder Di-(C₁-C₄-Alkyl- oder Alkoxy)phenyl, Benzyl, Mono- oder Di-(C₁-C₄-Alkyl- oder -Alkoxy)benzyl; oder 1,2-, 1,3- oder 1,4-Imidazolyl-C₁-C₆-Alkyl darstellt, oder Rₐ₁ und Rₐ₂ zusammen Tetra- oder Pentamethylen, 3-Oxa-1,5-pentylen, -CH₂-NRₐ₃-CH₂CH₂- oder -CH₂CH₂-NRₐ₃-CH₂CH₂- darstellen, worin Rₐ₃ für H oder C₁-C₄-Alkyl steht, wobei die Aminogruppe im Aminoalkyl unsubstituiert oder mit ein oder zwei C₁-C₄-Alkyl oder -Hydroxyalkylgruppen substituiert ist, und die Hydroxylgruppe im Hydroxyalkyl gegebenenfalls mit C₁-C₄-Alkyl verethert ist.

8. Verbindungen gemäss Anspruch 6, worin es sich bei dem Primäramino und Sekundäramino um Methyl-, Ethyl-, Dimethyl-, Diethyl-, Allyl-, Mono- oder Di-(hydroxyeth-2-yl)-, Phenyl- und Benzyl-, Acetyl-, Isobutyryl und Benzoylamino handelt.

9. Verbindungen gemäss Anspruch 4, worin R_{b1} in den Formeln II, IIb, IIc, IId und IIe für Wasserstoff steht.

10. Verbindungen gemäss Anspruch 4, worin R_{b5} in Formel IId für Wasserstoff steht.

11. Verbindungen gemäss Anspruch 4, worin R_{b2} und R_{b3} in den Formeln II, IIa, IIb, IIc, IId und IIf unabhängig voneinander H, F, Cl, Br, OH, SH, NH₂, NHOH, NHNH₂, Methylamino, Dimethylamino, Benzoylamino, Methoxy, Ethoxy und Methylthio darstellen.

12. Verbindungen gemäss Anspruch 4, worin B ein Purinrest oder ein Rest eines Purinanalogen aus der Reihe Adenin, N-Methyladenin, N-Benzoyladenin, 2-Methylthioadenin, 2-Aminoadenin, 6-Hydroxypurin, 2-Amino-6-chlorpurin, 2-Amino-6-methylthiopurin, Guanin und N-lsobutyrylguanin ist.

13. Verbindungen gemäss Anspruch 1, worin B in Formel I als Pyrimidinrest einen Uracil-, Thymin- oder Cytosinrest der Formel III, IIIa, IIIb oder IIIc darstellt, 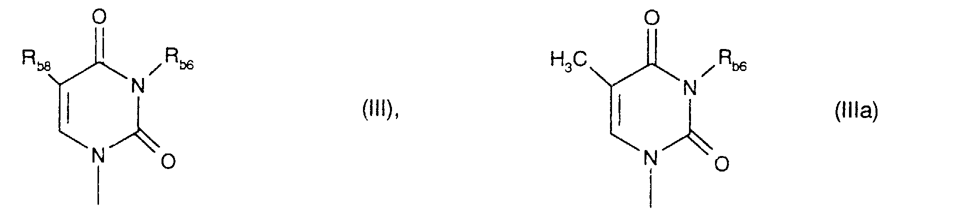 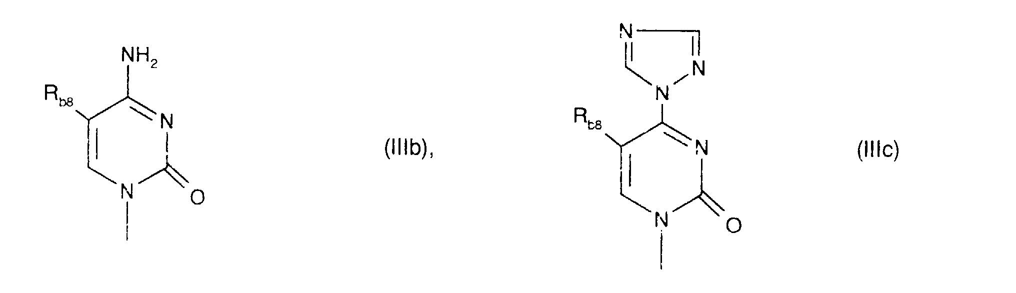 worin
R_{b6} H oder C₁-C₄-Alkyl und
R_{b8} H, OH, SH, NH₂, NHNH₂, NHOH, NHO-C₁-C₁₂-Alkyl, -N=CH-N(C₁-C₁₂-Alkyl)₂, -N=CH-N-Cycloalkyl, F, Cl, Br, C₁-C₁₂-Alkyl, Hydroxy-C₁-C₁₂-alkyl, Amino-C₁-C₁₂-alkyl, C₁-C₁₂-Alkoxy, Benzyloxy, C₁-C₁₂-Alkylthio, wobei die Hydroxyl- und Aminogruppen unsubstituiert oder mit einer Schutzgruppe substituiert sind, Phenyl, Benzyl, Primäramino mit 1 bis 20 C-Atomen, Sekundäramino mit 2 bis 30 C-Atomen, C₁-C₁₂-Alkenyl oder C₁-C₁₂-Alkinyl bedeuten, und die NH₂-Gruppe in Formel IIIb unsubstituiert oder mit C₁-C₆-Alkyl, Benzoyl oder einer Schutzgruppe substituiert ist, sowie die Dihydroderivate der Reste der Formeln III, IIIa, IIIb und IIIc.

14. Verbindungen gemäss Anspruch 13, worin R_{b8} H, C₁-C₆-Alkyl oder -Hydroxyalkyl, C₂-C₆-Alkenyl oder -Alkinyl, F, Cl, Br, NH₂, Benzoylamino, Mono- oder Di-C₁-C₆-alkylamino darstellt.

15. Verbindungen gemäss Anspruch 13, worin R_{b8} H, C₁-C₆-Alkyl oder -Alkoxy oder -Hydroxyalkyl, C₂-C₆ Alkenyl oder Alkinyl, F, Cl, Br, NH₂, Benzoylamino, Mono- oder Di-C₁-C₆-alkylamino darstellt.

16. Verbindungen gemäss Anspruch 14, worin R_{b8} H, F, Cl, Br, NH₂, NHCH₃, N(CH₃)₂, C₁-C₄-Alkyl, C₂-C₄-Alkenyl-(1) oder C₂-C₄-Alkinyl-(1) bedeutet.

17. Verbindungen gemäss Anspruch 15, worin R_{b8} H, C₁-C₄-Alkyl, C₂-C₄-Alkenyl-(1), C₂-C₄-Alkinyl-(1), NH₂, NHCH₃ oder (CH₃)₂N bedeutet.

18. Verbindungen gemäss Anspruch 1, worin B als Rest eines Pyrimidinanalogen sich von Uracil, Thymin, Cytosin, 5-Fluoruracil, 5-Chloruracil, 5-Bromuracil, Dihydrouracil, 5-Methylcytosin, 5-Propinyluracil und 5-Propinylcytosin ableitet.

19. Verbindungen gemäss Anspruch 1, dadurch gekennzeichnet, dass R₂ als phosphorhaltiger eine Nukleotid-Brückengruppe bildender Rest der Formel P1 oder P2 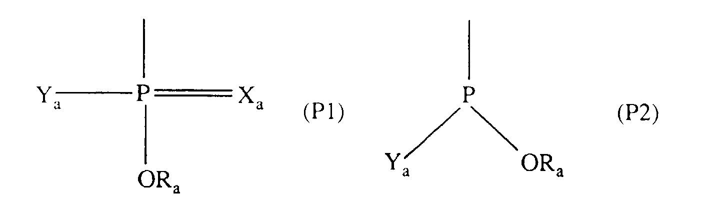 entspricht, worin Yₐ Wasserstoff, C₁-C₁₂-Alkyl, C₆-C₁₂-Aryl, C₇-C₂₀-Aralkyl, C₇-C₂₀-Alkaryl, -OR_{b}, -SR_{b}, -NH₂, Primäramino, Sekundäramino, O^{⊖}M^{⊕} oder S^{⊖} M^{⊕} darstellt; Xₐ Sauerstoff oder Schwefel bedeutet; Rₐ Wasserstoff, M^{⊕}, C₁-C₁₂-Alkyl, C₂-C₁₂-Alkenyl, C₆-C₁₂-Aryl, oder die Gruppe RₐO- für N-Heteroaryl-N-yl mit 5 Ringgliedern und 1 bis 3 N-Atomen steht; R_{b} Wasserstoff, C₁-C₁₂-Alkyl oder C₆-C₁₂-Aryl bedeutet; und M^{⊕} für Na^{⊕}, K^{⊕}, Li^{⊕}, NH₄^{⊕} steht oder Primär-, Sekundär-, Tertiär- oder Quatenärammonium darstellt; wobei Alkyl, Aryl, Aralkyl und Alkaryl in Yₐ, Rₐ und R_{b}, unsubstituiert oder mit Alkoxy, Alkylthio, Halogen, -CN, -NO₂, Phenyl, Nitrophenyl, oder Halogenphenyl substituiert ist.

20. Verbindungen gemäss Anspruch 19, dadurch gekennzeichnet, dass Rₐ β-Cyanoethyl bedeutet, und Yₐ Di(i-propyl)amino darstellt.

21. Verbindung gemäss Anspruch 1, dadurch gekennzeichnet, dass R₃ einen Rest der Formel Ia bedeutet.

22. Verbindungen gemäss Anspruch 21, dadurch gekennzeichnet, dass n für einen Zahlenwert von 1 bis 8 steht.

23. Verbindungen gemäss Anspruch 22, dadurch gekennzeichnet, dass n für einen Zahlenwert von 1 bis 6 steht.

24. Verbindungen gemäss Anspruch 21, dadurch gekennzeichnet, dass R₄ Wasserstoff, C₁-C₂₁-Alkyl, C₂-C₂₁-Alkenyl oder C₂-C₂₁-Alkinyl bedeutet.

25. Verbindungen gemäss Anspruch 24, dadurch gekennzeichnet, dass R₄ für Wasserstoff oder C₁-C₄-Alkyl steht.

26. Verbindungen gemäss Anspruch 21, dadurch gekennzeichnet, dass R₅ Wasserstoff, C₁-C₅-Alkyl oder -CH₂-OH, -CH₂-O-C₁-C₂₂-Alkyl oder -CH₂-O-[(CH₂)₂-O]ₘ-C₁-C₁₀-alkyl, worin m eine Zahl von 1 bis 4 ist, bedeutet.

27. Verbindungen gemäss Anspruch 26, dadurch gekennzeichnet, dass R₅ Wasserstoff, Methyl oder -CH₂-OH, -CH₂-O-C₁-C₂₂-Alkyl oder -CH₂-O-[(CH₂)₂-O]ₘ-C₁-C₁₀-alkyl, worin m eine Zahl von 1 bis 4 ist, bedeutet.

28. Verbindungen gemäss Anspruch 1, dadurch gekennzeichnet, dass R₃ einen Rest der Formel Ib bedeutet.

29. Verbindungen gemäss Anspruch 28, dadurch gekennzeichnet, dass Z -CH₂- oder substituiertes -CH₂- bedeutet.

30. Verbindungen gemäss Anspruch 1, dadurch gekennzeichnet, dass R₃ einen Rest der Formel Ic bedeutet.

31. Verfahren zur Herstellung von Verbindungen der Formel I, 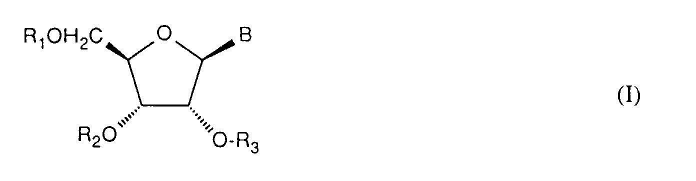 worin R₁ und R₂ unabhängig voneinander für Wasserstoff oder eine Schutzgruppe stehen oder R₁ diese Bedeutungen hat und R₂ für einen eine phosphorhaltige Nukleotid-Brückengruppe bildenden Rest steht; B einen Purin- oder Pyrimidinrest oder ein Analoges davon bedeutet; und
(a) R₃ einen Rest der Formel Ia bedeutet 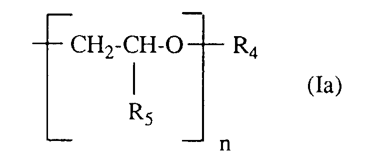 worin R₄ Wasserstoff, C₁-C₂₁-Alkyl, C₂-C₂₁-Alkenyl, C₂-C₂₁-Alkinyl oder -C(=O)-Alkyl; R₅ Wasserstoff, C₁-C₁₀-Alkyl, -CH₂-O-R₆ oder einen Rest der Formel Ib; R₆ Wasserstoff, C₁-C₂₂-Alkyl, C₃-C₂₁-Alkenyl, teilweise oder vollständig mit Fluor substituiertes C₁-C₁₀-Alkyl oder -[(CH₂)₂-O]ₘ-R₇; R₇ Wasserstoff oder C₁-C₂₁-Alkyl; n eine Zahl von 1 bis 12; und m eine Zahl von 1 bis 4 bedeuten; wobei R₄ für den Fall n=1 und R₅=Wasserstoff nicht Wasserstoff ist,
dadurch gekennzeichnet, dass man eine Verbindung der Formel IVa 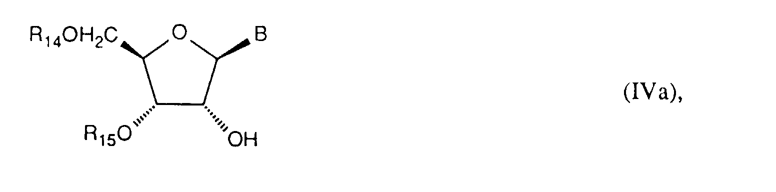 worin R₁₄ und R₁₅ für gleiche oder verschiedene Schutzgruppen stehen und B einen Purin- oder Pyrimidinrest oder ein Analoges davon bedeutet, wobei funktionelle Gruppen im Basenrest B durch Schutzgruppen geschützt sind, in einem inerten Lösungsmittel mit einer Verbindung der Formel A umsetzt 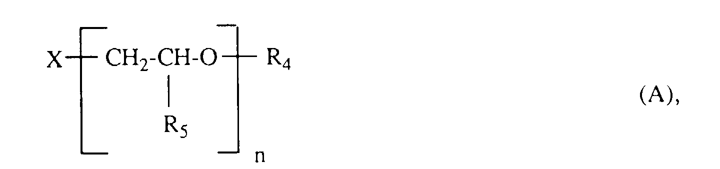 worin R₄, R₅ und n die oben genannten Bedeutungen haben und X Cl, Br, I, Tosyl-O oder Mesyl-O bedeutet;
(b) R₃ einen Rest der Formel Ic bedeutet 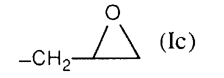 dadurch gekennzeichnet, dass man eine Verbindung der Formel IVa in einem inerten Lösungsmittel mit einer Verbindung der Formel B umsetzt 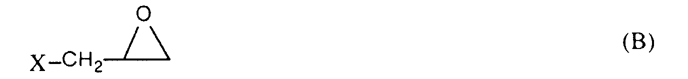 worin X Cl, Br, I, Tosyl-O oder Mesyl-O bedeutet;
(c) R₃ einen Rest der Formel Ib bedeutet 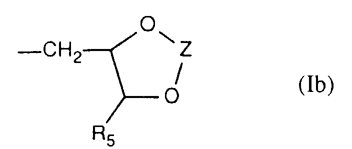 worin R₅ Wasserstoff, C₁-C₁₀-Alkyl, -CH₂-O-R₆ oder einen Rest der Formel Ib; R₆ Wasserstoff, C₁-C₂₂-Alkyl, C₃-C₂₁-Alkenyl, teilweise oder vollständig mit Fluor substituiertes C₁-C₁₀-Alkyl oder -[(CH₂)₂-O]ₘ-R₇; R₇ Wasserstoff oder C₁-C₂₁-Alkyl; Z -(CH₂)ₚ- oder -(CH₂-CH-O)_{q}-CH₂CH₂-, wobei Z im Fall von -CH₂- unsubstituiert oder mit einem oder mehreren C₁-C₁₀-Alkyl, C₅-C₆-Cycloalkyl oder unsubstituiertem oder mit C₁-C₄-Alkyl substituiertem Phenyl unabhängig voneinander substituiert sein kann; m eine Zahl von 1 bis 4; p eine Zahl von 1 bis 10; und q eine Zahl von 1 bis 4 bedeuten;
dadurch gekennzeichnet, dass man das in (b) erhaltene Epoxid in Gegenwart von H₂O und BF₃ öffnet und mit einer Verbindung der Formel X'-(CH₂)_{q}-X' oder X'-(CH₂-CH-O)ᵣ-X', worin X' Cl, Br, I, Tosyl-O oder Mesyl-O bedeutet, zu einem neuen Ring schliesst;
(d) R₃ einen Rest der Formel Ia darstellt,
dadurch gekennzeichnet, dass man das in (b) erhaltene Epoxid in Gegenwart von R₆OH und BF₃ umsetzt und gegebenenfalls die freiwerdende Hydroxylgruppe in einen Ether oder Ester überführt;
(e) R₃ einen Rest der Formel Ia darstellt,
dadurch gekennzeichnet, dass man das in (b) erhaltene Epoxid in Gegenwart von BF₃ und beispielsweise NaBH₄ hydriert und gegebenenfalls die freiwerdende Hydroxylgruppe in einen Ether oder Ester überführt;
(f) R₃ einen Rest der Formel Ia darstellt,
dadurch gekennzeichnet, dass man das in (b) erhaltene Epoxid mit einem entsprechenden Grignard-Reagenz umsetzt und gegebenenfalls die freiwerdende Hydroxylgruppe in einen Ether oder Ester überführt; oder
(g) R₃ einen Rest der Formel Ia, Ib oder Ic bedeutet,
dadurch gekennzeichnet, dass man eine Verbindung der Formel IVb 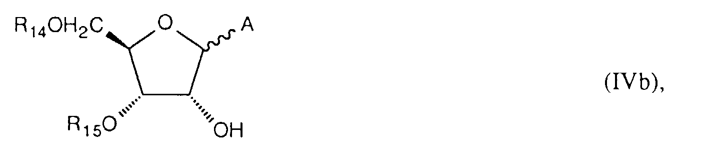
worin R₁₄ und R₁₅ die oben erwähnte Bedeutung haben und A für eine Abgangsgruppe steht, nach einem der in (a) bis (f) beschriebenen Verfahren an der 2'-OH Gruppe substituiert und den Basenrest B durch Substitution einführt; gegebenenfalls die Schutzgruppen R₁₄ und R₁₅ abspaltet und den die phosphorhaltige Nukleotid-Brückengruppe bildenden Rest einführt.

32. Verwendung der Verbindungen gemäss Anspruch 1 zur Herstellung von Oligonukleotiden, die gleiche oder verschiedene Monomereinheiten von Verbindungen der Formeln I oder die mindestens eine Monomereinheit von Verbindungen der Formeln I in Kombination mit Monomereinheiten von anderen natürlichen oder synthetischen Nukleosiden enthalten, wobei die Oligonukleotide aus 2 bis 200 Monomereinheiten bestehen.

33. Verwendung gemäss Anspruch 32 zur Herstellung von Oligonukleotiden mit 2 bis 100 Monomereinheiten.

34. Verwendung gemäss Anspruch 33 zur Herstellung von Oligonukleotiden mit 2 bis 50 Monomereinheiten.

35. Verwendung gemäss Anspruch 34 zur Herstellung von Oligonukleotiden mit 4 bis 30 Monomereinheiten.

36. Verwendung gemäss Anspruch 32 zur Herstellung von Oligonukleotiden mit gleichen oder verschiedenen Monomereinheiten von Verbindungen der Formeln I.

37. Verwendung gemäss Anspruch 32 zur Herstellung von Oligonukleotiden mit gleichen Monomereinheiten von Verbindungen der Formeln I und Monomereinheiten von natürlichen oder synthetischen Nukleosiden.

38. Oligonukleotide der Formel V
5'-U-(O-Y-O-V-)ₓO-Y-O-W-3' (V),
worin x eine Zahl von 0 bis 200 und Y eine Nukleotid-Brückengruppe bedeuten; U, V und W je für sich gleiche oder verschiedene Reste von natürlichen oder synthetischen Nukleosiden darstellen und mindestens einer der Reste U, V und/oder W einen Rest der Formel VI bedeutet 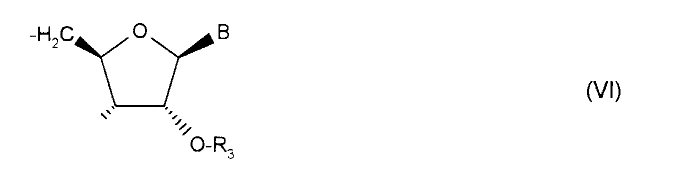 worin B einen Purin- oder Pyrimidinrest oder ein Analoges davon darstellt; und R₃ einen Rest der Formel Ia, Ib oder Ic bedeutet 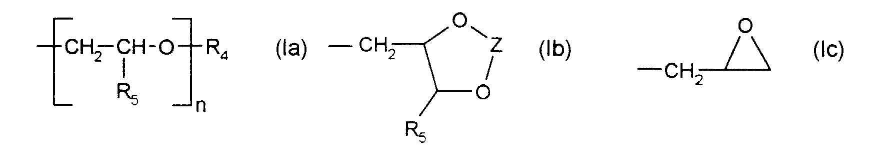 worin R₄ Wasserstoff, C₁-C₂₁-Alkyl, C₂-C₂₁-Alkenyl, C₂-C₂₁-Alkinyl oder -C(=O)-Alkyl; R₅ Wasserstoff, C₁-C₁₀-Alkyl, -CH₂-O-R₆ oder einen Rest der Formel Ib; R₆ Wasserstoff, C₁-C₂₂-Alkyl, C₃-C₂₁-Alkenyl, teilweise oder vollständig mit Fluor substituiertes C₁-C₁₀-Alkyl oder-[(CH₂)₂-O]ₘ-R₇; R₇ Wasserstoff oder C₁-C₂₁-Alkyl; Z -(CH₂)ₚ- oder -(CH₂-CH₂-O)_{q}-CH₂CH₂-, wobei Z im Fall von -CH₂- unsubstituiert oder mit einem oder mehreren C₁-C₁₀-Alkyl, C₅-C₆-Cycloalkyl oder unsubstituiertem oder mit C₁-C₄-Alkyl substituiertem Phenyl unabhängig voneinander substituiert sein kann; n eine Zahl von 1 bis 12; m eine Zahl von 1 bis 4; p eine Zahl von 1 bis 10; und q eine Zahl von 1 bis 4 bedeuten.

39. Oligonukleotide gemäss Anspruch 38, dadurch gekennzeichnet, dass es sich bei der Brückengruppe Y um -P(O)O^{⊖}-, -P(O)S^{⊖} -, -P(S)S^{⊖}-, -P(O)R₁₆-, -P(O)NR₁₇R₁₈-, oder -CH₂- handelt, worin R₁₆ H oder C₁-C₆-Alkyl darstellt, und R₁₇ und R₁₈ unabhängig voneinander die Bedeutung von R₁₆ haben.

40. Oligonukleotide gemäss Anspruch 38, dadurch gekennzeichnet, dass sich bei der Brückengruppe Y um -P(O)O^{⊖}- handelt.

41. Oligonukleotide gemäss Anspruch 38, dadurch gekennzeichnet, dass x für eine Zahl von 0 bis 100 steht.

42. Oligonukleotide gemäss Anspruch 41, dadurch gekennzeichnet, dass x für eine Zahl von 1 bis 50 steht.

43. Oligonukleotide gemäss Anspruch 42, dadurch gekennzeichnet, dass x für eine Zahl von 3 bis 29 steht.

44. Oligonukleotide gemäss Anspruch 38, dadurch gekennzeichnet, dass die Reste der Formel VI endständig und/oder in der Nukleotidsequenz gebunden sind.

45. Oligonukleotide gemäss Anspruch 38, dadurch gekennzeichnet, dass die Reste der Formel VI zwischen Resten von natürlichen oder synthetischen Nukleosiden gebunden sind.

46. Oligonukleotide gemäss den Ansprüchen 44 und 45, dadurch gekennzeichnet, dass 2 bis 5 gleiche oder verschiedene Reste der Formel VI aufeinander folgen.

47. Oligonukleotide gemäss Anspruch 38, dadurch gekennzeichnet, dass insgesamt 4 bis 30 Nukleosideinheiten und 1 bis 12 Reste der Formel VI enthalten sind.

48. Oligonukleotide gemäss Anspruch 38, worin B als Purinrest oder einem Analogen davon einen Rest der Formeln II, IIa, IIb, IIc, IId, IIe oder IIf darstellt, 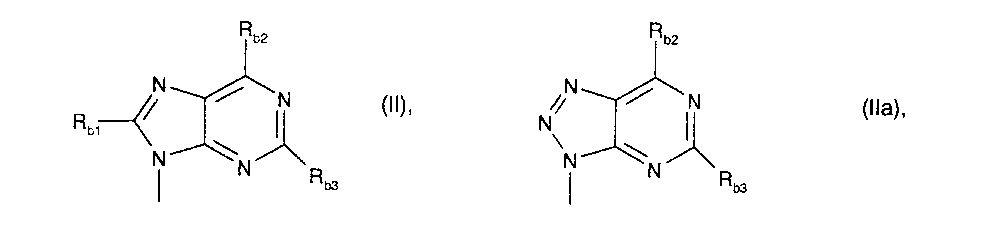 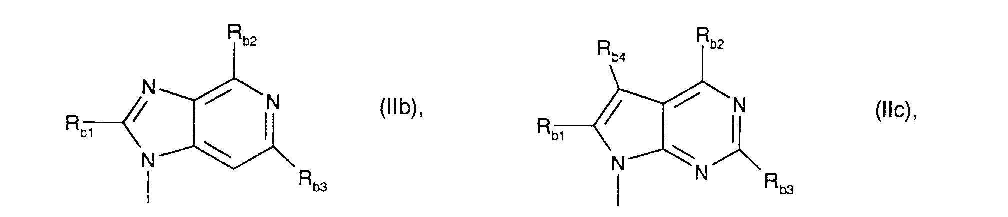 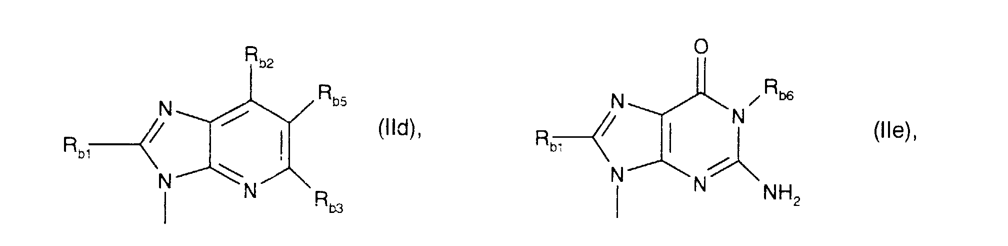 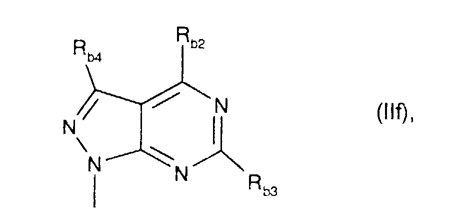 worin R_{b1} für H, Cl, Br, OH oder -O-C₁-C₁₂-Alkyl steht, und R_{b2}, R_{b3} und R_{b5} unabhängig voneinander H, OH, SH, NH₂, NHNH₂, NHOH, NHO-C₁-C₁₂-Alkyl, -N=CH-N(C₁-C₁₂-Alkyl)₂, -N=CH-N-Cycloalkyl, F, Cl, Br, C₁-C₁₂-Alkyl, Hydroxy-C₁-C₁₂-alkyl, Amino-C₁-C₁₂-alkyl, C₁-C₁₂-Alkoxy, Benzyloxy, C₁-C₁₂-Alkylthio, wobei die Hydroxyl- und Aminogruppen unsubstituiert oder mit einer Schutzgruppe substituiert sind, Phenyl, Benzyl, Primäramino mit 1 bis 20 C-Atomen oder Sekundäramino mit 2 bis 30 C-Atomen bedeuten, R_{b4} Wasserstoff, CN oder -C≡C-R_{b7}, R_{b6} und R_{b7} Wasserstoff oder C₁-C₄-Alkyl darstellen.

49. Oligonukleotide gemäss Anspruch 38, worin B einen Uracil-, Thymin- oder Cytosinrest der Formel III, IIIa, IIIb oder IIIc darstellt, 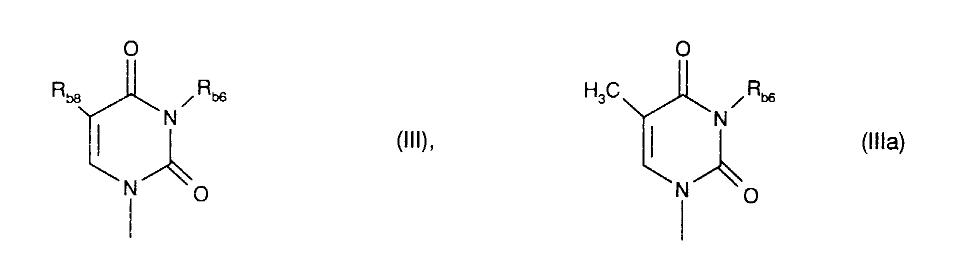 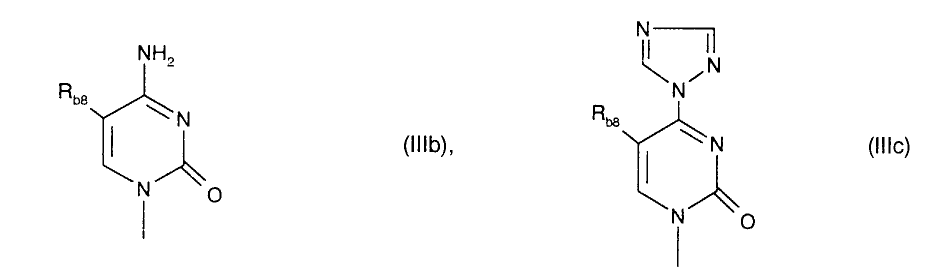 worin R_{b6} H oder C₁-C₄-Alkyl und R_{b8} H, OH, SH, NH₂, NHNH₂, NHOH, NHO-C₁-C₁₂-Alkyl, -N=CH-N(C₁-C₁₂-Alkyl)₂, -N=CH-N-Cycloalkyl, F, Cl, Br, C₁-C₁₂-Alkyl, Hydroxy-C₁-C₁₂-alkyl, Amino-C₁-C₁₂-alkyl, C₁-C₁₂-Alkoxy, Benzyloxy, C₁-C₁₂-Alkylthio, wobei die Hydroxyl- und Aminogruppen unsubstituiert oder mit einer Schutzgruppe substituiert sind, Phenyl, Benzyl, Primäramino mit 1 bis 20 C-Atomen, Sekundäramino mit 2 bis 30 C-Atomen, C₁-C₁₂-Alkenyl oder C₁-C₁₂-Alkinyl bedeuten, und die NH₂-Gruppe in Formel Illb unsubstituiert oder mit C₁-C₆-Alkyl, Benzoyl oder einer Schutzgruppe substituiert ist, sowie die Dihydroderivate der Reste der Formeln III, IIIa, IIIb und IIIc.

50. Oligonukleotide gemäss Anspruch 38, worin R₃ einen Rest der Formel Ia bedeutet.

51. Oligonukleotide gemäss Anspruch 50, dadurch gekennzeichnet, dass n für einen Zahlenwert von 1 bis 8 steht.

52. Oligonukleotide gemäss Anspruch 51, dadurch gekennzeichnet, dass n für einen Zahlenwert von 1 bis 6 steht.

53. Oligonukleotide gemäss Anspruch 50, dadurch gekennzeichnet, dass R₄ Wasserstoff, C₁-C₂₁-Alkyl, C₂-C₂₁-Alkenyl oder C₂-C₂₁-Alkinyl bedeutet.

54. Oligonukleotide gemäss Anspruch 53, dadurch gekennzeichnet, dass R₄ für Wasserstoff oder C₁-C₄-Alkyl steht.

55. Oligonukleotide gemäss Anspruch 50, dadurch gekennzeichnet, dass R₅ Wasserstoff, C₁-C₅-Alkyl oder -CH₂-OH, -CH₂-O-C₁-C₂₂-Alkyl oder -CH₂-O-[(CH₂)₂-O]ₘ-C₁-C₁₀-alkyl, worin m eine Zahl von 1 bis 4 ist, bedeutet.

56. Oligonukleotide gemäss Anspruch 55, dadurch gekennzeichnet, dass R₅ Wasserstoff, Methyl oder -CH₂-OH, -CH₂-O-C₁-C₂₂-Alkyl oder -CH₂-O-[(CH₂)₂-O]ₘ-C₁-C₁₀-alkyl, worin m eine Zahl von 1 bis 4 ist, bedeutet.

57. Oligonukleotide gemäss Anspruch 38, dadurch gekennzeichnet, dass R₃ einen Rest der Formel Ib bedeutet.

58. Oligonukleotide gemäss Anspruch 57, dadurch gekennzeichnet, dass Z -CH₂- oder substituiertes -CH₂- bedeutet.

59. Oligonukleotide gemäss Anspruch 38, dadurch gekennzeichnet, dass R₃ einen Rest der Formel Ic bedeutet.

60. Verwendung der Oligonukleotide der Formel V gemäss Anspruch 38 als Diagnostika zum Nachweis von viralen Infektionen oder genetisch bedingten Krankheiten.

61. Oligonukleotide der Formel V gemäss Anspruch 38 zur Anwendung in einem therapeutischen Verfahren zur Behandlung von Krankheiten bei Warmblütern einschliesslich des Menschen durch Wechselwirkung mit Nukleotidsequenzen im Körper.

62. Pharmazeutisches Präparat, enthaltend eine wirksame Menge eines Nukleosids der Formel I gemäss Anspruch 1 oder eines Oligonukleotids der Formel V gemäss Anspruch 38 alleine oder zusammen mit anderen Wirkstoffen, ein pharmazeutisches Trägermaterial und gegebenenfalls Hilfsstoffe.

63. Verbindung gemäss Anspruch 21, wobei
n für eine ganze Zahl von 1 bis 3 steht,
R₄ für Wasserstoff, Methyl oder Acetyl steht, und
R₅ für Wasserstoff, Methyl oder -CH₂OH steht.

64. Verbindung gemäss Anspruch 63, wobei n = 1, R₄ für Acetyl und R₅ für Wasserstoff steht.

65. Verbindung gemäss Anspruch 63, wobei n = 1, R₄ für Methyl und R₅ für Wasserstoff steht.

66. Verbindung gemäss Anspruch 63, wobei n = 1, R₄ für Methyl und R₅ für Methyl steht.

67. Verbindung gemäss Anspruch 63, wobei n = 1, R₄ für Wasserstoff und R₅ für -CH₂OH steht.

68. Verbindung gemäss Anspruch 63, wobei n = 3, R₄ für Methyl und R₅ für Wasserstoff steht.

69. Oligonukleotid gemäss Anspruch 50, wobei
n für eine ganze Zahl von 1 bis 3 steht,
R₄ für Wasserstoff, Methyl oder Acetyl steht, und
R₅ für Wasserstoff, Methyl oder -CH₂OH steht.

70. Oligonukleotid gemäss Anspruch 69, wobei n = 1, R₄ für Acetyl und R₅ für Wasserstoff steht.

71. Oligonukelotid gemäss Anspruch 69, wobei n = 1, R₄ für Methyl und R₅ für Wasserstoff steht.

72. Oligonukleotid gemäss Anspruch 69, wobei n = 1, R₄ für Methyl und R₅ für Methyl steht.

73. Oligonukleotid gemäss Anspruch 69, wobei n = 1, R₄ für Wasserstoff und R₅ für-CH₂OH steht.

74. Oligonukleotid gemäss Anspruch 69, wobei n = 3, R₄ für Methyl und R₅ für Wasserstoff steht.

## Claims

1. A compound of formula I 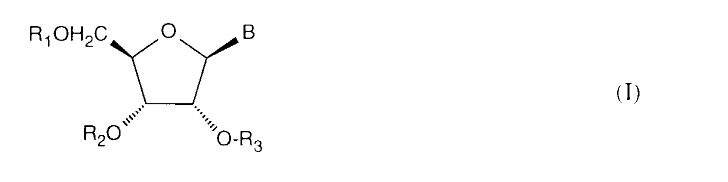 wherein
R₁ and R₂ are each independently of the other hydrogen or a protecting group, or R₁ has those definitions and R₂ is a radical forming a phosphorus-containing nucleotide bridge group;
B is a purine or pyrimidine radical or an analogue thereof; and
R₃ is a radical of formula Ia, Ib or Ic 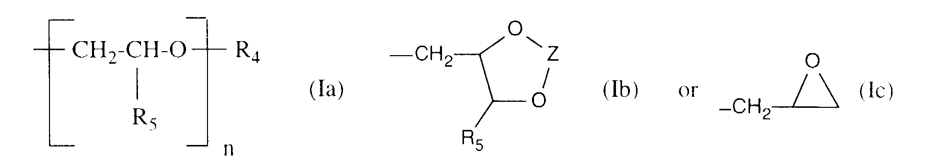 wherein
R₄ is hydrogen, C₁-C₂₁alkyl, C₂-C₂₁alkenyl, C₂-C₂₁alkynyl or -C(=O)-alkyl;
R₅ is hydrogen, C₁-C₁₀alkyl, -CH₂-O-R₆ or a radical of formula Ib;
R₆ is hydrogen C₁-C₂₂alkyl, C₃-C₂₁ alkenyl or partially or completely fluorine-substituted C₁-C₁₀alkyl or -[(CH₂)₂-O]ₘ-R₇;
R₇ is hydrogen or C₁-C₂₁alkyl;
Z is -(CH₂)ₚ- or -(CH₂-CH₂-O)_{q}-CH₂CH₂-, it being possible for Z in the case of -CH₂- to be unsubstituted or substituted by one or more identical or different substituents selected from C₁-C₁₀alkyl, C₅-C₆cycloalkyl and unsubstituted or C₁-C₄alkyl-substituted phenyl;
n is number from 1 to 12;
m is a number from 1 to 4;
p is a number from 1 to 10; and
q is a number from 1 to 4;
and R₄ is not hydrogen when n = 1 and R₅ = hydrogen.

2. A compound according to claim 1 wherein R₁ and R₂ are each hydrogen.

3. A compound according to claim 1 wherein R₁ and R₂ are identical protecting groups.

4. A compound according to claim 1 wherein B as a purine radical or an analogue thereof is a radical of formula II, IIa, IIb, IIC, IId, IIe or IIf 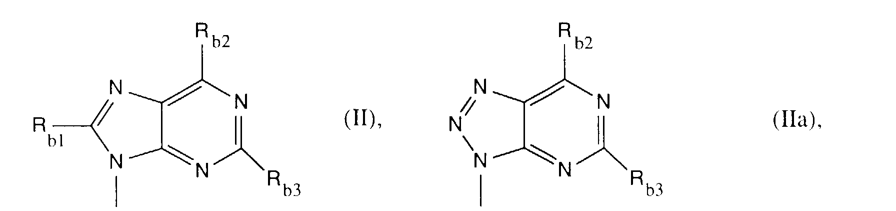 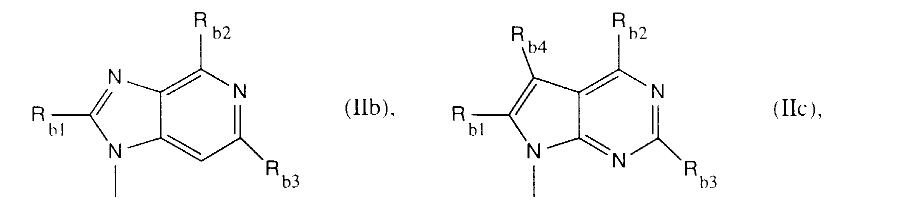 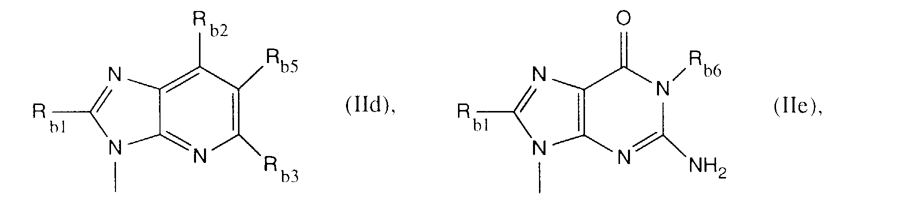 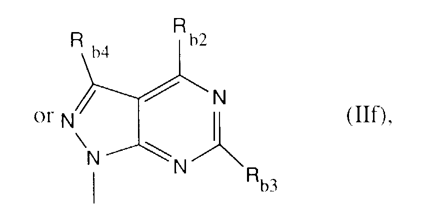 wherein
R_{b1} is H, Cl, Br, OH or -O-C₁-C₁₂alkyl, and
R_{b2}, R_{b3} and R_{b5} are each independently of the others H, OH, SH, NH₂, NHNH₂, NHOH, NHO-C₁-C₁₂alkyl, -N=CH-N(C₁-C₁₂alkyl)₂, -N=CH-N-cycloalkyl, F, Cl, Br, C₁-C₁₂alkyl, hydroxy-C₁-C₁₂alkyl , amino-C₁-C₁₂alkyl, C₁-C₁₂alkoxy, benzyloxy or C₁-C₁₂alkylthio, the hydroxy and amino groups being unsubstituted or substituted by a protecting group; or phenyl, benzyl, primary amino having from 1 to 20 carbon atoms or secondary amino having from 2 to 30 carbon atoms,
R_{b4} is hydrogen, CN or -C≡C-R_{b7}, and
R_{b6} and R_{b7} are hydrogen or C₁-C₄alkyl.

5. A compound according to claim 4 wherein the protecting group for hydroxy and amino groups is C₁-C₈acyl.

6. A compound according to claim 4 wherein the primary amino contains from 1 to 12 carbon atoms and the secondary amino contains from 2 to 12 carbon atoms.

7. A compound according to claim 4 wherein the primary amino and the secondary amino are radicals of the formula Rₐ₁Rₐ₂N, wherein Rₐ₁ is hydrogen or, independently, has the definition of Rₐ₂, and Rₐ₂ is C₁-C₂₀alkyl, C₁-C₂₀aminoalkyl or C₁-C₂₀hydroxyalkyl; carboxyalkyl or carbalkoxyalkyl, the carbalkoxy group containing from 2 to 8 carbon atoms and the alkyl group from 1 to 6, preferably from 1 to 4, carbon atoms; C₂-C₂₀alkenyl; phenyl, mono- or di-(C₁-C₄alkyl or C₁-C₄alkoxy)phenyl, benzyl, mono- or di-(C₁-C₄alkyl or C₁-C₄alkoxy)benzyl; or 1,2-, 1,3- or 1,4-imidazolyl-C₁-C₆alkyl, or Rₐ₁ and Rₐ₂ together are tetra- or penta-methylene, 3-oxa-1,5-pentylene, -CH₂-NRₐ₃-CH₂CH₂- or -CH₂CH₂-NRₐ₃-CH₂CH₂-, wherein Rₐ₃ is hydrogen or C₁-C₄alkyl; the amino group in aminoalkyl being unsubstituted or substituted by one or two C₁-C₄alkyl or C₁-C₄hydroxyalkyl groups, and the hydroxy group in hydroxyalkyl being unsubstituted or etherified by C₁-C₄alkyl.

8. A compound according to claim 6 wherein the primary amino and the secondary amino are methyl-, ethyl-, dimethyl-, diethyl-, allyl-, mono- or di-(hydroxy-eth-2-yl)-, phenyl- and benzyl-, acetyl-, isobutyryl- and benzoyl-amino.

9. A compound according to claim 4 wherein R_{b1} in formulae II, IIb, IIc, IId and IIe is hydrogen.

10. A compound according to claim 4 wherein R_{b5} in formula IId is hydrogen.

11. A compound according to claim 4 wherein R_{b2} and R_{b3} in formulae II, IIa, IIb, IIc, IId and IIf are, independently of one another, H, F, Cl, Br, OH, SH, NH₂, NHOH, NHNH₂, methylamino, dimethylamino, benzoylamino, methoxy, ethoxy and methylthio.

12. A compound according to claim 4 wherein B is a purine radical or a radical of a purine analogue from the series adenine, N-methyladenine, N-benzoyladenine, 2-methylthioadenine, 2-aminoadenine, 6-hydroxypurine, 2-amino-6-chloropurine, 2-amino-6-methylthiopurine, guanine and N-isobutyrylguanine.

13. A compound according to claim 1 wherein B in formula I, as a pyrimidine radical, is a uracil, thymine or cytosine radical of formula III, IIIa, IIIb or IIIc 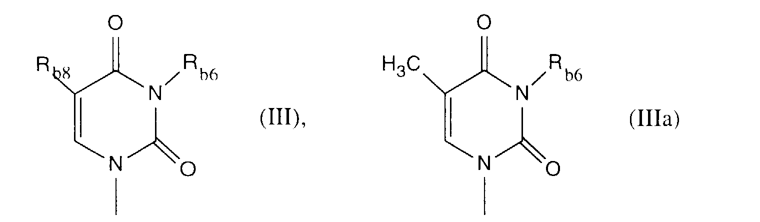 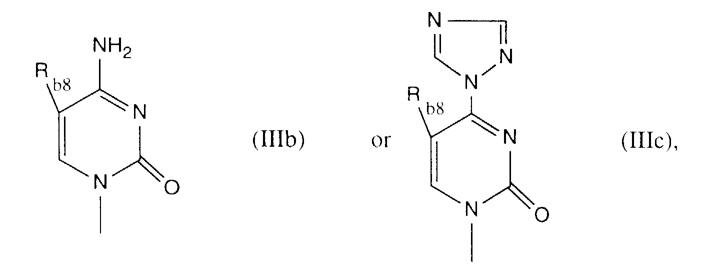 wherein R_{b6} is hydrogen or C₁-C₄alkyl and R_{b8} is H, OH, SH, NH₂, NHNH₂, NHOH, NHO-C₁-C₁₂alkyl -N=CH-N(C₁-C₁₂alkyl)₂, -N=CH-N-cycloalkyl, F, Cl, Br, C₁-C₁₂alkyl, hydroxy-C₁-C₁₂alkyl, amino-C₁-C₁₂alkyl, C₁-C₁₂alkoxy, benzyloxy or C₁-C₁₂alkylthio, the hydroxy and amino groups being unsubstituted or substituted by a protecting group, or is phenyl, benzyl, primary amino having from 1 to 20 carbon atoms, secondary amino having from 2 to 30 carbon atoms, C₁-C₁₂alkenyl or C₁-C₁₂alkynyl, and the NH₂ group in formula IIIb is unsubstituted or substituted by C₁-C₆alkyl, benzoyl or by a protecting group; or a dihydro derivative of a radical of formula III, IIIa, IIIb or IIIc.

14. A compound according to claim 13 wherein R_{b8} is hydrogen, C₁-C₆alkyl or C₁-C₆-hydroxyalkyl, C₂-C₆alkenyl or C₂-C₆alkynyl, F, Cl, Br, NH₂, benzoylamino or mono- or di-C₁-C₆alkylamino.

15. A compound according to claim 13 wherein R_{b8} is hydrogen, C₁-C₆alkyl or C₁-C₆-alkoxy or C₁-C₆hydroxyalkyl, C₂-C₆alkenyl or C₂-C₆alkynyl, F, Cl, Br, NH₂, benzoyl-amino or mono- or di-C₁-C₆alkylamino.

16. A compound according to claim 14 wherein R_{b8} is H, F, Cl, Br, NH₂, NHCH₃, N(CH₃)₂, C₁-C₄alkyl, C₂-C₄alkenyl-(1) or C₂-C₄alkynyl-(1).

17. A compound according to claim 15 wherein R_{b8} is hydrogen, C₁-C₄alkyl, C₂-C₄alkenyl-(1), C₂-C₄alkynyl-(1), NH₂, NHCH₃ or (CH₃)₂N.

18. A compound according to claim 1 wherein B as a radical of a pyrimidine analogue is derived from uracil, thymine, cytosine, 5-fluorouracil, 5-chlorouracil, 5-bromouracil, dihydrouracil, 5-methylcytosine, 5-propynyluracil and 5-propynylcytosine.

19. A compound according to claim 1 wherein R₂ as a phosphorus-containing, nucleotide-bridge-group-forming radical corresponds to formula P1 or P2 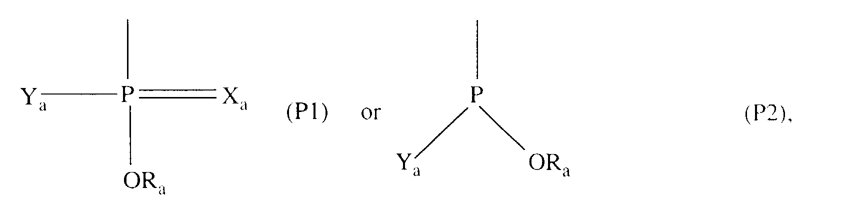 wherein
Yₐ is hydrogen, C₁-C₁₂alkyl, C₆-C₁₂aryl, C₇-C₂₀aralkyl, C₇-C₂₀alkaryl, -OR_{b}, -SR_{b}, -NH₂, primary amino, secondary amino, O^{⊖}M^{⊕} or S^{⊖}M^{⊕};
Xₐ is oxygen or sulfur;
Rₐ is hydrogen, M^{⊕}, C₁-C₁₂alkyl, C₂-C₁₂alkenyl or C₆-C₁₂aryl, or the group RₐO- is N-heteroaryl-N-yl having 5 ring members and from 1 to 3 nitrogen atoms;
R_{b} is hydrogen, C₁-C₁₂alkyl or C₆-C₁₂aryl; and
M^{⊕} is Na^{⊕}, K^{⊕}, Li^{⊕}, NH₄^{⊕} or primary, secondary, tertiary or quaternary ammonium; alkyl, aryl, aralkyl and alkaryl in Yₐ, Rₐ and R_{b} being unsubstituted or substituted by alkoxy, alkylthio, halogen, -CN, -NO₂, phenyl, nitrophenyl or halophenyl.

20. A compound according to claim 19 wherein Rₐ is β-cyanoethyl and Yₐ is di(isopropyl)amino.

21. A compound according to claim 1 wherein R₃ is a radical of formula Ia.

22. A compound according to claim 21 wherein n is a number from 1 to 8.

23. A compound according to claim 22 wherein n is a number from 1 to 6.

24. A compound according to claim 21 wherein R₄ is hydrogen, C₁-C₂₁alkyl, C₂-C₂₁-alkenyl or C₂-C₂₁alkynyl.

25. A compound according to claim 24 wherein R₄ is hydrogen or C₁-C₄alkyl.

26. A compound according to claim 21 wherein R₅ is hydrogen, C₁-C₅alkyl or -CH₂-OH, -CH₂-O-C₁-C₂₂alkyl or -CH₂-O-[(CH₂)₂-O]ₘ-C₁-C₁₀alkyl wherein m is a number from 1 to 4.

27. A compound according to claim 26 wherein R₅ is hydrogen, methyl or -CH₂-OH, -CH₂-O-C₁-C₂₂alkyl or -CH₂-O-[(CH₂)₂-O]ₘ-C₁-C₁₀alkyl wherein m is a number from 1 to 4.

28. A compound according to claim 1 wherein R₃ is a radical of formula Ib.

29. A compound according to claim 28 wherein Z is -CH₂- or substituted -CH₂-.

30. A compound according to claim 1 wherein R₃ is a radical of formula Ic.

31. A process for the preparation of a compound of formula I 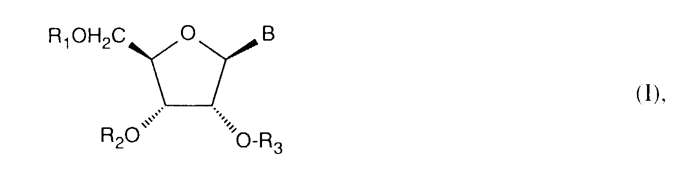 wherein R₁ and R₂ are each independently of the other hydrogen or a protecting group or R₁ has those definitions and R₂ is a radical forming a phosphorus-containing nucleotide bridge group; B is a purine or pyrimidine radical or an analogue thereof; and
(a) R₃ is a radical of formula Ia 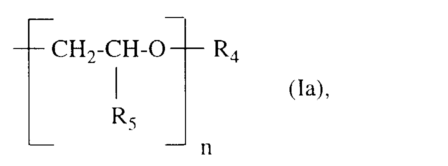 wherein
R₄ is hydrogen, C₁-C₂₁alkyl, C₂-C₂₁alkenyl, C₂-C₂₁alkynyl or -C(=O)-alkyl;
R₅ is hydrogen, C₁-C₁₀alkyl, -CH₂-O-R₆ or a radical of formula Ib;
R₆ is hydrogen, C₁-C₂₂alkyl, C₃-C₂₁alkenyl, partially or completely fluorine-substituted C₁-C₁₀alkyl or -[(CH₂)₂-O]ₘ-R₇;
R₇ is hydrogen or C₁-C₂₁alkyl;
n is a number from 1 to 12; and
m is a number from 1 to 4;
and R₄ is not hydrogen when n = 1 and R₅ = hydrogen,
which process comprises
reacting a compound of formula IVa 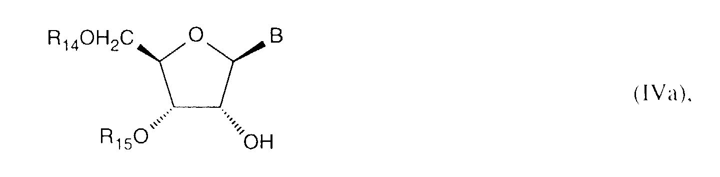 wherein R₁₄ and R₁₅ are identical or different protecting groups and B is a purine or pyrimidine radical or an analogue thereof, functional groups in the base radical B being protected by protecting groups, in an inert solvent, with a compound of formula A 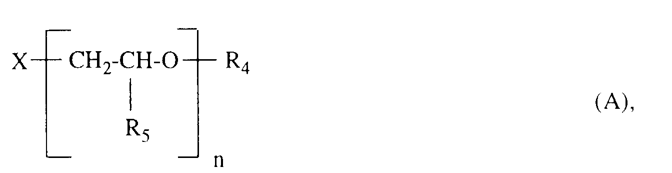 wherein R₄, R₅ and n are as defined above and X is Cl, Br, I, tosyl-O or mesyl-O;
(b) R₃ is a radical of formula Ic 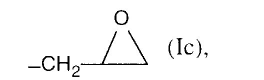 which process comprises reacting a compound of formula IVa in an inert solvent with a compound of formula B 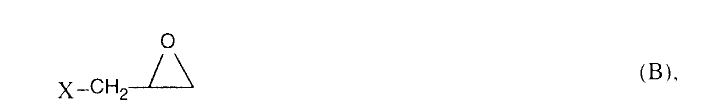 wherein X is Cl, Br, I, tosyl-O or mesyl-O;
(c) R₃ is a radical of formula Ib 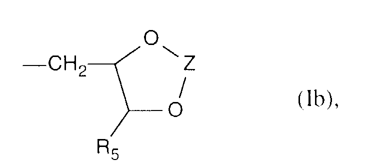 wherein
R₅ is hydrogen, C₁-C₁₀alkyl, -CH₂-O-R₆ or a radical of formula Ib;
R₆ is hydrogen, C₁-C₂₂alkyl, C₃-C₂₁alkenyl, partially or completely fluorine-substituted C₁-C₁₀alkyl or -[(CH₂)₂-O]ₘ-R₇;
R₇ is hydrogen or C₁-C₂₁alkyl;
Z is -(CH₂)ₚ- or -(CH₂-CH-O)_{q}-CH₂CH₂-, it being possible for Z in the case of -CH₂- to be unsubstituted or substituted by one or more identical or different substituents selected from C₁-C₁₀alkyl, C₅-C₆cycloalkyl and unsubstituted or C₁-C₄alkyl-substituted phenyl;
m is a number from 1 to 4;
p is a number from 1 to 10; and
q is a number from 1 to 4;
which process comprises opening the epoxide obtained in (b) in the presence of H₂O and BF₃ and closing it with a compound of formula X'-(CH₂)_{q}-X' or X^{'}-(CH₂-CH-O)ᵣ-X', wherein X' is Cl, Br, I, tosyl-O or mesyl-O, to form a new ring;
(d) R₃ is a radical of formula Ia,
which process comprises reacting the epoxide obtained in (b) in the presence of R₆OH and BF₃ and, where appropriate, converting the freed hydroxy group into an ether or an ester;
(e) R₃ is a radical of formula Ia,
which process comprises hydrogenating the epoxide obtained in (b) in the presence of BF₃ and, for example, NaBH₄, and, where appropriate, converting the freed hydroxy group into an ether or an ester;
(f) R₃ is a radical of formula Ia,
which process comprises reacting the epoxide obtained in (b) with a suitable Grignard reagent and, where appropriate, converting the freed hydroxy group into an ether or an ester; or
g) R₃ is a radical of formula Ia, Ib or Ic,
which process comprises substituting at the 2'-OH group by one of the processes described in (a) to (f) a compound of formula IVb 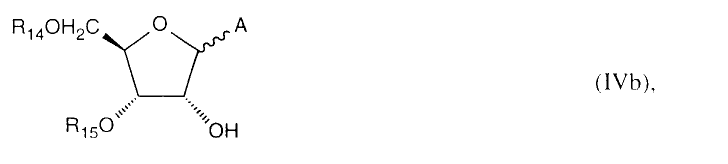 wherein R₁₄ and R₁₅ are as defined above and A is a leaving group, and introducing the base radical B by substitution; where appropriate removing the protecting groups R₁₄ and R₁₅, and introducing the radical forming a phosphorus-containing nucleotide bridge group.

32. The use of a compound according to claim 1 in the preparation of oligonucleotides comprising identical or different monomer units of compounds of formula I or comprising at least one monomer unit of compounds of formula I in combination with monomer units of other natural or synthetic nucleosides, the oligonucleotides consisting of from 2 to 200 monomer units.

33. The use according to claim 32 in the preparation of oligonucleotides having from 2 to 100 monomer units.

34. The use according to claim 33 in the preparation of oligonucleotides having from 2 to 50 monomer units.

35. The use according to claim 34 in the preparation of oligonucleotides having from 4 to 30 monomer units.

36. The use according to claim 32 in the preparation of oligonucleotides having identical or different monomer units of compounds of formula I.

37. The use according to claim 32 in the preparation of oligonucleotides having identical monomer units of compounds of formula I and monomer units of natural or synthetic nucleosides.

38. An oligonucleotide of formula V
5'-U-(O-Y-O-V-)ₓO-Y-O-W-3' (V),
wherein x is a number from 0 to 200 and Y is a nucleotide bridge group; U, V and W are, each individually, identical or different radicals of natural or synthetic nucleosides, and at least one of the radicals U, V and/or W is a radical of formula VI 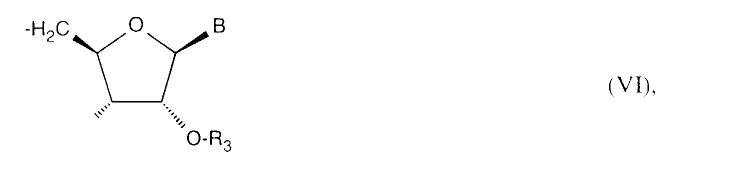 wherein B is a purine or pyrimidine radical or an analogue thereof; and R₃ is a radical of formula Ia, Ib or Ic 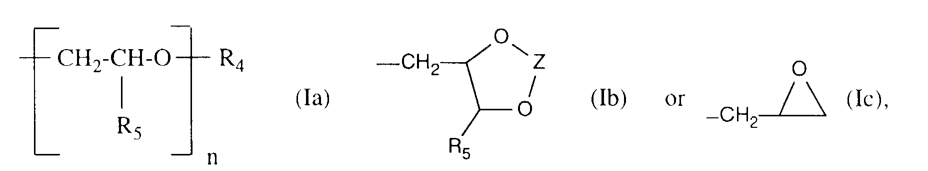 wherein
R₄ is hydrogen, C₁-C₂₁alkyl, C₂-C₂₁alkenyl, C₂-C₂₁alkynyl or -C(=O)-alkyl;
R₅ is hydrogen, C₁-C₁₀alkyl, -CH₂-O-R₆ or a radical of formula Ib;
R₆ is hydrogen, C₁-C₂₂alkyl, C₃-C₂₁alkenyl, or partially or completely fluorine-substituted C₁-C₁₀alkyl or -[(CH₂)₂-O]ₘ-R₇;
R₇ is hydrogen or C₁-C₂₁alkyl;
Z is -(CH₂)ₚ- or -(CH₂-CH₂-O)_{q}-CH₂CH₂-, it being possible for Z in the case of -CH₂- to be unsubstituted or substituted by one or more identical or different substituents selected from C₁-C₁₀alkyl, C₅-C₆cycloalkyl and unsubstituted or C₁-C₄alkyl-substituted phenyl;
n is a number from 1 to 12;
m is a number from 1 to 4;
p is a number from 1 to 10; and
q is a number from 1 to 4.

39. An oligonucleotide according to claim 38 wherein the bridge group Y is -P(O)O^{⊖}-, -P(O)S^{⊖}-, -P(S)S^{⊖}-, -P(O)R₁₆-, -P(O)NR₁₇R₁₈- or -CH₂-, wherein R₁₆ is hydrogen or C₁-C₆alkyl, and R₁₇ and R₁₈, each independently of the other, have the definition of R₁₆.

40. An oligonucleotide according to claim 38 wherein the bridge group Y is -P(O)O^{⊖}-.

41. An oligonucleotide according to claim 38 wherein x is a number from 0 to 100.

42. An oligonucleotide according to claim 41 wherein x is a number from 1 to 50.

43. An oligonucleotide according to claim 42 wherein x is a number from 3 to 29.

44. An oligonucleotide according to claim 38 wherein the radicals of formula VI are bonded terminally and/or in the nucleotide sequence.

45. An oligonucleotide according to claim 38 wherein the radicals of formula VI are bonded between radicals of natural or synthetic nucleosides.

46. An oligonucleotide according to claims 44 and 45 wherein from 2 to 5 identical or different radicals of formula VI follow one another.

47. An oligonucleotide according to claim 38 comprising a total of from 4 to 30 nucleoside units and from 1 to 12 radicals of formula VI.

48. An oligonucleotide according to claim 38 wherein B as a purine radical or as an analogue thereof is a radical of formula II, IIa, IIb, IIc, IId, IIe or IIf 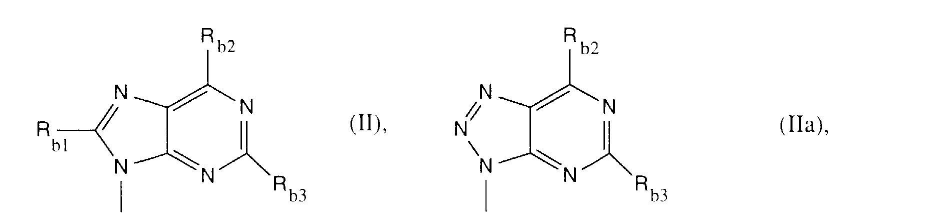 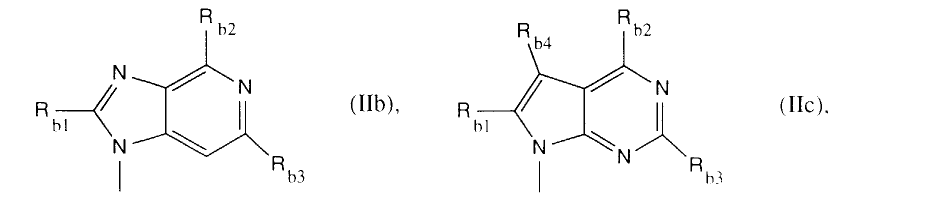 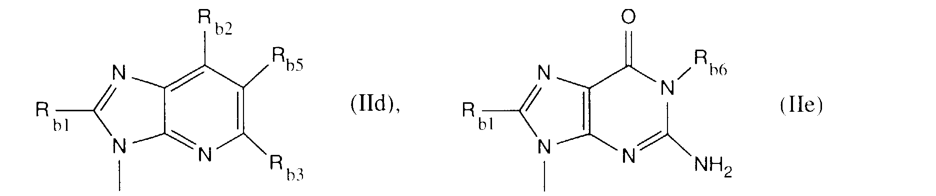 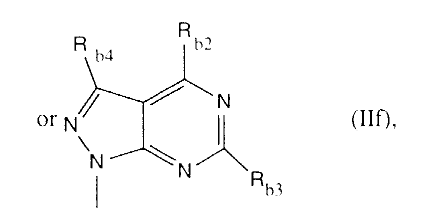 wherein
R_{b1} is H, Cl, Br, OH or -O-C₁-C₁₂alkyl, and
R_{b2}, R_{b3} and R_{b5} are each independently of the others H, OH, SH, NH₂, NHNH₂, NHOH, NHO-C₁-C₁₂alkyl, -N=CH-N(C₁-C₁₂alkyl)₂, -N=CH-N-cycloalkyl, F, Cl, Br, C₁-C₁₂-alkyl, hydroxy-C₁-C₁₂alkyl, amino-C₁-C₁₂alkyl, C₁-C₁₂alkoxy, benzyloxy or C₁-C₁₂-alkylthio, the hydroxy and amino groups being unsubstituted or substituted by a protecting group; or phenyl, benzyl, primary amino having from 1 to 20 carbon atoms or secondary amino having from 2 to 30 carbon atoms,
R_{b4} is hydrogen, CN or -C≡C-R_{b7}, and
R_{b6} and R_{b7} are hydrogen or C₁-C₄alkyl.

49. An oligonucleotide according to claim 38 wherein B is a uracil, thymine or cytosine radical of formula III, IIIa, IIIb or IIIc 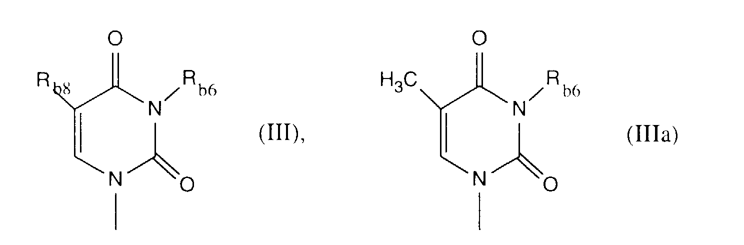 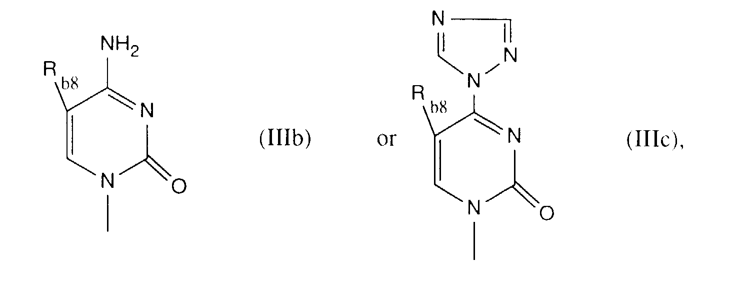 wherein R_{b6} is hydrogen or C₁-C₄alkyl and R_{b8} is H, OH, SH, NH₂, NHNH₂, NHOH, NHO-C₁-C₁₂alkyl, -N=CH-N(C₁-C₁₂alkyl)₂, -N=CH-N-cycloalkyl, F, Cl, Br, C₁-C₁₂alkyl, hydroxy-C₁-C₁₂alkyl, amino-C₁-C₁₂alkyl, C₁-C₁₂alkoxy, benzyloxy or C₁-C₁₂alkylthio, the hydroxy and amino groups being unsubstituted or substituted by a protecting group, or is phenyl, benzyl, primary amino having from 1 to 20 carbon atoms, secondary amino having from 2 to 30 carbon atoms, C₁-C₁₂alkenyl or C₁-C₁₂alkynyl, and the NH₂ group in formula IIIb is unsubstituted or substituted by C₁-C₆alkyl, benzoyl or by a protecting group; or a dihydro derivative of a radical of formula III, IIIa, IIIb or IIIc.

50. An oligonucleotide according to claim 38 wherein R₃ is a radical of formula Ia.

51. An oligonucleotide according to claim 50 wherein n is a number from 1 to 8.

52. An oligonucleotide according to claim 51 wherein n is a number from 1 to 6.

53. An oligonucleotide according to claim 50 wherein R₄ is hydrogen, C₁-C₂₁alkyl, C₂-C₂₁alkenyl or C₂-C₂₁alkynyl.

54. An oligonucleotide according to claim 53 wherein R₄ is hydrogen or C₁-C₄alkyl.

55. An oligonucleotide according to claim 50 wherein R₅ is hydrogen, C₁-C₅alkyl or -CH₂-OH, -CH₂-O-C₁-C₂₂alkyl or -CH₂-O-[(CH₂)₂-O]ₘ-C₁-C₁₀alkyl, wherein m is a number from 1 to 4.

56. An oligonucleotide according to claim 55 wherein R₅ is hydrogen, methyl or -CH₂-OH, -CH₂-O-C₁-C₂₂alkyl or -CH₂-O-[(CH₂)₂-O]ₘ-C₁-C₁₀alkyl, wherein m is a number from 1 to 4.

57. An oligonucleotide according to claim 38 wherein R₃ is a radical of formula Ib.

58. An oligonucleotide according to claim 57 wherein Z is -CH₂- or substituted -CH₂-.

59. An oligonucleotide according to claim 38 wherein R₃ is a radical of formula Ic.

60. The use of an oligonucleotide of formula V according to claim 38 as a diagnostic agent in the detection of viral infections or genetic diseases.

61. An oligonucleotide of formula V according to claim 38 for use in a therapeutic method for the treatment of diseases in warm-blooded animals, including humans, by interaction with nucleotide sequences in the body.

62. A pharmaceutical composition comprising an effective amount of a nucleotide of formula I according to claim 1 or of an oligonucleotide of formula V according to claim 38 alone or together with other active ingredients, a pharmaceutical carrier and, where appropriate, excipients.

63. A compound according to claim 21, wherein
n is an integer from 1 to 3,
R₄ is hydrogen, methyl or acetyl, and
R₅ is hydrogen, methyl or -CH₂OH.

64. A compound according to claim 63, wherein n = 1, R₄ is acetyl and R₅ is hydrogen.

65. A compound according to claim 63, wherein n = 1, R₄ is methyl and R₅ is hydrogen.

66. A compound according to claim 63, wherein n = 1, R₄ is methyl and R₅ is methyl.

67. A compound according to claim 63, wherein n = 1, R₄ is hydrogen and R₅ is -CH₂OH.

68. A compound according to claim 63, wherein n = 3, R₄ is methyl and R₅ is hydrogen.

69. An oligonucleotide according to claim 50, wherein
n is an integer from 1 to 3,
R₄ is hydrogen, methyl or acetyl, and
R₅ is hydrogen, methyl or -CH₂OH.

70. An oligonucleotide according to claim 69, wherein n = 1, R₄ is acetyl and R₅ is hydrogen.

71. An oligonucleotide according to claim 69, wherein n = 1, R₄ is methyl and R₅ is hydrogen.

72. An oligonucleotide according to claim 69. wherein n = 1, R₄ is methyl and R₅ is methyl.

73. An oligonucleotide according to claim 69, wherein n = 1, R₄ is hydrogen and R₅ is -CH₂OH.

74. An oligonucleotide according to claim 69, wherein n = 3, R₄ is methyl and R₅ is hydrogen.

## Revendications

1. Composés de formule I 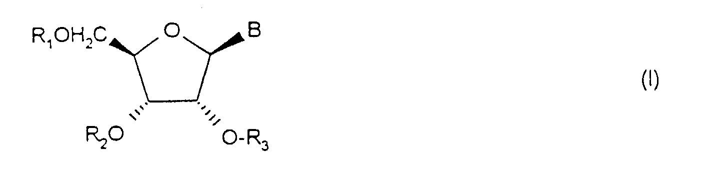 dans laquelle R₁ et R₂ représentent indépendamment l'un de l'autre un hydrogène ou un groupe protecteur, ou R₁ a ces significations et R₂ représente un radical formant un groupe de ponts de nucléotides contenant du phosphore, B représente un radical purine ou pyrimidine ou un de leurs analogues ; et R₃ représente un radical de formule Ia, Ib ou Ic 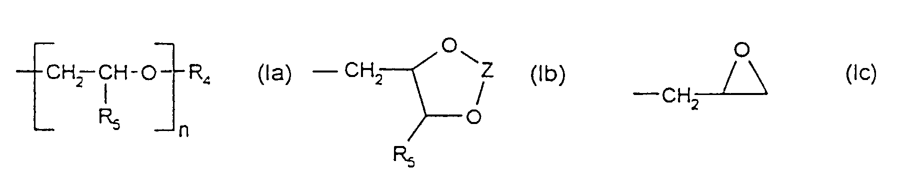 où
R₄ représente un hydrogène, un alkyle en C₁ à C₂₁, un alcényle en C₂ à C₂₁, un alcynyle en C₂ à C₂₁, ou -C(=O)-alkyle;
R₅ représente un hydrogène, un alkyle en C₁ à C₁₀, -CH₂-O-R₆ ou un radical de formule Ib;
R₆ représente un hydrogène, un alkyle en C₁ à C₂₂, un alcényle en C₃ à C₂₁, un alkyle en C₁ à C₁₀ partiellement ou entièrement substitué par des atomes de fluor, ou -[(CH₂)₂-O]ₘ-R₇ ;
R₇ représente un hydrogène ou un alkyle en C₁ à C₂₁ ;
Z représente -(CH₂)ₚ- ou (CH₂-CH₂-O)_{q}-CH₂CH₂-, où Z dans le cas de -CH₂- peut être non substitué ou substitué indépendamment par un ou plusieurs groupes alkyle en C₁ à C₁₀, cycloalkyle en C₅ à C₆ ou un phényle non substitué ou substitué par un alkyle en C₁ à C₄ ;
n représente un nombre compris entre 1 et 12 ;
m représente un nombre compris entre 1 et 4 ;
p représente un nombre compris entre 1 et 10 ; et
q représente un nombre compris entre 1 et 4;
où R₄ dans le cas où n = 1 et R₅ = hydrogène, ne représente pas un hydrogène.

2. Composés selon la revendication 1, caractérisés en ce que R₁ et R₂ représentent chacun un hydrogène.

3. Composés selon la revendication 1, caractérisés en ce que R₁ et R₂ représentent des groupes protecteurs identiques.

4. Composés selon la revendication 1, dans laquelle B en tant que radical purine ou un de ses analogues représente un radical de formule II, IIa, IIb, IIc, IId, IIe ou IIf, 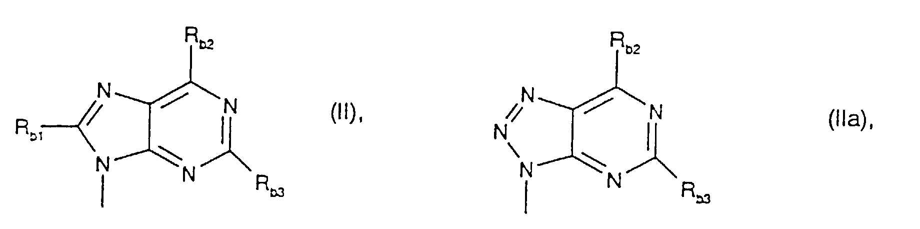 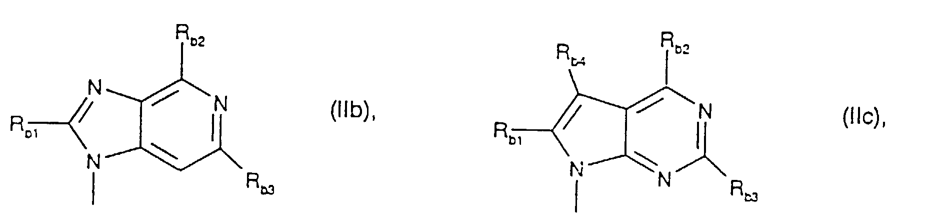 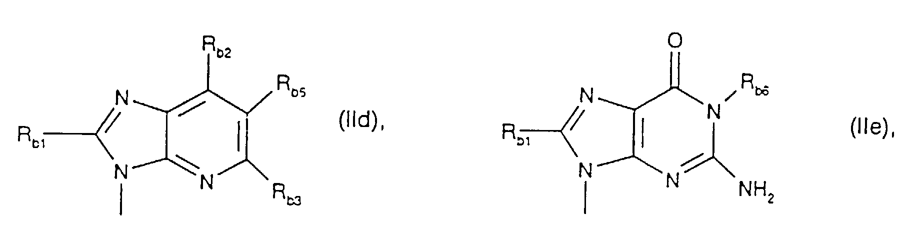 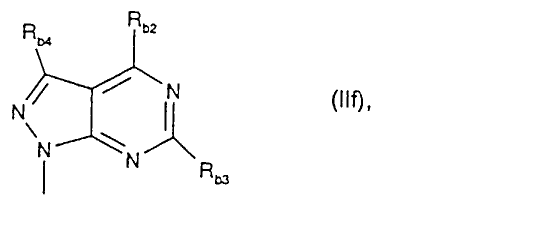 où
R_{b1} représente H, Cl, Br, OH ou un -O-alkyle en C₁ à C₁₂, et
R_{b2}, R_{b3} et R_{b5} représentent indépendamment l'un de l'autre H, OH, SH, NH₂, NHNH₂, NHOH, NHO-alkyle en C₁ à C₁₂, -N=CH-N(alkyle en C₁ à C₁₂)₂, -N=CH-N-cycloalkyle F, Cl, Br, alkyle en C₁ à C₁₂, hydroxy-alkyle en C₁ à C₁₂, amino-alkyle en C₁ à C₁₂, alcoxy en C₁ à C₁₂, benzyloxy, alkylthio en C₁ à C₁₂, où les groupes hydroxyle et amino sont non substitués ou substitués par un groupe protecteur, un phényle, un benzyle, un amino primaire ayant 1 à 20 atome(s) de carbone ou un amino secondaire ayant 2 à 30 atomes de carbone,
R_{b4} représente un hydrogène, CN ou -C=C-R_{b7} ainsi que
R_{b6} et R_{b7} représentent un hydrogène ou un alkyle en C₁ à C₄.

5. Composés selon la revendication 4, dans lesquels le groupe protecteur de groupes hydroxyle et amino est un acyle en C₁ à C₈.

6. Composés selon la revendication 4, dans lesquels l'amino primaire contient 1 à 12 atome(s) de carbone et l'amino secondaire contient 2 à 12 atomes de carbone.

7. Composés selon la revendication 4, dans lesquels, en ce qui concerne les groupes amino primaire et amino secondaire, il s'agit de radicaux de formule Rₐ₁Rₐ₂N, où Rₐ₁ représente H ou a indépendamment la signification de Rₐ₂, et Rₐ₂ représente un alkyle, un aminoalkyle ou un hydroxyalkyle en C₁ à C₂₀; un carboxyalkyle ou un carbalcoxyalkyle, où le groupe carbalcoxy contient 2 à 8 atomes de carbone et le groupe alkyle contient 1 à 6, de préférence, 1 à 4 atome(s) de carbone; un alcényle en C₂ à C₂₀; un phényle ; un mono- ou di-(alkyle ou alcoxy en C₁ à C₄)phényle, un benzyle, un mono- ou un di-(alkyle ou alcoxy en C₁ à C₄)benzyle ; ou un 1,2-, 1,3- ou 1,4-imidazolyl-alkyle en C₁ à C₆, ou Rₐ₁ et Rₐ₂ représentent ensemble un tétra- ou un pentaméthylène, un 3-oxa-1,5-pentylène, -CH₂-NRₐ₃-CH₂CH₂- ou -CH₂CH₂-NRₐ₃-CH₂CH₂-, où Rₐ₃ représente H ou un alkyle en C₁ à C₄, où le groupe amino dans l'amino-alkyle est non substitué ou substitué par un ou deux groupe(s) alkyle en C₁ à C₄ ou hydroxy-alkyle en C₁ à C₄, et le groupe hydroxyle dans l'hydroxyalkyle est éventuellement éthérifié par un alkyle en C₁ à C₄.

8. Composés selon la revendication 6, dans lesquels il s'agit, en ce qui concerne l'amino primaire et l'amino secondaire, d'un groupe méthyl-, éthyl-, diméthyl-, diéthyl-, allyl-, mono- ou di-(hydroxy-éth-2-yl)-, phényl- et benzyl-, acétyl-, isobutyryl- et benzoylamino.

9. Composés selon la revendication 4, où R_{b1} dans les formules II, IIb, IIc, IId et IIe représente un hydrogène.

10. Composés selon la revendication 4, où R_{b5} dans la formule IId représente un hydrogène.

11. Composés selon la revendication 4, où R_{b2} et R_{b3} dans les formules II, IIa, IIb, IIc, IId, et IIf représentent indépendamment l'un de l'autre H, F, Cl, Br, OH, SH, NH₂, NHOH, NHNH₂, un méthylamino, diméthylamino, benzoylamino, méthoxy, éthoxy ou méthylthio.

12. Composés selon la revendication 4, où B représente un radical purine ou un radical d'un analogue de purine de la série adénine, N-méthyladénine, N-benzoyladénine, 2-méthylthioadénine, 2-aminoadénine, 6-hydroxypurine, 2-amino-6-chloropurine, 2-amino-6-méthylthiopurine, guanine et N-isobutyrylguanine.

13. Composés selon la revendication 1, où B dans la formule I en tant que radical pyrimidine représente un radical uracyle, thymine ou cytosine de formule III, IIIa, IIIb ou IIIc, 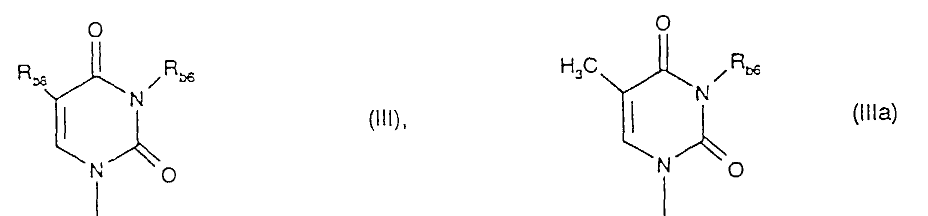 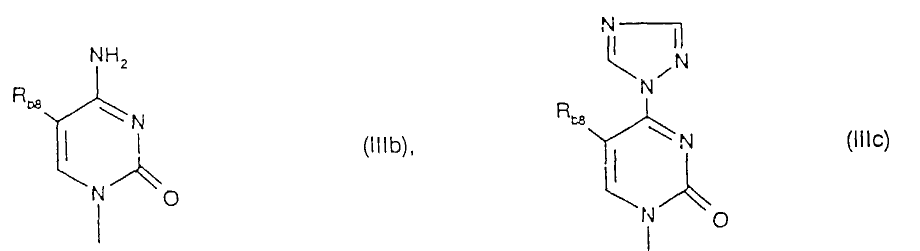 où
R_{b6} représente H ou un alkyle en C₁ à C₄ et
R_{b8} représente H, OH, SH, NH₂, NHNH₂, NHOH, NHO-alkyle en C₁ à C₁₂, -N=CH-N(alkyle en C₁ à C₁₂)₂, -N=CH-N-cycloalkyle, F, Cl, Br, alkyle en C₁ à C₁₂, hydroxy-alkyle en C₁ à C₁₂, amino-alkyle en C₁ à C₁₂, alcoxy en C₁ à C₁₂, benzyloxy, alkylthio en C₁ à C₁₂, où les groupes hydroxyle et amino sont non substitués ou substitués par un groupe protecteur, un phényle, un benzyle, un amino primaire ayant 1 à 20 atome(s) de carbone ou un amino secondaire ayant de 2 à 30 atomes de carbone, un amino secondaire ayant 2 à 30 atomes de carbone, un alcényle en C₁ à C₁₂ ou un alcynyle en C₁ à C₁₂ et le groupe NH₂ dans la formule IIIb est non substitué ou substitué par un alkyle en C₁ à C₆, un benzoyle ou un groupe protecteur, ainsi que les dérivés dihydro des radicaux de formules III, IIIa, IIIb et IIIc.

14. Composés selon la revendication 13, où R_{b8} représente H, un alkyle ou un hydroxyalkyle en C₁ à C₆, un alcényle ou un alcynyle en C₂ à C₆, F, Cl, Br, NH₂, un benzoylamino, un mono- ou di-alkylamino en C₁ à C₆.

15. Composés selon la revendication 13, où R_{b8} représente H, un alkyle, un alcoxy ou un hydroxyalkyle en C₁ à C₆, un alcényle ou un alcynyle en C₂ à C₆, F, Cl, Br, NH₂, un benzoylamino, un mono- ou dialkylamino en C₁ à C₆.

16. Composés selon la revendication 14, où R_{b8} représente H, F, Cl, Br, NH₂, NHCH₃, N(CH₃)₂, un alkyle en C₁ à C₄, un alcényle-(1) en C₂ à C₄ ou un alcynyle-(1) en C₂ à C₄.

17. Composés selon la revendication 15, où R_{b8} représente H, un alkyle en C₁ à C₄, un alcényle-(1) en C₂ à C₄, un alcynyle(1) en C₂ à C₄, NH₂, NHCH₃ ou (CH₃)₂N.

18. Composés selon la revendication 1, où B en tant que radical d'un analogue de pyrimidine dérive de l'uracile, de la thymine, de la cytosine, du 5-fluorouacile, du 5-chlorouracile, du 5-bromouracile, du dihydrouracile, de la 5-méthylcytosine, du 5-propinyluracile ou de la 5-propinylcytosine.

19. Composés selon la revendication 1, caractérisés en ce que R₂ en tant que radical formant un groupe de ponts de nucléotides contenant du phosphore correspond à la formule P1 ou P2 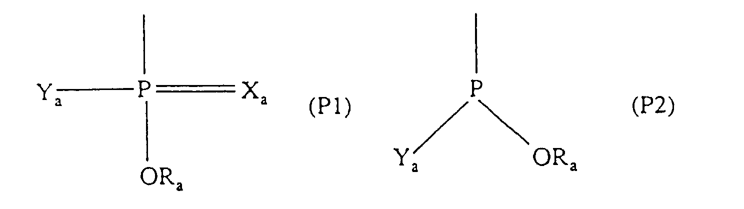 dans laquelle Yₐ représente un hydrogène, un alkyle en C₁ à C₁₂, un aryle en C₆ à C₁₂, un aralkyle en C₇ à C₂₀, un alcaryle en C₇ à C₂₀, -OR_{b}, -SR_{b}, -NH₂, un amino primaire, un amino secondaire, O⁻, M⁺ ou S⁻M⁺ ; Xₐ représente un oxygène ou un soufre, Rₐ représente un hydrogène, M⁺, un alkyle en C₁ à C₁₂, un alcényle en C₂ à C₁₂, un aryle en C₆ à C₁₂, ou le groupe RₐO-représente un N-hétéroaryl-N-yle avec 5 chaînons et 1 à 3 atome(s) de N ; R_{b} représente un hydrogène, un alkyle en C₁ à C₁₂ ou un aryle en C₆ à C₁₂ ; et M⁺ représente Na⁺, K⁺, Li⁺, NH₄⁺ ou un ammonium primaire, secondaire, tertiaire ou quaternaire ; où alkyle, aryle, aralkyle et alkaryle dans Y, Rₐ et R_{b} sont non substitués ou substitués par un alcoxy, un alkylthio, un halogène, -CN, -NO₂, un phényle, nitrophényle ou halogénophényle.

20. Composés selon la revendication 19, caractérisés en ce que Rₐ représente un β-cyanoéthyle, et Yₐ représente un di(i-propyl)amino.

21. Composés selon la revendication 1, caractérisés en ce que R₃ représente un radical de formule Ia.

22. Composés selon la revendication 21, caractérisés en ce que n représente un nombre compris entre 1 et 8.

23. Composés selon la revendication 22, caractérisés en ce que n représente un nombre compris entre 1 et 6.

24. Composés selon la revendication 21, caractérisés en ce que R₄ représente un hydrogène, un alkyle en C₁ à C₂₁, un alcényle en C₂ à C₂₁ ou un alcynyle en C₂ à C₂₁.

25. Composés selon la revendication 24, caractérisés en ce que R₄ représente un hydrogène ou un alkyle en C₁ à C₄.

26. Composés selon la revendication 21, caractérisés en ce que R₅ représente un hydrogène, un alkyle en C₁ à C₅ ou -CH₂-OH, -CH₂-O-alkyle en C₁ à C₂₂ ou -CH₂-O-[(CH₂)₂-O]ₘ-alkyle en C₁ à C₁₀, où m représente un nombre compris entre 1 et 4.

27. Composés selon la revendication 26, caractérisés en ce que R₅ représente un hydrogène, un méthyle ou -CH₂OH, -CH₂-O-alkyle en C₁ à C₂₂ ou -CH₂-O-[(CH₂)₂-O]ₘ-alkyle en C₁ à C₁₀, où m représente un nombre compris entre 1 et 4.

28. Composés selon la revendication 1, caractérisés en ce que R₃ représente un radical de formule Ib.

29. Composés selon la revendication 28, caractérisés en ce que Z représente -CH₂- ou un -CH₂-substitué.

30. Composés selon la revendication 1, caractérisés en ce que R₃ représente un radical de formule Ic.

31. Procédé de préparation de composés de formule I, 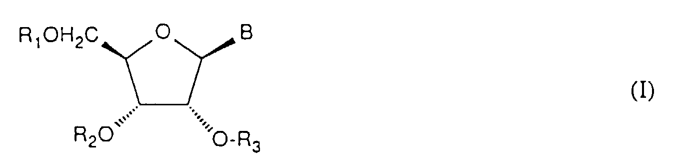 dans laquelle R₁ et R₂ représentent indépendamment l'un de l'autre un hydrogène ou un groupe protecteur, ou R₁ possède ces significations et R₂ représente un radical formant un groupe de ponts de nucléotides contenant du phosphore ; B représente un radical purine ou pyrimidine ou un de leurs analogues ; et
(a) R₃ représente un radical de formule la 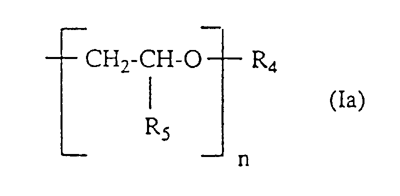 dans laquelle R₄ représente un hydrogène, un alkyle en C₁ à C₂₁, un alcényle en C₂ à C₂₁, un alcynyle en C₂ à C₂₁, ou -C(=O)-alkyle ; R₅ représente un hydrogène, un alkyle en C₁ à C₁₀, -CH₂-O-R₆ ou un radical de formule Ib ; R₆ représente un hydrogène, un alkyle en C₁ à C₂₂, un alcényle en C₃ à C₂₁, un alkyle en C₁ à C₁₀ partiellement ou entièrement substitué par des atomes de fluor ou -[(CH₂)₂-O]ₘ-R₇ ; R₇ représente un hydrogène ou un alkyle en C₁ à C₂₁ ; n représente un nombre de 1 à 12 ; et m représente un nombre de 1 à 4; où R₄ dans le cas où n = 1 et R₅ = hydrogène, n'est pas un hydrogène,
caractérisé en ce qu'on fait réagir un composé de formule IVa 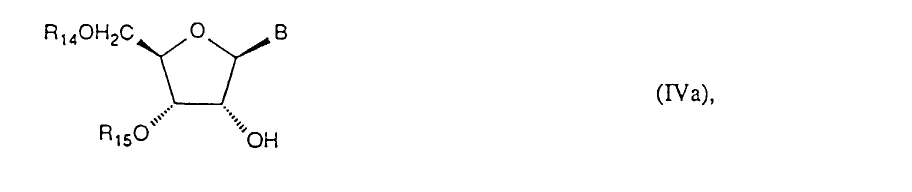 dans laquelle R₁₄ et R₁₅ représentent des groupes protecteurs identiques ou différents et B représente un radical purine ou pyrimidine ou un de leurs analogues, où les groupes fonctionnels dans le radical de base B sont protégés par des groupes protecteurs, dans un solvant inerte, avec un composé de formule A 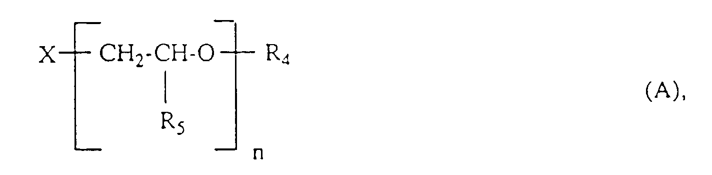 dans laquelle R₄, R₅ et n ont les significations données ci-dessus et X représente Cl, Br, I, un tosyl-O ou un mésyl-O ;
(b) R₃ représente un radical de formule Ic 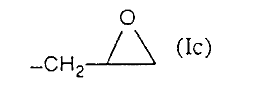 caractérisé en ce qu'on fait réagir un composé de formule IVa dans un solvant inerte, avec un composé de formule B 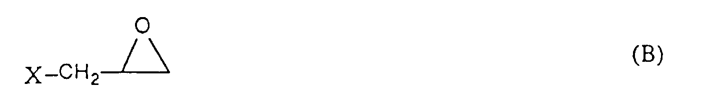 dans laquelle X représente Cl, Br, I, un tosyl-O ou un mésyl-O ;
(c) R₃ représente un radical de formule Ib 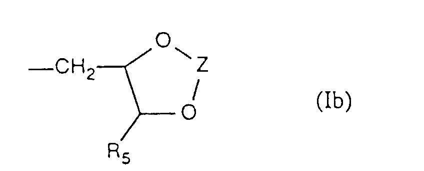 dans laquelle R₅ représente un hydrogène, un alkyle en C₁ à C₁₀, -CH₂-O-R₆ ou un radical de formule Ib ; R₆ représente un hydrogène, un alkyle en C₁ à C₂₂, un alcényle en C₃ à C₂₁, un alkyle en C₁ à C₁₀ partiellement ou entièrement substitué par des atomes de fluor ou -[(CH₂)₂-O]ₘ-R₇ ; R₇ représente un hydrogène ou un alkyle en C₁ à C₂₁ ; Z représente -(CH₂)ₚ- ou -(CH₂-CH-O)_{q}-CH₂CH₂-, où Z dans le cas de -CH₂- peut être non substitué ou substitué indépendamment par un ou plusieurs radicaux alkyle en C₁ à C₁₀, cycloalkyle en C₅ à C₆ ou un phényle non substitué ou substitué par un alkyle en C₁ à C₄ ; m représente un nombre compris entre 1 et 4 ; p représente un nombre compris entre 1 et 10 ; et q représente un nombre compris entre 1 et 4 ;
caractérisé en ce que l'on ouvre l'époxyde obtenu en (b) en présence d'H₂O et de BF₃ et en ce qu'on procède à une cyclisation pour obtenir un nouveau noyau avec un composé de formule X'-(CH₂)_{q}-X' ou X'-(CH₂-CH-O)ᵣ-X', dans laquelle X' représente Cl, Br, I, un tosyl-O ou un mésyl-O ;
(d) R₃ représente un radical de formule Ia,
caractérisé en ce qu'on fait réagir l'époxyde obtenu en (b) en présence de R₆OH et de BF₃ et, le cas échéant, en ce qu'on transforme le groupe hydroxyle qui se libère en un éther ou ester ;
(e) R₃ représente un radical de formule Ia,
caractérisé en ce qu'on hydrogène l'époxyde obtenu en (b) en présence de BF₃ et par exemple de NaBH₄ et, le cas échéant, en ce qu'on transforme le groupe hydroxyle qui se libère en un éther ou un ester;
(f) R₃ représente un radical de formule Ia, caractérisé en ce qu'on fait réagir l'époxyde obtenu en (b) avec un réactif de Grignard correspondant et, le cas échéant, en ce qu'on transforme le groupe hydroxyle qui se libère en un éther ou un ester ; ou
(g) R₃ représente un radical de formule Ia, Ib ou Ic, caractérisé en ce qu'on substitue un composé de formule IVb 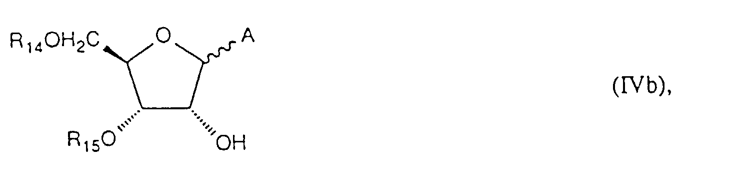
dans laquelle R₁₄ et R₁₅ ont la signification mentionnée ci-dessus et A représente un groupe sortant, selon l'un des procédés décrits en (a) à (f) et en ce qu'on introduit le radical de base B par substitution, le cas échéant en ce qu'on sépare les groupes protecteurs R₁₄ et R₁₅ et en ce qu'on introduit le radical formant le groupe de pont nucléotidique contenant du phosphore.

32. Utilisation des composés selon la revendication 1 pour la préparation d'oligonucléotides qui contiennent des unités monomères identiques ou différentes de composés de formule I ou qui contiennent au moins une unité monomère de composés de formule I en combinaison avec des unités monomères d'autres nucléosides d'origine naturelle ou synthétique. les oligonucléotides étant constitués de 2 à 200 unités monomères.

33. Utilisation selon la revendication 32 pour la préparation d'oligonucléotides ayant 2 à 100 unités monomères.

34. Utilisation selon la revendication 33 pour la préparation d'oligonucléotides ayant 2 à 50 unités monomères.

35. Utilisation selon la revendication 34 pour la préparation d'oligonucléotides ayant 4 à 30 unités monomères.

36. Utilisation selon la revendication 32 pour la préparation d'oligonucléotides ayant des unités monomères identiques ou différentes de composés de formule I.

37. Utilisation selon la revendication 32 pour la préparation d'oligonucléotides ayant des unités monomères identiques de composés de formule I et d'unités monomères de nucléosides naturels ou synthétiques.

38. Oligonucléotides de formule V
5'-U-(O-Y-O-V)ₓO-Y-O-W-3' (V)
dans laquelle x représente un nombre compris entre 0 et 200 et Y représente un groupe de ponts de nucléotides ; U, V et W représentent chacun des radicaux identiques ou différents de nucléosides d'origine naturelle ou synthétique, et au moins un des radicaux U, V et/ou W représente un radical de formule VI 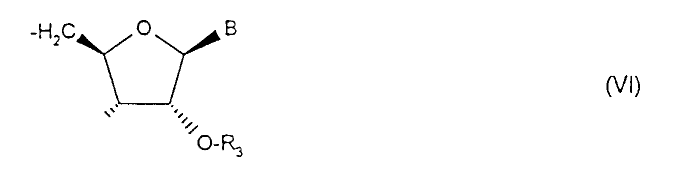 dans laquelle B représente un radical purine ou pyrimidine ou un de leurs analogues ; et R₃ représente un radical de formule Ia, Ib ou Ic 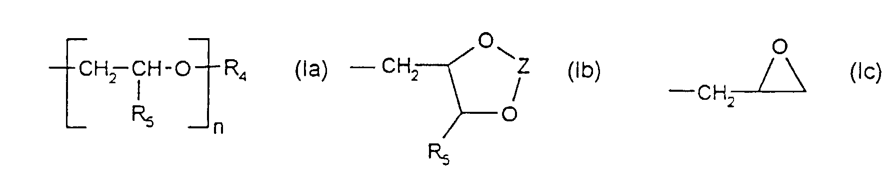 où R₄ représente un hydrogène, un alkyle en C₁ à C₂₁, un alcényle en C₂ à C₂₁, un alcynyle en C₂ à C₂₁, ou -C(=O)-alkyle ; R₅ représente un hydrogène, un alkyle en C₁ à C₁₀, -CH₂-O-R₆ ou un radical de formule Ib ; R₆ représente un hydrogène, un alkyle en C₁ à C₂₂, un alcényle en C₃ à C₂₁, un alkyle en C₁ à C₁₀ partiellement ou entièrement substitué par des atomes de fluor, ou [(CH₂)₂-O]ₘ-R₇ ; R₇ représente un hydrogène ou un alkyle en C₁ à C₂₁ ; Z représente -(CH₂)ₚ- ou -(CH₂-CH₂-O)_{q}-CH₂CH₂-, où Z dans le cas de -CH₂- peut être non substitué ou substitué par un ou plusieurs radicaux alkyle en C₁ à C₁₀, cycloalkyle en C₅ à C₆ ou un phényle non substitué ou substitué par un alkyle en C₁ à C₄ ; n représente un nombre compris entre 1 et 12, m représente un nombre compris entre 1 et 4, p représente un nombre compris entre 1 et 10 et q représente un nombre compris entre 1 et 4.

39. Oligonucléotides selon la revendication 38, caractérisés en ce que, pour ce qui est du groupe de pont Y, il s'agit de -P(O)O⁻, -P(O)S⁻, -P(S)S⁻, -P(O)R₁₆ , -P(O)NR₁₇R₁₈- ou -CH₂-, où R₁₆ représente H ou un alkyle en C₁ à C₆, et R₁₇ et R₁₈ ont indépendamment l'un de l'autre la signification de R₁₆.

40. Oligonucléotides selon la revendication 38, caractérisés en ce que, pour ce qui est du groupe de pont Y, il s'agit de -P(O)O⁻.

41. Oligonucléotides selon la revendication 38, caractérisés en ce que x représente un nombre compris entre 0 et 100.

42. Oligonucléotides selon la revendication 41, caractérisés en ce que x représente un nombre compris entre 1 et 50.

43. Oligonucléotides selon la revendication 42, caractérisés en ce que x représente un nombre compris entre 3 et 29.

44. Oligonucléotides selon la revendication 38, caractérisés en ce que les radicaux de formule VI sont liés en position finale et/ou dans la séquence nucléotidique.

45. Oligonucléotides selon la revendication 38, caractérisés en ce que les radicaux de formule VI sont liés entre des radicaux de nucléosides naturels ou synthétiques.

46. Oligonucléotides selon les revendications 44 et 45, caractérisés en ce que 2 à 5 radicaux de formule VI identiques ou différents se suivent.

47. Oligonucléotides selon la revendication 38, caractérisés en ce qu'ils contiennent au total 4 à 30 unités nucléosides et 1 à 12 radicaux de formule VI.

48. Oligonucléotides selon la revendication 38, dans lesquels B en tant que radical purine ou un de ses analogues représente un radical de formules II, IIa, IIb, IIc, IId, IIe ou IIf, 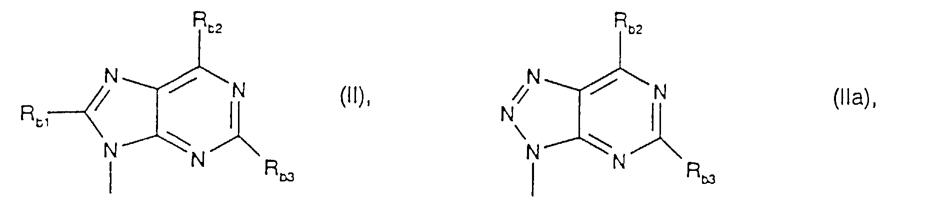 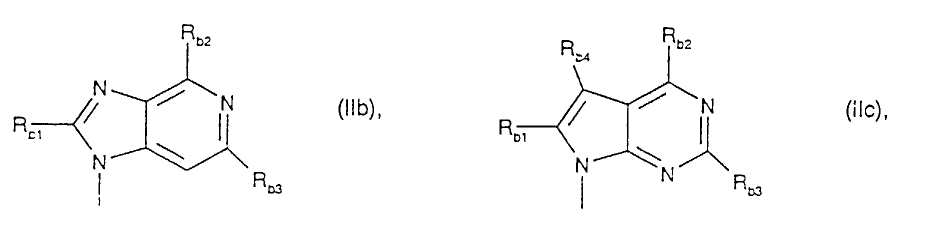 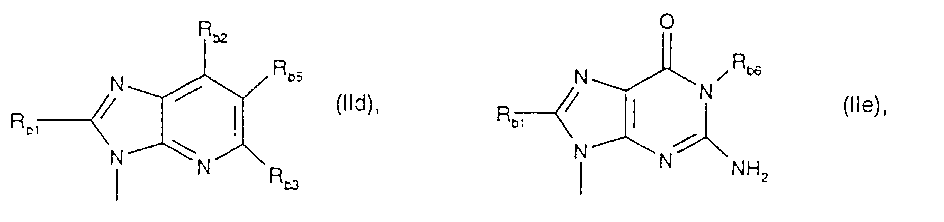 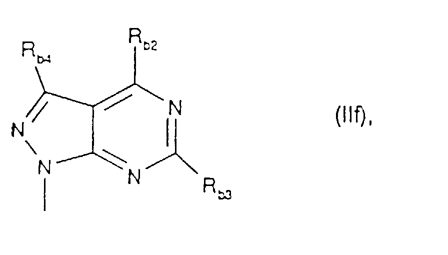 dans lesquelles R_{b1} représente H, Cl, Br, OH ou -O-alkyle en C₁ à C₁₂, et R_{b2}, R_{b3} et R_{b5} représentent indépendamment l'un de l'autre H, OH, SH, NH₂, NHNH₂, NHOH, NHO-alkyle en C₁ à C₁₂, -N=CH-N―alkyle en C₁ à C₁₂)₂, -N=CH-N-cycloalkyle, F, Cl, Br, un alkyle en C₁ à C₁₂, un hydroxy-alkyle en C₁ à C₁₂, un amino-alkyle en C₁ à C₁₂, un alcoxy en C₁ à C₁₂, un benzyloxy, un alkylthio en C₁ à C₁₂, où les groupes hydroxyle et amino sont non substitués ou substitués par un groupe protecteur, un phényle, un benzyle, un amino primaire ayant 1 à 20 atome(s) de carbone ou un amino secondaire ayant 2 à 30 atomes de carbone, R_{b4} représente un hydrogène, CN ou -C=C-R_{b7}, R_{b6} et R_{b7} représentent un hydrogène ou un alkyle en C₁ à C₄.

49. Oligonucléotides selon la revendication 38, où B représente un radical uracile, thymine ou cytosine de formule III, IIIa, IIIb ou IIIc, 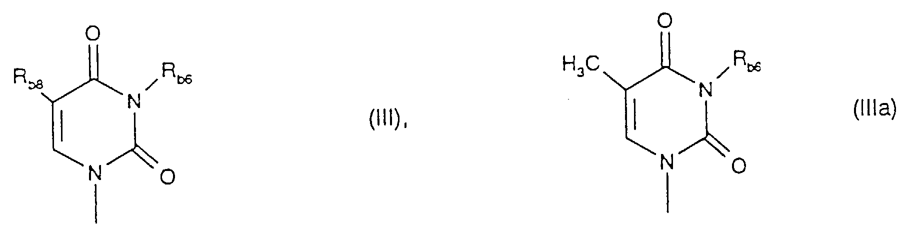 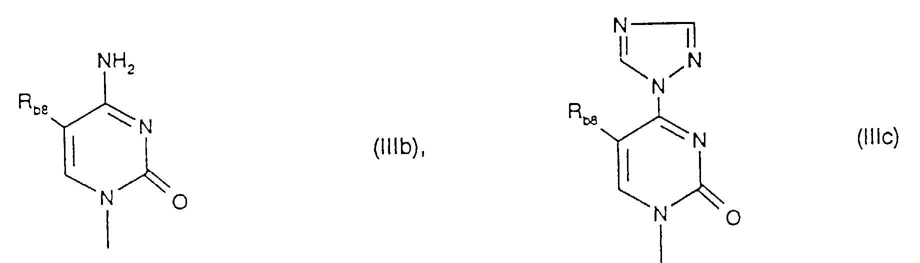 où R_{b6} représente H ou un alkyle en C₁ à C₄ et R_{b8} représente H, OH, SH, NH₂, NHNH₂, NHOH, NHO-alkyle en C₁ à C₁₂, -N=CH-N(alkyle en C₁ à C₁₂)₂, -N=CH-N-cycloalkyle, F, Cl, Br, un alkyle en C₁ à C₁₂, un hydroxy-alkyle en C₁ à C₁₂, un amino-alkyle en C₁ à C₁₂, un alcoxy en C₁ à C₁₂, un benzyloxy, un alkylthio en C₁ à C₁₂, où les groupes hydroxyle et amino sont non substitués ou substitués par un groupe protecteur, un phényle, un benzyle, un amino primaire ayant 1 à 20 atome(s) de carbone, un amino secondaire ayant 2 à 30 atomes de carbone, un alcényle en C₁ à C₁₂ ou un alcynyle en C₁ à C₁₂ et le groupe NH₂ dans la formule IIIb est non substitué ou substitué par un alkyle en C₁ à C₆, un benzoyle ou un groupe protecteur, ainsi que les dérivés dihydro des radicaux de formules III, IIIa, IIIb et IIIc.

50. Oligonucléotides selon la revendication 38, où R₃ représente un radical de formule Ia.

51. Oligonucléotides selon la revendication 50, caractérisés en ce que n représente une valeur numérique comprise entre 1 et 8.

52. Oligonucléotides selon la revendication 51, caractérisés en ce que n représente une valeur numérique comprise entre 1 et 6.

53. Oligonucléotides selon la revendication 50, caractérisés en ce que R₄ représente un hydrogène, un alkyle en C₁ à C₂₁, un alcényle en C₂ à C₂₁ ou un alcynyle en C₂ à C₂₁.

54. Oligonucléotides selon la revendication 53, caractérisés en ce que R₄ représente un hydrogène ou un alkyle en C₁ à C₄.

55. Oligonucléotides selon la revendication 50, caractérisés en ce que R₅ représente un hydrogène, un alkyle en C₁ à C₅ ou -CH₂-OH, -CH₂-O-alkyle en C₁ à C₂₂ ou -CH₂-O-[(CH₂)₂-O]ₘ-alkyle en C₁ à C₁₀, où m représente un nombre compris entre 1 et 4.

56. Oligonucléotides selon la revendication 55, caractérisés en ce que R₅ représente un hydrogène, un méthyle ou -CH₂-OH, -CH₂-O-alkyle en C₁ à C₂₂ ou -CH₂-O-[(CH₂)₂-O]ₘ-alkyle en C₁ à C₁₀, où m représente un nombre compris entre 1 et 4.

57. Oligonucléotides selon la revendication 38, caractérisés en ce que R₃ représente un radical de formule Ib.

58. Oligonucléotides selon la revendication 57, caractérisés en ce que Z représente -CH₂- ou un -CH₂- substitué.

59. Oligonucléotides selon la revendication 38, caractérisés en ce que R₃ représente un radical de formule Ic.

60. Utilisation des oligonucléotides de formule V selon la revendication 38 comme agents de diagnostic pour la mise en évidence d'infections virales ou de maladies d'origine génétique.

61. Oligonucléotides de formule V selon la revendication 38 aux fins d'application dans un procédé thérapeutique de traitement des maladies chez les homéothermes, y compris l'homme, par interaction avec des substances nucléotidiques dans le corps.

62. Préparation pharmaceutique contenant une quantité efficace d'un nucléoside de formule I selon la revendication 1 ou d'un oligonucléotide de formule V selon la revendication 38, seul ou avec d'autres substances actives, un support pharmaceutique et le cas échéant des adjuvants.

63. Composé selon la revendication 21, dans lequel
n représente un nombre entier compris entre 1 et 3,
R₄ représente un hydrogène, un méthyle ou un acétyle, et
R₅ représente un hydrogène, un méthyle ou -CH₂OH.

64. Composé selon la revendication 63, dans lequel n = 1, R₄ représente un acétyle et R₅ représente un hydrogène.

65. Composé selon la revendication 63, dans lequel n = 1, R₄ représente un méthyle et R₅ représente un hydrogène.

66. Composé selon la revendication 63, dans lequel n = 1, R₄ représente un méthyle et R₅ représente un méthyle.

67. Composé selon la revendication 63, dans lequel n = 1, R₄ représente un hydrogène et R₅ représente -CH₂OH.

68. Composé selon la revendication 63, dans lequel n = 1, R₄ représente un méthyle et R₅ représente un hydrogène.

69. Oligonucléotide selon la revendication 50, dans lequel
n représente un nombre entier compris entre 1 et 3,
R₄ représente un hydrogène, un méthyle ou un acétyle, et
R₅ représente un hydrogène, un méthyle ou -CH₂OH.

70. Oligonucléotide selon la revendication 69, dans lequel n = 1, R₄ représente un acétyle et R₅ représente un hydrogène.

71. Oligonucléotide selon la revendication 69, dans lequel n = 1, R₄ représente un méthyle et R₅ représente un hydrogène.

72. Oligonucléotide selon la revendication 69, dans lequel n = 1, R₄ représente un méthyle et R₅ représente un méthyle.

73. Oligonucléotide selon la revendication 69, dans lequel n = 1, R₄ représente un hydrogène et R₅ représente un -CH₂OH.

74. Oligonucléotide selon la revendication 69, dans lequel n = 3, R₄ représente un méthyle et R₅ représente un hydrogène.
